# EUROPEAN PATENT APPLICATION

(11) **EP 4 737 566 A1**
(43) Date of publication of application: **06.05.2026**
(21) Application number: 25806968.1
(22) Date of filing: 23.05.2025
(51) Int. Cl.: C12N 9/22, C12N 15/85, C12N 15/113, C12N 5/10, C07K 19/00, A61K 38/46, A61K 48/00

(54) **CAS12 PROTEIN AND USE THEREOF**

(30) Priority: 24.05.2024 CN 202410661837; 13.05.2025 CN 202510611056
(71) Applicant: Reforgene Medicine, Guangzhou, Guangdong 510535 (CN); Zheijiang Synsorbio Technology Co., Ltd., Shaoxing City, Zhejiang 312300 (CN); Zhejiang Synsorbio Gene Technology Co., Ltd., Shaoxing, Zhejiang 312000 (CN)
(72) Inventor: LIANG, Junbin, Guangzhou, Guangdong 510535 (CN); HUANG, Liancheng, Guangzhou, Guangdong 510535 (CN); SI, Kaiwei, Guangzhou, Guangdong 510535 (CN); CHEN, Chongjian, Shaoxing, Zhejiang 312300 (CN); SUN, Yang, Shaoxing, Zhejiang 312300 (CN); PAN, Weiye, Shaoxing, Zhejiang 312300 (CN); CAI, Jinxiu, Guangzhou, Guangdong 510535 (CN); LIAO, Qing, Guangzhou, Guangdong 510535 (CN)
(74) Representative: Dai, Simin
(86) International application number: PCT/CN2025/096995
(87) International publication number: WO 2025/242229

(57) **Abstract**

Disclosed is a Cas12 protein, a guide polynucleotide, an inactivated Cas12 mutant, a fusion protein or conjugate including the Cas12 protein, an isolated nucleic acid, a CRISPR-Cas12 system, a vector system, a delivery system, a cell, a pharmaceutical composition, and a kit, and uses thereof.

## Description

This application claims priority to Chinese Patent Application No. 202410661837.4, filed on May 24, 2024, and Chinese Patent Application No. 202510611056.9, filed on May 13, 2025, the entire contents of each of which are incorporated herein by reference.

### TECHNICAL FIELD

The present disclosure generally relates to the field of CRISPR gene editing, and in particular, to Cas12 proteins and uses thereof.

### BACKGROUND

The clustered regularly interspaced short palindromic repeats (CRISPR) and the CRISPR-associated protein system (CRISPR-Cas system) form an adaptive immune defense developed by bacteria and archaea over a long period of time, and these mechanisms are used to fight against invading viruses and exogenous DNA. Based on extensive research, CRISPR-Cas system are being employed make changes to gene sequences directly in cells, providing a fast and effective approach for gene editing.

It is therefore always desirable to develop new Cas12 proteins and CRISPR-Cas12 gene editing systems.

### SUMMARY

The present disclosure provides Cas12 proteins and uses thereof.

One or more embodiments of the present disclosure provide a Cas12 protein, and the Cas12 protein is selected from the group consisting of a CLUSTER1 protein, a CLUSTER2 protein, a CLUSTER3 protein, a CLUSTER4 protein, a CLUSTERS protein, a CLUSTER6 protein, a CLUSTER7 protein, a CLUSTERS protein, a CLUSTERS protein, a CLUSTER10 protein, a CLUSTER11 protein, and a CLUSTER12 protein.

In some embodiments, the Cas12 protein is the CLUSTER1 protein.

In another aspect, one or more embodiments of the present disclosure provide a Cas12 protein, the Cas12 protein belongs to a Cas12h subtype (subtype V-H), and the Cas12 protein specifically binds to a target nucleic acid in a eukaryotic cell.

In some embodiments, the Cas12 protein forms a complex with a guide polynucleotide, and the complex specifically binds to the target nucleic acid in the eukaryotic cell. In some embodiments, the Cas12 protein forms a complex with a guide polynucleotide, and the complex specifically binds to and cleaves the target nucleic acid in the eukaryotic cell.

In some embodiments, the target nucleic acid is located within a nucleus of the eukaryotic cell. In some embodiments, the target nucleic acid is located within a mitochondrion of the eukaryotic cell. In some embodiments, the target nucleic acid is located within a chloroplast of the eukaryotic cell.

In some embodiments, the eukaryotic cell is a mammalian cell. In some embodiments, the eukaryotic cell is a human cell.

In some embodiments, the Cas12 protein is the CLUSTER1 protein.

In some embodiments, the Cas12 protein comprises an amino acid sequence having at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to any one of the amino acid sequences shown in SEQ ID NO: 3 or SEQ ID NO: 18.

In another aspect, one or more embodiments of the present disclosure provides a Cas12 protein, and the Cas12 protein comprises an amino acid sequence having at least 50% sequence identity to any one of the amino acid sequences shown in SEQ ID NOs: 1-35.

Table 1 lists Cas proteins having the amino acid sequences shown in SEQ ID NOs: 1-35, and Table 2 lists a direct repeat (DR) sequence corresponding to each Cas protein.

In some embodiments, the at least 50% sequence identity comprises at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity.

In some embodiments, the Cas12 protein comprises an amino acid sequence having at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to any one of the amino acid sequences shown in SEQ ID NOs: 1-35.

In some embodiments, the Cas12 protein comprises an amino acid sequence having at least 80% sequence identity to any one of the amino acid sequences shown in SEQ ID NOs: 1-35.

In some embodiments, the Cas12 protein comprises an amino acid sequence having at least 85% sequence identity to any one of the amino acid sequences shown in SEQ ID NOs: 1-35.

In some embodiments, the Cas12 protein comprises an amino acid sequence having at least 90% sequence identity to any one of the amino acid sequences shown in SEQ ID NOs: 1-35.

In some embodiments, the Cas12 protein comprises an amino acid sequence having at least 95% sequence identity to any one of the amino acid sequences shown in SEQ ID NOs: 1-35.

In some embodiments, the Cas12 protein comprises an amino acid sequence having at least 97% sequence identity to any one of the amino acid sequences shown in SEQ ID NOs: 1-35.

In some embodiments, the Cas12 protein comprises an amino acid sequence having at least 98% sequence identity to any one of the amino acid sequences shown in SEQ ID NOs: 1-35.

In some embodiments, the Cas12 protein comprises an amino acid sequence having at least 99% sequence identity to any one of the amino acid sequences shown in SEQ ID NOs: 1-35.

In some embodiments, the Cas12 protein comprises an amino acid sequence having at least 99.5% sequence identity to any one of the amino acid sequences shown in SEQ ID NOs: 1-35.

In some embodiments, the Cas12 protein comprises an amino acid sequence having at least 99.7% sequence identity to any one of the amino acid sequences shown in SEQ ID NOs: 1-35.

In some embodiments, the Cas12 protein comprise an amino acid sequence having at least 99.8% sequence identity to any one of the amino acid sequences shown in SEQ ID NOs: 1-35.

**In** some embodiments, the Cas12 protein comprises an amino acid sequence having 100% sequence identity to any one of the amino acid sequences shown in SEQ ID NOs: 1-35.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 1.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 2.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 3.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 4.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 5.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 6.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 7.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 8.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 9.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 10.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 11.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 12.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 13.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 14.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 15.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 16.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 17.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 18.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 19.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 20.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 21.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 22.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 23.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 24.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 25.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 26.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 27.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 28.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 29.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 30.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 31.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 32.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 33.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 34.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 35.

In some embodiments, the Cas12 protein retains a function of a protein having an amino acid sequence as shown in any one of SEQ ID NOs: 1-35.

In some embodiments, the Cas12 protein forms a complex with a guide polynucleotide. In some embodiments, the Cas12 protein and the guide polynucleotide specifically bind to a target nucleic acid.

In some embodiments, the Cas12 protein forms a complex with a guide polynucleotide, and the complex specifically binds to a target nucleic acid. In some embodiments, the Cas12 protein forms a complex with a guide polynucleotide, and the complex specifically binds to a target DNA.

In some embodiments, the Cas12 protein and a guide polynucleotide specifically bind to and cleave a target nucleic acid. In some embodiments, the Cas12 protein and a guide polynucleotide specifically bind to and cleave a target DNA. In some embodiments, the Cas12 protein forms a complex with a guide polynucleotide, and the complex specifically binds to and cleaves a target nucleic acid. In some embodiments, the Cas12 protein forms a complex with a guide polynucleotide, and the complex specifically binds to and cleaves a target DNA.

As used herein, the phrase "retaining a function of a protein having an amino acid sequence as shown in any one of SEQ ID NOs: 1-35" refers to retaining the ability to form a complex with a guide polynucleotide, retaining the ability to bind to a target nucleic acid complementary to a guide sequence of the guide polynucleotide, retaining the ability to specifically cleave a target nucleic acid with the guide polynucleotide, and/or retaining the ability to process an RNA transcript containing the guide sequence into guide polynucleotide molecules.

In some embodiments, the retaining a function of a protein having an amino acid sequence as shown in any one of SEQ ID NOs: 1-35 refers to retaining the ability to form a complex with a guide polynucleotide.

In some embodiments, the retaining a function of a protein having an amino acid sequence as shown in any one of SEQ ID NOs: 1-35 refers to retaining the ability to bind to a target nucleic acid complementary to a guide sequence of a guide polynucleotide.

In some embodiments, the retaining a function of a protein having an amino acid sequence as shown in any one of SEQ ID NOs: 1-35 refers to retaining the ability to specifically cleave a target nucleic acid with a guide polynucleotide.

In some embodiments, the retaining a function of a protein having an amino acid sequence as shown in any one of SEQ ID NOs: 1-35 refers to retaining the ability to process an RNA transcript containing a guide sequence into guide polynucleotide molecules.

In some embodiments, the Cas12 protein comprises an amino acid sequence as shown in any one of SEQ ID NOs: 1-35.

In some embodiments, a protospacer adjacent motif (PAM) sequence (5'→3') recognized by the Cas12 protein is selected from any one or more of the following:
A, C, T, G,
TA, TC, GN, AA, AG, TG, AN, GG, CG, TN, NT, NG, GT, NA, CC, AC, GC, AT, CT, GA, TT, CN, NC, CA,
NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG,
NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, or NNGC.

N is A, T, C, or G.

In some embodiments, a PAM sequence (5'→3') recognized by the Cas12 protein is selected from one or more of the following: WYR, BMCTTH, TTN, VNWTV, VNWTC, or VNTTC.

W is A or T, Y is C or T, R is A or G, B is C, G, or T, M is A or C, H is A, T, or C, N is A, T, C, or G, and V is A, C, or G.

In some embodiments, the PAM sequence recognized by the Cas12 protein is WYR. W is A or T, Y is C or T, and R is A or G.

In some embodiments, a PAM sequence (5'→3') recognized by a conjugate is selected from any one, two, or more of the following degenerate sequences or non-degenerate sequences (where the non-degenerate sequence refers to any specific sequence encompassed by the degenerate sequence): WYR, BMCTTH, TTN, VNWTV, VNWTC, or VNTTC.

W is A or T, Y is C or T, R is A or G, B is C, G, or T, M is A or C, H is A, T, or C, N is A, T, C, or G, and V is A, C, or G.

In some embodiments, the PAM sequence recognized by the conjugate is WYR. W is A or T, Y is C or T, and R is A or G.

In some embodiments, the PAM sequence is adjacent to a target sequence on the target nucleic acid, indicating that the PAM sequence is directly covalently linked to the target sequence on the target nucleic acid with no intervening nucleotides.

In some embodiments, the PAM sequence (5'→3') recognized by the Cas12 protein is selected from any one or more of the sequences shown in FIG. 3. In some embodiments, the PAM sequence (5'→3') recognized by the Cas12 protein is selected from any one or more of the sequences shown in FIG. 4. In some embodiments, the PAM sequence (5'→3') recognized by the Cas12 protein is selected from any one or more of the sequences shown in FIG. 5. In some embodiments, the PAM sequence (5'→3') recognized by the Cas12 protein is selected from any one or more of the sequences shown in FIG. 6. In some embodiments, the PAM sequence (5'→3') recognized by the Cas12 protein is selected from any one or more of the sequences shown in FIG. 7.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 18.

In some embodiments, the Cas12 protein forms a complex with a guide polynucleotide and the complex binds to the target nucleic acid in a sequence-specific manner. In some embodiments, the complex binds to and cleaves the target nucleic acid in a sequence-specific manner, or the complex binds to the target nucleic acid in a sequence-specific manner but does not cleave the target nucleic acid.

In some embodiments, the guide polynucleotide comprises a guide sequence and a DR sequence. In some embodiments, the guide sequence is reversely complementary to the target nucleic acid, and a scaffold sequence interacts with the Cas12 protein.

In some embodiments, the PAM sequence recognized by the Cas12 protein is 5'-WYR-3'. W is A or T, Y is C or T, and R is A or G. In some embodiments, the PAM sequence recognized by the Cas12 protein is 5'-ACA-3', 5'-TCA-3', 5'-ATA-3', 5'-TTA-3', 5'-ACG-3', 5'-TCG-3', 5'-ATG-3', and/or 5'-TTG-3'.

In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 84-86, or SEQ ID NOs: 187-195. In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 84.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 18.

In some embodiments, the Cas12 protein is a non-natural or engineered protein.

In some embodiments, the Cas12 protein forms a complex with a guide polynucleotide and the complex binds to the target nucleic acid in a sequence-specific manner. In some embodiments, the complex binds to and cleaves the target nucleic acid in a sequence-specific manner, or the complex binds to the target nucleic acid in a sequence-specific manner but does not cleave the target nucleic acid. In some embodiments, the complex is non-natural or engineered complex.

In some embodiments, the guide polynucleotide comprises a guide sequence and a scaffold sequence. In some embodiments, the guide sequence is reversely complementary to the target nucleic acid, and the scaffold sequence interacts with the Cas12 protein. In some embodiments, the scaffold sequence is a DR sequence. In some embodiments, the guide sequence is located at the 5' end or the 3' end of the scaffold sequence. In some embodiments, the guide polynucleotide is non-natural or engineered polynucleotide.

In some embodiments, the PAM sequence recognized by the Cas12 protein is 5'-WYR-3'. W is A or T, Y is C or T, and R is A or G. In some embodiments, the PAM sequence recognized by the Cas12 protein is 5'-ACA-3', 5'-TCA-3', 5'-ATA-3', 5'-TTA-3', 5'-ACG-3', 5'-TCG-3', 5'-ATG-3', 5'-TTG-3', and/or 5'-TTN-3'.

In some embodiments, the scaffold sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 84-86, or SEQ ID NOs: 187-195. In some embodiments, the scaffold sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 84.

In some embodiments, the Cas12 protein has at least one mutation in at least 1, at least 2, at least 3, at least 4, at least 5, at least 6, at least 7, at least 8, at least 9, at least 10, at least 11, or at least 12 of amino acid residues corresponding to positions 1, 2, 3, 4, 5, 6, 7, 8, 9, 10, 12, 13, 14, 15, 16, 19, 21, 22, 23, 24, 25, 26, 27, 28, 29, 30, 32, 33, 34, 35, 36, 37, 38, 39, 40, 41, 42, 43, 44, 46, 47, 48, 49, 50, 51, 53, 54, 55, 56, 57, 58, 59, 60, 61, 62, 64, 65, 66, 67, 68, 69, 70, 71, 72, 73, 74, 75, 76, 77, 78, 79, 80, 81, 82, 83, 84, 85, 86, 88, 89, 90, 91, 92, 93, 94, 95, 96, 97, 98, 99, 101, 102, 103, 104, 105, 106, 108, 109, 110, 111, 112, 114, 115, 116, 117, 118, 119, 120, 121, 123, 124, 125, 126, 127, 128, 129, 130, 131, 132, 133, 134, 135, 136, 137, 138, 139, 140, 141, 142, 143, 144, 145, 146, 147, 148, 149, 151, 152, 153, 154, 155, 156, 157, 158, 159, 160, 161, 162, 163, 164, 165, 166, 167, 169, 170, 171, 172, 174, 175, 176, 177, 178, 179, 180, 182, 183, 184, 185, 186, 187, 188, 189, 190, 191, 192, 194, 195, 196, 197, 198, 199, 200, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 233, 234, 235, 236, 237, 238, 239, 240, 242, 243, 244, 245, 247, 248, 249, 250, 251, 252, 253, 255, 256, 257, 258, 259, 260, 261, 262, 263, 265, 266, 267, 268, 269, 270, 271, 272, 273, 274, 275, 276, 278, 279, 281, 282, 283, 284, 285, 286, 287, 288, 289, 290, 291, 292, 293, 294, 295, 296, 297, 298, 299, 300, 301, 302, 303, 305, 306, 308, 309, 310, 313, 315, 316, 317, 318, 319, 320, 321, 322, 323, 324, 325, 327, 328, 329, 330, 331, 332, 333, 334, 335, 336, 337, 339, 340, 341, 342, 343, 344, 345, 346, 347, 348, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 431, 432, 433, 435, 436, 437, 439, 440, 441, 442, 443, 444, 446, 447, 448, 449, 450, 451, 452, 453, 454, 455, 456, 457, 458, 459, 460, 461, 462, 463, 464, 467, 469, 470, 471, 472, 473, 474, 475, 476, 477, 478, 479, 480, 481, 482, 483, 484, 485, 486, 487, 488, 489, 490, 491, 492, 493, 494, 496, 497, 499, 500, 501, 502, 503, 504, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 546, 547, 548, 549, 550, 552, 553, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 589, 590, 592, 593, 594, 595, 596, 597, 598, 599, 601, 602, 603, 604, 605, 606, 607, 608, 609, 610, 611, 612, 613, 614, 615, 616, 618, 619, 620, 621, 622, 623, 624, 625, 626, 627, 628, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 678, 679, 680, 681, 683, 684, 685, 686, 688, 689, 691, 692, 693, 694, 695, 696, 697, 698, 699, 700, 701, 702, 703, 704, 705, 706, 707, 708, 709, 710, 711, 712, 713, 715, 716, 717, 719, 720, 721, 722, 723, 724, 725, 727, 728, 729, 730, 731, 732, 733, 734, 736, 737, 738, 739, 740, 741, 742, 743, 744, 745, 746, 747, 748, 749, 751, 752, 753, 754, 755, 756, 758, 759, 760, 761, 762, 764, 765, 766, 767, 768, 769, 771, 772, 773, 774, 775, 776, 779, 780, 781, 782, 783, 784, 785, 786, 787, 789, 790, 791, 792, 794, 795, 797, 798, 800, 801, 802, 804, 805, 806, 807, 808, 809, 810, 811, 812, 813, 814, 815, 817, 818, 819, 821, 822, 823, 824, 825, 826, 827, 828, 829, 830, 831, 832, 833, 834, 835, 836, 837, 838, 839, 840, 841, 842, 844, 845, 846, 847, 848, 849, 850, 851, 852, 853, 854, 855, 856, 857, 858, 859, 860, 862, 863, 864, 865, 866, 867, 868, 870, 872, 873, 874, 875, 876, 877, 879, 880, 881, 882, 883, 884, 885, 886, 887, 888, 890, or 891 of the amino acid sequence shown in SEQ ID NO: 18. In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 18, and the positions are selected from: W8, D9, I10, Q11, R12, C13, Q14, K15, L16, K17, L18, G19, K20, K21, Y38, F42, T54, E62, V93, A94, E95, M96, P97, Q98, A99, S100, A101, S102, S103, F104, Y105, G106, Y109, N111, Y112, S113, C114, N115, D116, K117, A118, K119, W120, T121, Q122, A123, K124, S125, F127, K142, G145, D146, S147, C148, L149, Q151, K171, W174, E175, S178, L181, A182, N183, K184, V185, N186, S187, Y189, R206, E207, S210, E214, R217, L218, Q219, V220, K221, S222, C223, Y224, Q225, K226, N227, L228, D229, H230, V233, T234, L237, S259, L262, Y263, I265, G266, T267, G268, L269, S270, K271, N272, V273, L274, R276, C280, T285, L286, A287, S288, N289, P290, T291, Y292, K293, I294, I296, Y319, K323, D326, Q327, L328, K329, R330, R331, K332, V333, Y334, P335, R336, L337, P338, S339, F340, K341, N342, D343, Y344, K345, M347, F348, L350, S351, S352, L353, K355, L376, F377, M378, N379, S380, H381, Y382, F383, N394, K395, T396, A397, K398, Q399, F404, R405, H406, K407, L408, K409, S410, A415, V416, S417, D418, I420, Y423, V424, K425, Q426, I427, G428, Q430, K431, K432, N433, G434, S435, F436, Y437, V438, T439, L440, M441, F442, T443, M444, E448, E454, R455, F456, F457, K458, T459, A460, S461, P462, D463, K466, Y467, D480, L481, N482, I483, S484, N485, P486, D522, N527, K530, R531, K533, Q534, L535, F537, K538, K540, D541, I543, K544, D545, C546, K547, F548, S549, N550, S551, N552, M556, N557, D558, A559, T560, I561, S562, F563, L564, R566, S569, P570, S571, Q572, S573, P574, R575, C576, M577, I578, Q579, T580, W581, I582, K583, N584, L585, K586, K587, L589, K590, K591, L592, H593, S594, I595, I596, R597, A598, S599, G600, Y601, V602, L608, R609, M610, L611, E612, Q614, D615, A616, M617, K618, S619, L620, I621, S622, S623, Y624, E625, R626, F627, H628, L629, K630, S631, G632, E633, M634, L635, A636, A637, K638, K639, N640, I641, T642, A643, N644, N645, R646, R647, Q648, N649, F650, R651, Q652, F653, I654, S655, R656, K657, I658, A659, S660, K661, I662, V663, Q664, Y665, S666, K667, G668, E675, D676, L677, S678, L679, D680, F681, D682, S683, D684, N685, K686, N687, N688, S689, L690, I691, R692, L693, F694, S695, A696, D697, G698, L699, K701, C702, I703, T704, D705, A706, A707, Y708, K709, A710, G711, I712, L716, P719, M720, G721, T722, S723, K724, R735, N736, L737, K738, N739, K740, N741, A756, D757, A760, H771, S772, I773, Y776, K777, F778, Y779, V780, K781, G782, K784, E794, K795, E796, V797, G798, K799, R800, L801, Q802, R803, F805, E838, N839, A840, F841, Y843, T851, A852, D853, N854, H855, or R856. In some embodiments, the mutation is a mutation to any other natural amino acid residue. In some embodiments, the mutation is a mutation to residue R, H, K, or A. In some embodiments, the mutation is a mutation to residue R. In some embodiments, the mutation is a mutation to residue A.

In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 18, and the positions are selected from N5, D9, E58, S100, N115, K142, C148, S147, K232, S245, I251, Y263, D279, A297, L300, E303, L337, M378, N394, T396, T443, K458, T468, K533, F537, F548, N550, D697, A706, or I788. In some embodiments, the mutation is a mutation to residue R.

In some embodiments, the Cas12 protein has at least one mutation at amino acid residues corresponding to any 1, any 2, or any 3 positions in the amino acid sequence shown in SEQ ID NO: 18, and the positions are selected from D480, E675, or D757. In some embodiments, the mutation is a mutation to any other natural amino acid residue. In some embodiments, the mutation is a mutation to residue A.

Table 5 and Table 6 list sites where editing activity is maintained or improved after mutation (e.g., an editing efficiency after mutation is at least 60%, at least 70%, at least 80%, at least 90%, at least 100%, at least 110%, at least 120%, at least 130%, or at least 140% of that of a wild-type protein having the amino acid sequence shown in SEQ ID NO: 18) and sites where the editing activity is significantly reduced after mutation (e.g., the editing efficiency after mutation is reduced by at least 40%, at least 50%, at least 60%, at least 70%, at least 80%, at least 90%, at least 95%, at least 96%, at least 97%, at least 98%, or at least 99% compared to that of the wild-type protein). The sites may be mutation sites of the Cas12 protein. In some embodiments, the Cas12 protein is obtained by mutating the sites to other amino acid residues. In some embodiments, the mutation is a mutation to any other natural amino acid residue. In some embodiments, the mutation is a mutation to residue R, H, K, or A. In some embodiments, the mutation is a mutation to residue R. In some embodiments, the mutation is a mutation to residue A.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 19.

In some embodiments, the Cas12 protein forms a complex with a guide polynucleotide and the complex binds to a target nucleic acid in a sequence-specific manner. In some embodiments, the complex binds to and cleaves the target nucleic acid in a sequence-specific manner, or the complex binds to the target nucleic acid in a sequence-specific manner but does not cleave the target nucleic acid.

In some embodiments, the guide polynucleotide comprises a guide sequence and a DR sequence. In some embodiments, the guide sequence is reversely complementary to a target nucleic acid, and the scaffold sequence interacts with the Cas12 protein.

In some embodiments, a PAM sequence recognized by the Cas12 protein is 5'-BMCTTH-3'.

In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 87-89.

In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 19, and the positions are selected from I1, V2, K3, P4, K5, S6, I7, K8, S9, Y10, S11, S12, M13, L14, D15, V16, D17, H18, R19, K20, N21, T62, D64, L82, P84, N120, F121, D122, K125, Y126, E159, G160, Y161, G163, L164, K165, C166, G167, K168, T169, W170, G171, T172, I173, S174, G175, L176, F177, G178, T179, G180, E181, K182, A183, D184, R185, K188, L192, R207, E224, K227, L228, Y230, G231, N232, I233, G234, R235, A236, S237, F238, V239, I240, V241, R242, E244, D253, K255, Y256, Q259, I260, K263, A266, D267, K270, Q271, D274, L275, Y294, Y295, Q296, P297, S300, E301, S303, N304, N305, L307, P308, I309, I310, Q311, G312, K313, T314, T315, K316, N317, Y318, N319, F320, Q324, Y345, F346, K349, F350, F351, T352, A353, D354, N355, V356, F357, S358, I359, C360, F362, H363, D383, E386, E387, T388, V389, S390, A391, C393, H394, I396, N397, E398, N399, G400, R401, M402, P403, I404, Y405, S406, L407, M409, E431, S434, K435, I436, E437, R438, Q439, K440, L441, N442, P443, I444, V445, E446, G447, K448, A449, S450, F451, N452, W453, G454, N455, V456, S457, K458, I459, S460, G461, C462, I463, I464, S465, K467, E468, K469, E470, K471, H472, I473, V474, S476, K477, H478, N479, H480, D481, S482, S483, I484, W485, I486, E487, T489, W497, K499, H500, H501, F502, R503, M504, F505, N506, T507, R508, F509, Y510, E511, E512, Y514, I529, S531, R532, R533, F534, F536, N537, N538, Q539, V540, V541, L542, S543, E544, D545, Q546, I547, N548, T549, I550, R551, N552, A553, S554, K555, S556, M557, R558, K559, A560, M561, K562, R563, Q564, V565, R566, D583, D584, F585, N586, I587, N588, I589, S590, N591, D592, R594, R597, T598, T599, L600, S601, Y602, K603, I604, E605, R608, V609, E610, T611, F615, D619, Q620, N621, Q622, T623, A624, R625, S659, S660, Q661, L662, V663, N664, D665, K666, S667, F668, D669, Q670, L671, Y673, D674, G675, I676, S677, W678, D679, R680, F681, Q682, S683, W684, C698, V699, S700, K701, N702, R703, K704, A705, Q706, D707, V708, P709, I710, D711, E714, I715, R718, S719, S720, K721, Y722, P724, L726, Y727, D728, R732, C734, G735, I737, K738, K739, I740, M741, K742, G743, K744, Q764, F765, S766, V767, L768, R769, L770, S771, S772, L773, N774, H775, N776, S777, F778, M780, L781, R782, N783, K785, G786, I787, I788, S789, A790, Y791, F792, N793, N794, L795, I796, G797, K798, H799, C800, T801, D802, E803, Q804, K805, F813, R816, I817, E818, L819, E820, E821, K822, R823, Q824, N825, K826, A827, I828, S829, K830, K831, N832, L833, I834, S835, N836, R837, V839, T840, V854, V856, G857, E858, N859, I860, S861, N862, T863, T864, S865, K866, S867, N868, K869, S870, K871, Q872, N873, A874, R875, A876, M877, D878, W879, L880, S881, R882, G883, V884, A885, D886, K887, Q890, M891, T892, E893, M894, H895, R899, F900, R901, D902, I903, N904, P905, A906, Y907, T908, S909, H910, Q911, H916, R917, K921, V924, M926, A928, R929, K931, E937, T939, E940, V941, D942, Y945, Y957, Y958, R1004, S1005, G1006, G1007, R1008, S1034, D1035, N1041, I1042, A1043, L1044, V1045, G1046, I1047, E1048, F1049, or E1050.

In some embodiments, the Cas12 protein has at least one mutation at amino acid residues corresponding to any 1, any 2, or any 3 positions in the amino acid sequence shown in SEQ ID NO: 19, and the positions are selected from D619, E858, or D1035. In some embodiments, the mutation is a mutation to any other natural amino acid residue. In some embodiments, the mutation is a mutation to residue A.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 20.

In some embodiments, the Cas12 protein forms a complex with a guide polynucleotide and the complex binds to a target nucleic acid in a sequence-specific manner. In some embodiments, the complex binds to and cleaves the target nucleic acid in a sequence-specific manner, or the complex binds to the target nucleic acid in a sequence-specific manner but does not cleave the target nucleic acid.

In some embodiments, the guide polynucleotide comprises a guide sequence and a DR sequence. In some embodiments, the guide sequence is reversely complementary to the target nucleic acid, and the scaffold sequence interacts with the Cas12 protein.

In some embodiments, a PAM sequence recognized by the Cas12 protein is 5'-TTN-3'.

In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 90-91.

In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 20, and the positions are selected from M1, K2, T3, L4, I5, R6, K7, T8, Y9, V10, M11, L12, V13, K19, Y30, L93, C98, K99, T100, G101, M102, K103, S104, E105, K106, D107, L108, E109, Q110, K111, L112, R113, K114, L115, D117, E118, F136, K140, I141, V143, S144, S145, L146, K147, S148, W149, D150, D151, R152, N153, V155, T156, E166, N170, A179, L180, W183, S186, N187, K188, L189, F190, L191, T192, K193, K194, V195, A196, S197, K198, F199, K200, K201, F202, G203, W204, D205, T206, Y211, V219, N220, S221, D222, A223, S224, Y225, W226, K228, M229, F230, W232, Q233, K236, R243, P244, T245, S246, L247, C248, T249, L250, P251, E252, L253, A254, V255, S256, E257, R258, E259, I260, P261, Y262, G263, V264, R277, A289, L290, R291, T292, L293, Y294, F295, K296, K304, N305, S306, Y307, Y311, R313, G314, N315, N316, A325, V326, L327, K328, E329, I330, T331, Y333, K335, N336, G337, N338, Y339, Y340, V341, G342, L343, S344, L345, N346, L347, Q348, K354, R357, T358, V359, K360, D361, Y362, Y363, F364, F365, K366, D380, L381, G382, I383, T384, N385, P386, V421, K425, K428, A429, S432, F435, A436, I438, T439, E440, L441, H442, P443, I444, K445, K446, Q451, E452, E453, W454, S455, K456, L457, R458, Y459, P460, I461, S462, Q463, M464, I465, E466, K467, L468, S469, K470, E471, M472, R473, Q474, L475, R476, R477, G478, D479, L480, N481, R483, N484, H485, G486, T487, H489, Q491, M492, Q493, F494, L496, Y498, K499, F501, V502, D503, L504, L505, K506, K507, W508, T509, Y510, F511, G512, S513, K514, P515, K518, K519, R521, R522, K523, G524, F525, E526, K527, H528, I529, R530, R531, L532, E533, N534, L535, K536, K537, D538, F539, R540, K541, K542, L543, A544, C545, E546, V548, R549, E562, D563, L564, E565, H566, F567, T568, P569, D570, S571, T572, K573, D574, S575, N576, L577, N578, E579, L580, L581, M582, L583, W584, G585, S586, G587, Q588, I589, G590, K591, W592, E594, H595, F596, Q599, Y600, K606, V607, D608, P609, R610, M611, T612, S613, Q614, I615, R625, S626, K627, Y628, D629, K630, F633, A646, D647, A650, N653, I654, R657, R661, P664, F665, K667, D682, D683, N684, S685, R686, R687, R688, H689, E719, V720, Y721, Y723, G732, K735, Y736, or R751.

In some embodiments, the Cas12 protein has at least one mutation at amino acid residues corresponding to any 1, any 2, or any 3 positions in the amino acid sequence shown in SEQ ID NO: 20, and the positions are selected from D380, E562, or D647. In some embodiments, the mutation is a mutation to any other natural amino acid residue. In some embodiments, the mutation is a mutation to residue A.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 22.

In some embodiments, the Cas12 protein forms a complex with a guide polynucleotide and the complex binds to a target nucleic acid in a sequence-specific manner. In some embodiments, the complex binds to and cleaves the target nucleic acid in a sequence-specific manner, or the complex binds to the target nucleic acid in a sequence-specific manner but does not cleave the target nucleic acid.

In some embodiments, the guide polynucleotide comprises a guide sequence and a DR sequence. In some embodiments, the guide sequence is reversely complementary to the target nucleic acid, and the scaffold sequence interacts with the Cas12 protein.

In some embodiments, a PAM sequence recognized by the Cas12 protein is 5'-VNWTV-3', 5'-VNWTC-3', or 5'-VNTTC-3'.

In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 101-114.

In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 22, and the positions are selected from M1, A2, S3, K4, H5, V6, V7, R8, P9, F10, N11, S12, V13, C14, T15, A16, K17, G18, D19, R20, L21, R22, Y23, E35, E62, L74, P76, G110, F111, D112, K115, Y116, N154, C155, D156, A157, G158, A159, G160, S161, N162, N163, A164, V165, S166, M167, L168, F169, G170, D171, G172, P173, K174, S175, D176, Y177, K180, Y222, G223, K224, T225, G226, S227, P228, S229, A230, M231, A232, R233, F234, S250, K251, K253, K254, F255, D258, K261, Q262, K265, L267, R275, E287, F288, Y289, A290, R291, A292, S294, A295, A298, N299, A301, S302, E303, I304, N305, A306, K307, F308, T309, H310, N311, C312, T313, F314, D317, Y345, M346, T348, V349, A350, E351, D352, C353, R354, Y355, V356, L357, A358, Y360, H361, E380, F383, N384, W387, E388, L391, I394, D395, F396, N397, Q398, K399, P400, P401, V402, R403, E404, L405, K407, S429, D432, R433, I434, D435, N436, V437, Y438, P439, H440, P441, F442, V443, Q444, G445, K446, Q447, G448, Y449, T450, F451, G452, P453, S454, N455, I456, E457, A459, N461, D462, M465, Q466, I467, K468, S469, I472, A473, E475, R476, P477, M478, M479, W480, V481, T482, T483, K484, D487, W491, I492, N493, H494, H495, L496, P497, F498, A499, N500, S501, R502, Y503, Y504, E505, E506, Y508, D522, G523, K524, F527, V528, L529, G530, K531, T532, I533, D534, A535, F536, A537, T538, G539, R540, I541, K542, T543, S544, V545, G546, R547, Q548, K549, A550, A551, K552, A553, I554, E555, R556, K558, D568, K570, T571, T572, F573, C574, R576, R577, K578, R581, V583, I584, A585, I586, N587, H588, R589, H590, D609, Q610, N611, E612, G613, A614, P615, S647, I648, Q649, S650, G651, K652, D653, V654, F655, Y657, S658, G659, V660, H661, D664, K665, A666, N667, G668, F669, D670, V671, L672, T685, E687, D688, A690, Y691, R694, S695, E697, W698, C699, L702, Y703, L708, R711, G714, K715, L716, I717, R718, K719, S739, P740, L741, S742, P743, V744, R745, L746, H747, S748, L749, S750, K752, S753, L754, E755, T757, K758, K759, I761, S762, C763, I764, S765, S766, Y767, F768, S769, V770, C771, N772, M773, K774, T775, V776, E777, E778, K779, Y787, W790, N791, K792, Y794, A795, S796, L797, V798, E799, R800, R801, K802, E803, R804, V805, K806, L807, S808, A809, G810, L811, I812, I813, R814, E827, G828, D829, L830, P831, T832, V833, A834, S835, G836, K837, S838, R839, Q840, N841, N842, S843, G844, K845, Q846, D847, W848, C849, A850, R851, E852, L853, K855, R856, E859, M860, A861, V863, V869, P870, V871, F872, P873, Q874, W875, T876, S877, H878, R894, S904, R906, D907, L909, A910, N913, T920, G921, T922, A923, Y925, Y926, M965, R966, G967, G968, R969, A996, D997, A1000, or V1007.

In some embodiments, the Cas12 protein has at least one mutation at amino acid residues corresponding to any 1, any 2, or any 3 positions in the amino acid sequence shown in SEQ ID NO: 22, and the positions are selected from D609, E827, or D997. In some embodiments, the mutation is a mutation to any other natural amino acid residue. In some embodiments, the mutation is a mutation to residue A.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 23.

In some embodiments, the Cas12 protein forms a complex with a guide polynucleotide and the complex binds to a target nucleic acid in a sequence-specific manner. In some embodiments, the complex binds to and cleaves the target nucleic acid in a sequence-specific manner, or the complex binds to the target nucleic acid in a sequence-specific manner but does not cleave the target nucleic acid.

In some embodiments, the guide polynucleotide comprises a guide sequence and a DR sequence. In some embodiments, the guide sequence is reversely complementary to the target nucleic acid, and the scaffold sequence interacts with the Cas12 protein.

In some embodiments, a PAM sequence recognized by the Cas12 protein is 5'-TTN-3'.

In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 115-116.

In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 23, and the positions are selected from: M1, N2, N3, K4, N5, V6, K7, S8, Y9, N10, C11, Q12, I13, L14, T15, N16, R18, K19, F22, K60, A67, E68, E69, K70, N71, T72, K73, A74, S75, K76, K77, T78, N79, K80, I101, P103, N138, F139, N140, S141, E142, K143, Y144, K178, G181, L182, K183, F184, G185, E186, I187, W188, G189, I190, V191, S192, N193, L194, F195, G196, T197, G198, D199, K200, V201, P202, K203, K206, E225, L228, Q242, Y245, L246, Y248, F249, I250, S251, G252, R253, K254, P255, S256, E257, Y258, F259, Y260, K263, K269, I270, D271, K274, V275, K278, K281, N282, K285, Y286, L290, F309, N310, Q311, K312, S315, E316, F318, N319, A320, W322, P323, I324, I325, Q326, S327, K328, T329, T330, R331, N332, L333, N334, F335, E338, Q339, F361, S364, Y365, F366, K367, T368, D369, N370, K371, F372, I373, I374, K375, K377, H378, E382, E400, K401, E404, S407, I410, E411, D412, N413, S414, S415, K416, P417, D418, L419, M420, K423, Q445, Q446, F448, K449, I450, E451, N452, R453, F454, L455, N456, P457, I458, V459, D460, N461, S462, Y463, S464, Y465, N466, W467, G468, D469, K470, S471, K472, L473, N474, C476, I477, I478, S479, K482, K483, S484, K485, F486, N487, L488, K489, N490, N491, R492, P493, D494, Y495, D496, Y497, G498, I499, W500, M501, E502, L503, E504, W511, K513, H514, H515, F516, L517, V518, S519, N520, T521, R522, F523, M524, E525, E526, Y528, F545, T547, K548, R549, N550, F552, D553, N554, N555, V556, V557, L558, S559, D560, Q561, Q562, I563, Q564, N565, I566, R567, N568, A569, P570, K571, H572, R573, R574, R575, A576, I577, K578, R579, Q580, M581, R582, N599, D600, Y601, N602, I603, N604, I605, S606, K607, S608, N610, R613, A614, I615, I616, S617, K618, K619, F620, E621, I622, E623, I624, C625, K626, V628, D635, Q636, N637, Q638, S639, A640, N641, S675, K676, Q677, A678, V679, G680, K681, N682, E683, N684, K685, R686, E687, F688, D689, Q690, L691, S692, Y693, N694, G695, I696, K697, W698, G699, E700, F701, N702, D703, N717, V718, F719, K720, V721, N722, K723, F724, G725, V726, K727, S728, N729, V730, L732, L739, N742, N743, P744, V745, L746, Y747, Y748, M751, K752, N755, K758, N759, I760, L761, Y762, K763, K764, K784, F785, S786, V787, M788, K789, L790, S791, S792, L793, S794, G795, L796, S797, F798, S799, M800, I801, R802, S803, A804, K805, S806, L807, I808, S809, S810, Y811, F812, G813, N814, L815, L816, E817, G818, T819, T820, T821, D822, D823, Q824, K825, F833, R836, Q837, K838, E840, K841, K842, R843, K844, D845, K846, Q847, K848, S849, K850, K851, E852, L853, T854, A855, N856, K857, V859, S860, E877, D878, I879, G880, N881, M882, T883, S884, N885, S886, N887, K888, N889, S890, V891, N892, S893, A894, S895, M896, D897, W898, L899, A900, R901, G902, V903, A904, N905, K906, K908, Q909, L910, M913, H914, L918, Y919, Y920, S921, I922, N923, P924, F925, M926, T927, S928, H929, Q930, H935, N936, R940, F942, K943, A944, R945, Y953, L954, F955, E956, K957, D958, T972, R973, Q974, T975, T976, Y979, K1025, M1026, G1027, G1028, R1029, A1055, D1056, A1059, A1066, K1067, G1069, K1070, N1071, E1072, T1073, S1074, S1075, or D1076.

In some embodiments, the Cas12 protein has at least one mutation at amino acid residues corresponding to any 1, any 2, or any 3 positions in the amino acid sequence shown in SEQ ID NO: 23, and the positions are selected from D635, E877, or D1056. In some embodiments, the mutation is a mutation to any other natural amino acid residue. In some embodiments, the mutation is a mutation to residue A.

In some embodiments, the Cas12 protein is an inactivated Cas12 mutant. In some embodiments, the Cas12 protein is a nuclease-inactivated mutant. In some embodiments, the Cas12 protein is a dead Cas12 mutant or a nickase Cas12 mutant. In some embodiments, the Cas12 protein has an inactivated RuvC domain.

In some embodiments, the Cas12 protein is selected from active fragments constituting the Cas12 protein as described herein.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to any one of the amino acid sequences shown in SEQ ID NOs: 18-20, SEQ ID NO: 22, or SEQ ID NO: 23.

In some embodiments, the Cas12 protein forms a complex with a guide polynucleotide and the complex specifically binds to a target nucleic acid. In some embodiments, the complex cleaves the target nucleic acid, modifies the target nucleic acid, and/or modulates an expression of the target nucleic acid.

In some embodiments, the Cas12 protein forms a complex with a guide polynucleotide, and the guide polynucleotide comprises a guide sequence that is reversely complementary to a target nucleic acid. In some embodiments, the guide polynucleotide further comprises a scaffold sequence that interacts with the Cas12 protein. In some embodiments, the scaffold sequence comprises a DR sequence. In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 84-91, SEQ ID NOs: 101-116, or SEQ ID NOs: 187-195.

In some embodiments, the scaffold sequence does not comprise a tracrRNA sequence.

In some embodiments, the PAM sequence recognized by the Cas12 protein (5'→3') is selected from any one or more of the following: WYR, BMCTTH, TTN, VNWTV, VNWTC, or VNTTC. W is A or T, Y is C or T, R is A or G, B is C, G, or T, M is A or C, H is A, T, or C, N is A, T, C, or G, V is A, C, or G.

In some embodiments, the reverse complementation is partially complementary or completely complementary. In some embodiments, the guide sequence hybridizes to the target nucleic acid.

In some embodiments, the Cas12 protein is a mutant of a Cas protein having an amino acid sequence shown in any one of SEQ ID NOs: 1-35.

In some embodiments, the Cas12 protein is an inactivated mutant of a Cas protein having an amino acid sequence shown in any one of SEQ ID NOs: 1-35.

In some embodiments, compared to the Cas12 protein having an amino acid sequence shown in any one of SEQ ID NOs: 1-35, the Cas12 protein provided herein comprises one, two, or more mutations, e.g., a single amino acid insertion, a single amino acid deletion, a single amino acid substitution, or a combination thereof. In some embodiments, compared to the Cas12 protein having an amino acid sequence shown in any one of SEQ ID NOs: 1-35, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions) while retaining the ability to bind to a target nucleic acid molecule complementary to a guide sequence of a guide polynucleotide, and/or retaining the ability to process an RNA transcript containing a guide sequence into guide polynucleotide molecules. In some embodiments, compared to the Cas12 protein having an amino acid sequence shown in any one of SEQ ID NOs: 1-35, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions), while retaining the ability to bind to a target nucleic acid molecule complementary to a guide sequence of a guide polynucleotide.

In another aspect, one or more embodiments of the present disclosure provide a guide polynucleotide, and the guide polynucleotide comprises (i) a DR sequence having at least 50% sequence identity to the sequence shown in any one of SEQ ID NOs: 36-170 or SEQ ID NOs:187-195, and (ii) a guide sequence engineered to hybridize to a target nucleic acid. The DR sequence is linked to the guide sequence, and the guide polynucleotide forms a complex with a Cas12 protein and guides sequence-specific binding of the complex to the target nucleic acid.

In some embodiments, the DR sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 36-170 or SEQ ID NOs:187-195.

In some embodiments, the DR sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 84.

In some embodiments, the DR sequence has at least 60% sequence identity to any one of the sequences shown in SEQ ID NOs: 36-170 or SEQ ID NOs: 187-195.

In some embodiments, the DR sequence has at least 65% sequence identity to any one of the sequences shown in SEQ ID NOs: 36-170 or SEQ ID NOs: 187-195.

In some embodiments, the DR sequence has at least 70% sequence identity to any one of the sequences shown in SEQ ID NO: 36-170 or SEQ ID NOs: 187-195.

In some embodiments, the DR sequence has at least 75% sequence identity to any one of the sequences shown in SEQ ID NO: 36-170 or SEQ ID NOs: 187-195.

In some embodiments, the DR sequence has at least 80% sequence identity to any one of the sequences shown in SEQ ID NO: 36-170 or SEQ ID NO: 187-195.

In some embodiments, the DR sequence has at least 85% sequence identity to any one of the sequences shown in SEQ ID NOs: 36-170 or SEQ ID NOs: 187-195.

In some embodiments, the DR sequence has at least 90% sequence identity to any one of the sequences shown in SEQ ID NO: 36-170 or SEQ ID NO: 187-195.

In some embodiments, the DR sequence has at least 95% sequence identity to any one of the sequences shown in SEQ ID NO: 36-170 or SEQ ID NO: 187-195.

In some embodiments, the DR sequence has at least 96% sequence identity to any one of the sequences shown in SEQ ID NO: 36-170 or SEQ ID NO: 187-195.

In some embodiments, the DR sequence has at least 97% sequence identity to any one of the sequences shown in SEQ ID NO: 36-170 or SEQ ID NO: 187-195.

In some embodiments, the DR sequence has at least 98% sequence identity to any one of the sequences shown in SEQ ID NO: 36-170 or SEQ ID NO: 187-195.

In some embodiments, the DR sequence has 100% sequence identity to any one of the sequences shown in SEQ ID NO: 36-170 or SEQ ID NO: 187-195.

In some embodiments, the Cas12 protein is a Cas12 protein as described herein.

In some embodiments, the guide sequence comprises 15-60 nucleotides. In some embodiments, the guide sequence comprises 15-50 nucleotides. In some embodiments, the guide sequence comprises 15-40 nucleotides. In some embodiments, the guide sequence comprises 15-35 nucleotides. In some embodiments, the guide sequence comprises 15-30 nucleotides. In some embodiments, the guide sequence comprises 15-25 nucleotides. In some embodiments, the guide sequence comprises 18-25 nucleotides. In some embodiments, the guide sequence comprises 20-25 nucleotides. In some embodiments, the guide sequence comprises 18-22 nucleotides. In some embodiments, the guide sequence comprises 20-22 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, or 40 nucleotides.

In some embodiments, the guide sequence hybridizes to the target nucleic acid, and the guide sequence is 90%-100% complementary to the target nucleic acid.

In some embodiments, the guide sequence hybridizes to the target nucleic acid.

In some embodiments, the guide sequence hybridizes to the target nucleic acid, and the guide sequence is mismatched to the target nucleic acid by no more than one nucleotide.

In some embodiments, the DR sequence comprises 15-100 nucleotides. In some embodiments, the DR sequence comprises 15-90 nucleotides. In some embodiments, the DR sequence comprises 15-80 nucleotides. In some embodiments, the DR sequence comprises 15-70 nucleotides. In some embodiment, the DR sequence comprises 15-60 nucleotides. In some embodiments, the guide sequence comprises 15-50 nucleotides. In some embodiments, the guide sequence comprises 15-40 nucleotides. In some embodiments, the guide sequence comprises 20-40 nucleotides. In some embodiments, the guide sequence comprises 20-30 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides.

In some embodiments, the guide sequence is located at the 3' end of the DR sequence.

In some embodiments, the guide sequence is located at the 5' end of the DR sequence.

In some embodiments, the guide polynucleotide further comprises a tracrRNA.

In some embodiments, the tracrRNA is complementarily paired with the DR sequence. In general, the complementary pairing is complementary pairing for partial bases. In some embodiments, the tracrRNA interacts with the DR sequence.

In some embodiments, the tracrRNA sequence is linked to the DR sequence. In some embodiments, the tracrRNA sequence is linked to the DR sequence via a nucleotide sequence. In some embodiments, the tracrRNA sequence is linked to the DR sequence via a nucleotide sequence consisting of 1-10 nucleotides. In some embodiments, the tracrRNA sequence is linked to the DR sequence via a nucleotide sequence consisting of 1, 2, 3, 4, 5, 6, 7, 8, 9, or 10 nucleotides. In some embodiments, the tracrRNA sequence is linked to the DR sequence via a nucleotide sequence consisting of 4 nucleotides. In some embodiments, the tracrRNA sequence is linked to the DR sequence via a 5'-GAAA-3' sequence.

In some embodiments, the tracrRNA sequence is located at the 3' end of the DR sequence.

In some embodiments, the tracrRNA sequence is located at the 5' end of the DR sequence.

In some embodiments, the tracrRNA comprises 10-200 nucleotides. In some embodiments, the tracrRNA comprises 10-190, 10-180, 10-170, 10-160, 10-150, 10-140, 10-130, 10-120, 10-110, 10-100, 10-90, 10-80, 10-70, 10-60, 10-50, 10-40, 10-30, 10-20, 10-100, 10-100, 10-100, 10-100, 10-100, 10-100, 10-100, 20-100, 30-100, 40-100, 20-90, 20-80, 20-70, 20-60, 20-50, or 30-50 nucleotides. In some embodiments, the tracrRNA comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 nucleotides.

Table 1 shows amino acid sequences of the Cas proteins.

Table 2 shows DR sequences corresponding to the Cas proteins. When more than one DR sequences corresponding to a particular Cas protein are listed, any one DR sequence may be selected for use.

In another aspect, one or embodiments of the present disclosure provide an inactivated Cas12 mutant, and the inactivated Cas12 mutant is a nuclease-inactivated mutant of the Cas12 protein as described herein.

In the present disclosure, depending on the context, a reference scope of the Cas12 protein may encompass the inactivated Cas12 mutant. However, given the importance of the inactivated Cas12 mutant (non-limiting examples including fusion of the inactivated Cas12 mutant with a deaminase for single-base editing, fusion with a transcriptional activation domain or a transcriptional repression domain for transcriptional regulation, etc.), the inactivated Cas12 mutant is described separately and in detail herein, which does not imply that the reference scope of the Cas12 protein necessarily excludes the inactivated Cas12 mutant.

In some embodiments, the inactivated Cas12 mutant is selected from the Cas12 proteins as described herein.

In some embodiments, the inactivated Cas12 mutant is a mutant in which the nuclease activity is completely inactivated, i.e., a dead Cas12 mutant (dCas12). The dCas12 only binds to a target nucleic acid under a mediation of a guide polynucleotide, and has no or negligible cleaving activity against the target nucleic acid. For example, a target nucleic acid cleavage efficiency of the dCas12 is no more than 20%, 15%, 10%, 5%, 4%, 3%, 2%, or 1% of a target nucleic acid cleavage efficiency of the Cas12 protein before the inactivating mutation.

In some embodiments, the inactivated Cas12 mutant is a mutant in which the nuclease activity is partially inactivated. Further, the mutant with partially inactivated nuclease activity is a nickase Cas12 (nCas12), which binds to a target nucleic acid under a mediation of a guide polynucleotide, then cleaves one single strand of a double-stranded target nucleic acid without cleaving the other single strand.

In some embodiments, the inactivated Cas12 mutant is a Cas12 protein with an inactivated RuvC domain.

In some embodiments, the inactivated Cas12 mutant is a Cas12 protein within an inactivated RuvC-I, RuvC-II, or RuvC-III domain.

In some embodiments, the inactivated Cas12 mutant is obtained by introducing an inactivating mutation into the RuvC-I, RuvC-II, or RuvC-III domain of the Cas12 protein.

In some embodiments, the inactivated Cas12 mutant comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to any one of the amino acid sequences shown in SEQ ID NOs: 18-20, SEQ ID NO: 22, or SEQ ID NOs: 23.

In some embodiments, the inactivated Cas12 mutant forms a complex with a guide polynucleotide and the complex specifically binds to a target nucleic acid. In some embodiments, the complex cleaves the target nucleic acid, modifies the target nucleic acid, and/or modulates an expression of the target nucleic acid.

In some embodiments, the inactivated Cas12 mutant forms a complex with a guide polynucleotide and the guide polynucleotide comprises a guide sequence that is reversely complementary to a target nucleic acid. In some embodiments, the guide polynucleotide further comprises a scaffold sequence that interacts with the Cas12 protein. In some embodiments, the scaffold sequence comprises a DR sequence. In some embodiments, the DR sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 84-91, SEQ ID NOs: 101-116, or SEQ ID NOs: 187-195. In some embodiments, the scaffold sequence does not comprise a tracrRNA sequence.

In some embodiments, a PAM sequence (5'→3') recognized by the inactivated Cas12 mutant is selected from any one or more of the following: WYR, BMCTTH, TTN, VNWTV, VNWTC, or VNTTC. W is A or T, Y is C or T, R is A or G, B is C, G, or T, M is A or C, H is A, T, or C, N is A, T, C, or G, V is A, C, or G.

In some embodiments, the reverse complementation is partially complementary or completely complementary. In some embodiments, the guide sequence hybridizes to the target nucleic acid.

In some embodiments, the inactivated Cas12 mutant comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 18.

In some embodiments, the inactivated Cas12 mutant has at least one mutation at amino acid residues corresponding to any 1, any 2, or any 3 positions in the amino acid sequence shown in SEQ ID NO: 18, and the positions are selected from D480, E675, or D757. In some embodiments, the mutation is a mutation to any other natural amino acid residue. In some embodiments, the mutation is a mutation to residue A.

In some embodiments, the inactivated Cas12 mutant comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 19.

In some embodiments, the inactivated Cas12 mutant has at least one mutation at amino acid residues corresponding to any 1, any 2, or any 3 positions in the amino acid sequence shown in SEQ ID NO: 19, and the positions are selected from D619, E858, or D1035. In some embodiments, the mutation is a mutation to any other natural amino acid residue. In some embodiments, the mutation is a mutation to residue A.

In some embodiments, the inactivated Cas12 mutant comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 20.

In some embodiments, the inactivated Cas12 mutant has at least one mutation at amino acid residues corresponding to any 1, any 2, or any 3 positions in the amino acid sequence shown in SEQ ID NO: 20, and the positions are selected from D380, E562, or D647. In some embodiments, the mutation is a mutation to any other natural amino acid residue. In some embodiments, the mutation is a mutation to residue A.

In some embodiments, the inactivated Cas12 mutant comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 22.

In some embodiments, the inactivated Cas12 mutant has at least one mutation at amino acid residues corresponding to any 1, any 2, or any 3 positions in the amino acid sequence shown in SEQ ID NO: 22, and the positions are selected from D609, E827, or D997. In some embodiments, the mutation is a mutation to any other natural amino acid residue. In some embodiments, the mutation is a mutation to residue A.

In some embodiments, the inactivated Cas12 mutant comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 23.

In some embodiments, the inactivated Cas12 mutant has at least one mutation at amino acid residues corresponding to any 1, any 2, or any 3 positions in the amino acid sequence shown in SEQ ID NO: 23, and the positions are selected from D635, E877, or D1056. In some embodiments, the mutation is a mutation to any other natural amino acid residue. In some embodiments, the mutation is a mutation to residue A.

In some embodiments, the PAM sequence recognized by the inactivated Cas12 mutant is the same as the PAM sequence recognized by the Cas12 protein.

In some embodiments, the PAM sequence (5'→3') recognized by the inactivated Cas12 mutant is selected from any one or more of the following:
A, C, T, G,
TA, TC, GN, AA, AG, TG, AN, GG, CG, TN, NT, NG, GT, NA, CC, AC, GC, AT, CT, GA, TT, CN, NC, CA,
NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG,
NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, or NNGC.

N is A, T, C, or G.

In some embodiments, the PAM sequence (5'→3') recognized by the inactivated Cas12 mutant is selected from any one, two, or more of the following degenerate sequences or non-degenerate sequences (the non-degenerate sequence refers to any specific sequence encompassed by the degenerate sequence): WYR, BMCTTH, TTN, VNWTV, VNWTC, or VNTTC. W is A or T, Y is C or T, R is A or G, B is C, G, or T, M is A or C, H is A, T, or C, N is A, T, C, or G, V is A, C, or G.

In another aspect, one or more embodiments of the present disclosure provide a fusion protein or conjugate. The fusion protein or conjugate comprises: (a) the Cas12 protein, or the inactivated Cas12 mutant as described herein; and (b) a homologous or heterologous functional domain.

In the present disclosure, depending on the context, a reference scope of the Cas12 protein may encompass the inactivated Cas12 mutant. However, given the importance of the inactivated Cas12 mutant (non-limiting examples including the fusion of the inactivated Cas12 mutant with a deaminase for single-base editing, fusion with a transcriptional activation domain or a transcriptional repression domain for transcriptional regulation, etc.), the inactivated Cas12 mutant is described separately and in detail herein, which does not imply that the reference scope of the Cas12 protein necessarily excludes the inactivated Cas12 mutant.

In some embodiments, a fusion protein is provided. The fusion protein comprises: (a) the Cas12 protein, or the inactivated Cas12 mutant as described herein; and (2) a homologous or heterologous functional domain.

In some embodiments, a fusion protein is provided. The fusion protein comprises: (a) the Cas12 protein as described herein; and (b) a homologous or heterologous functional domain.

In some embodiments, a conjugate is provided. The conjugate comprises: (a) the Cas12 protein, or the inactivated Cas12 mutant as described herein; and (b) a homologous or heterologous functional domain.

In some embodiments, a conjugate is provided. The conjugate comprises: (a) the Cas12 protein as described herein; and (b) a homologous or heterologous functional domain.

In some embodiments, the functional domain has an epigenetic modification activity. The epigenetic modification comprises, but is not limited to, DNA methylation, RNA methylation, RNA interference, nucleosome positioning, chromatin conformation alteration, chromatin remodeling, histone modification, modification of long non-coding RNA sequences, etc.

In some embodiments, the functional domain has an enzymatic activity for modifying a target nucleic acid sequence. For example, the enzymatic activity comprises a nuclease activity, a methyltransferase activity, a demethylase activity, a DNA nucleotide methyltransferase activity, a DNA nucleotide demethylase activity, a base deaminase activity, a DNA repair activity, a DNA damage activity, a deaminase activity, a dismutase activity, an alkylation activity, a depurination activity, an oxidation activity, a pyrimidine dimer formation activity, an integrase activity, a transposase activity, a recombinase activity, a polymerase activity, a ligase activity, a helicase activity, a photolyase activity, a glycosylase activity, a deglycosylation activity, an acetyltransferase activity, a deacetylase activity, a histone acetyltransferase activity, a histone deacetylase activity, a kinase activity, a phosphatase activity, an ubiquitin ligase activity, a deubiquitination activity, an adenylation activity, a deadenylation activity, a SUMOylating activity, a deSUMOylating activity, a myristoylation activity, and/or a demyristoylation activity.

In some embodiments, the functional domain has a single-base editing activity. In some embodiments, the functional domain is a single-base editing functional domain. In some embodiments, the functional domain is a base conversion enzyme. In some embodiments, the functional domain or the base conversion enzyme is a base deaminase. In some embodiments, the functional domain or the base conversion enzyme is an adenine deaminase or a cytosine deaminase.

In some embodiments, the functional domain is selected from one or more of the following: a nuclease (e.g., FokI), a methyltransferase, a demethylase, a DNA repair enzyme, a DNA damage enzyme, a deaminase, a dismutase, an alkylase, a depurination enzyme, an oxidase, a pyrimidine dimer-forming enzyme, an integrase, a transposase, a recombinase, a polymerase, a ligase, a helicase, a photolyase, a glycosylase, a deglycosylase, an acetyltransferase, a deacetylase, a kinase, a phosphatase, a ubiquitin ligase, a deubiquitinating enzyme, an adenylating enzyme, a deadenylase, a SUMOylating enzyme, a deSUMOylating enzyme, a myristoylating enzyme, and/or a demyristoylating enzyme.

In some embodiments, the homologous or heterologous functional domain is selected from any one, two, three, four, or more of the following: a subcellular localization signal, a DNA-binding domain, a protease domain, a transcriptional activation domain, a transcriptional repression domain, a nuclease domain, a deaminase domain, a uracil DNA glycosylase domain (UDG), a uracil DNA glycosylase inhibitor domain (UGI), a methyltransferase, a demethylase, a transcription release factor, a histone acetyltransferase domain, a histone deacetylase domain, a DNA ligase, an affinity tag, a reporter tag, an affinity domain, or a reporter domain.

In some embodiments, the subcellular localization signal is selected from a nuclear localization signal, a nuclear export signal, a mitochondrial localization signal, or a chloroplast localization signal.

In some embodiments, the fusion protein or conjugate comprises 1, 2, 3, 4, 5, 6, 7, 8, 9, or more homologous or heterologous functional domains, and the functional domains are the same or different.

In some embodiments, the fusion protein or conjugate connects 0, 1, 2, 3, 4, 5, 6, 7, 8, or more functional domains at the N-terminus and/or C-terminus of the Cas12 protein.

In some embodiments, the fusion protein comprises 1, 2, 3, 4, or more nuclear localization signals.

In some embodiments, the fusion protein is used to achieve base editing, e.g., in conjunction with a guide polynucleotide to achieve base editing. In some embodiments, the fusion protein comprises a nuclear localization signal and a deaminase domain.

In some embodiments, the fusion protein comprises a nuclear localization signal, a cytidine deaminase domain, and optionally one or two UGI domains. The fusion protein is used to achieve C→T base editing of a target nucleic acid.

In some embodiments, the fusion protein comprises a nuclear localization signal and an adenosine deaminase domain. The fusion protein is used to achieve A→G base editing of a target nucleic acid.

In some embodiments, the fusion protein comprises a nuclear localization signal, a cytidine deaminase domain, and an adenosine deaminase domain. In some embodiments, the fusion protein comprises 1, 2, or 3 nuclear localization signals and a deaminase domain. In some embodiments, the fusion protein comprises a UGI domain. In some embodiments, the fusion protein comprises 1, 2, or 3 nuclear localization signals, a deaminase domain, and 1 or 2 UGI domains.

In some embodiments, the fusion protein is used to achieve transcriptional activation of a specific target gene, e.g., in conjunction with a guide polynucleotide for achieving transcriptional activation of a specific target gene. In some embodiments, the fusion protein comprises a nuclear localization signal and a transcriptional activation domain.

In some embodiments, the fusion protein is used to achieve transcriptional repression of a specific target gene, e.g., in conjunction with a guide polynucleotide for achieving the transcriptional repression of the specific target gene. In some embodiments, the fusion protein comprises a nuclear localization signal and a transcriptional repression domain.

In some embodiments, the fusion protein is used to achieve methylation of a specific target sequence, e.g., in conjunction with a guide polynucleotide for achieving the methylation of the specific target sequence. In some embodiments, the fusion protein comprises a nuclear localization signal and a DNA methylation domain.

In some embodiments, the fusion protein is used to achieve demethylation of a specific target sequence, e.g., in conjunction with a guide polynucleotide for achieving the demethylation of the specific target sequence. In some embodiments, the fusion protein comprises a nuclear localization signal and a DNA demethylation domain.

In some embodiments, the nuclease domain comprises a polypeptide with a single-stranded DNA (ssDNA) cleavage activity and/or a polypeptide with a double-stranded (dsDNA) cleavage activity.

In some embodiments, the nuclease domain comprises a polypeptide with an ssDNA cleavage activity.

In some embodiments, the nuclease domain comprises a polypeptide with a dsDNA cleavage activity.

In some embodiments, the Cas12 protein or the inactivated mutant is directly or indirectly linked to the homologous or heterologous functional domain.

In some embodiments, the direct linkage is a covalent linkage, and the indirect linkage is a linkage via an amino acid linker or a non-amino acid linker.

In some embodiments, the homologous or heterologous functional domain is fused or conjugated to the N-terminus, C-terminus, or internal region of the Cas12 protein or the inactivated mutant.

In the present disclosure, the fusion protein is obtained by linking the element (a) to the element (b) via a peptide linker or directly linking the element (a) to the element (b), and the conjugate is obtained by linking the element (a) and the element (b) via a non-peptide chemical bond.

In some embodiments, a PAM sequence recognized by the fusion protein or conjugate is the same as the PAM sequence recognized by the Cas12 protein.

In some embodiments, a PAM sequence (5'→3') recognized by the fusion protein or conjugate is optionally selected from any one or more of the following:
A, C, T, G,
TA, TC, GN, AA, AG, TG, AN, GG, CG, TN, NT, NG, GT, NA, CC, AC, GC, AT, CT, GA, TT, CN, NC, CA,
NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG,
NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, or NNGC.

N is A, T, C, or G.

In some embodiments, the PAM sequence (5'→3') recognized by the fusion protein is selected from any one, two, or more of the following degenerate sequences or non-degenerate sequences (the non-degenerate sequence refers to any specific sequence encompassed by the degenerate sequence): WYR, BMCTTH, TTN, VNWTV, VNWTC, or VNTTC. W is A or T, Y is C or T, R is A or G, B is C, G, or T, M is A or C, H is A, T, or C, N is A, T, C, or G, V is A, C, or G.

In some embodiments, the PAM sequence recognized by the fusion protein is 5'-WYR-3'.

In some embodiments, the PAM sequence recognized by the conjugate is 5'-WYR-3'.

In some embodiments, the PAM sequence (5'→3') recognized by the conjugate is selected from any one, two, or more of the following degenerate sequences or non-degenerate sequences (the non-degenerate sequence refers to any specific sequence encompassed by the degenerate sequence): WYR, BMCTTH, TTN, VNWTV, VNWTC, or VNTTC. W is A or T, Y is C or T, R is A or G, B is C, G, or T, M is A or C, H is A, T, or C, N is A, T, C, or G, V is A, C, or G.

In some embodiments, a fusion protein is provided. The fusion protein comprises a Cas12 protein and a homologous or heterologous functional domain.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 18.

In some embodiments, the Cas12 protein is a non-natural or engineered protein.

In some embodiments, the Cas12 protein forms a complex with a guide polynucleotide and the complex binds to a target nucleic acid in a sequence-specific manner. In some embodiments, the complex binds to and cleaves a target nucleic acid in a sequence-specific manner, or the complex binds to a target nucleic acid in a sequence-specific manner but does not cleave the target nucleic acid. In some embodiments, the complex is a non-natural or engineered complex.

In some embodiments, the guide polynucleotide comprises a guide sequence and a scaffold sequence. In some embodiments, the guide sequence is reversely complementary to the target nucleic acid, and the scaffold sequence interacts with the Cas12 protein. In some embodiments, the scaffold sequence is a DR sequence. In some embodiments, the guide sequence is located at the 5' end or a 3' end of the scaffold sequence. In some embodiments, the guide polynucleotide is a non-natural or engineered polynucleotide.

In some embodiments, a PAM sequence recognized by the Cas12 protein is 5'-WYR-3'. W is A or T, Y is C or T, R is A or G. In some embodiments, a PAM sequence recognized by the Cas12 protein is 5'-ACA-3', 5'-TCA-3', 5'-ATA-3', 5'-TTA-3', 5'-ACG-3', 5'-TCG-3', 5'-ATG-3', 5'-TTG-3', and/or 5'-TTN-3'.

In some embodiments, the scaffold sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NOs: 84-86 or SEQ ID NOs: 187-195. In some embodiments, the scaffold sequence comprises a sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 84.

In some embodiments, the Cas12 protein has at least one mutation in at least 1, at least 2, at least 3, at least 4, at least 5, at least 6, at least 7, at least 8, at least 9, at least 10, at least 11, or at least 12 of amino acid residues corresponding to positions 1, 2, 3, 4, 5, 6, 7, 8, 9, 10, 12, 13, 14, 15, 16, 19, 21, 22, 23, 24, 25, 26, 27, 28, 29, 30, 32, 33, 34, 35, 36, 37, 38, 39, 40, 41, 42, 43, 44, 46, 47, 48, 49, 50, 51, 53, 54, 55, 56, 57, 58, 59, 60, 61, 62, 64, 65, 66, 67, 68, 69, 70, 71, 72, 73, 74, 75, 76, 77, 78, 79, 80, 81, 82, 83, 84, 85, 86, 88, 89, 90, 91, 92, 93, 94, 95, 96, 97, 98, 99, 101, 102, 103, 104, 105, 106, 108, 109, 110, 111, 112, 114, 115, 116, 117, 118, 119, 120, 121, 123, 124, 125, 126, 127, 128, 129, 130, 131, 132, 133, 134, 135, 136, 137, 138, 139, 140, 141, 142, 143, 144, 145, 146, 147, 148, 149, 151, 152, 153, 154, 155, 156, 157, 158, 159, 160, 161, 162, 163, 164, 165, 166, 167, 169, 170, 171, 172, 174, 175, 176, 177, 178, 179, 180, 182, 183, 184, 185, 186, 187, 188, 189, 190, 191, 192, 194, 195, 196, 197, 198, 199, 200, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 233, 234, 235, 236, 237, 238, 239, 240, 242, 243, 244, 245, 247, 248, 249, 250, 251, 252, 253, 255, 256, 257, 258, 259, 260, 261, 262, 263, 265, 266, 267, 268, 269, 270, 271, 272, 273, 274, 275, 276, 278, 279, 281, 282, 283, 284, 285, 286, 287, 288, 289, 290, 291, 292, 293, 294, 295, 296, 297, 298, 299, 300, 301, 302, 303, 305, 306, 308, 309, 310, 313, 315, 316, 317, 318, 319, 320, 321, 322, 323, 324, 325, 327, 328, 329, 330, 331, 332, 333, 334, 335, 336, 337, 339, 340, 341, 342, 343, 344, 345, 346, 347, 348, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 431, 432, 433, 435, 436, 437, 439, 440, 441, 442, 443, 444, 446, 447, 448, 449, 450, 451, 452, 453, 454, 455, 456, 457, 458, 459, 460, 461, 462, 463, 464, 467, 469, 470, 471, 472, 473, 474, 475, 476, 477, 478, 479, 480, 481, 482, 483, 484, 485, 486, 487, 488, 489, 490, 491, 492, 493, 494, 496, 497, 499, 500, 501, 502, 503, 504, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 546, 547, 548, 549, 550, 552, 553, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 589, 590, 592, 593, 594, 595, 596, 597, 598, 599, 601, 602, 603, 604, 605, 606, 607, 608, 609, 610, 611, 612, 613, 614, 615, 616, 618, 619, 620, 621, 622, 623, 624, 625, 626, 627, 628, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 678, 679, 680, 681, 683, 684, 685, 686, 688, 689, 691, 692, 693, 694, 695, 696, 697, 698, 699, 700, 701, 702, 703, 704, 705, 706, 707, 708, 709, 710, 711, 712, 713, 715, 716, 717, 719, 720, 721, 722, 723, 724, 725, 727, 728, 729, 730, 731, 732, 733, 734, 736, 737, 738, 739, 740, 741, 742, 743, 744, 745, 746, 747, 748, 749, 751, 752, 753, 754, 755, 756, 758, 759, 760, 761, 762, 764, 765, 766, 767, 768, 769, 771, 772, 773, 774, 775, 776, 779, 780, 781, 782, 783, 784, 785, 786, 787, 789, 790, 791, 792, 794, 795, 797, 798, 800, 801, 802, 804, 805, 806, 807, 808, 809, 810, 811, 812, 813, 814, 815, 817, 818, 819, 821, 822, 823, 824, 825, 826, 827, 828, 829, 830, 831, 832, 833, 834, 835, 836, 837, 838, 839, 840, 841, 842, 844, 845, 846, 847, 848, 849, 850, 851, 852, 853, 854, 855, 856, 857, 858, 859, 860, 862, 863, 864, 865, 866, 867, 868, 870, 872, 873, 874, 875, 876, 877, 879, 880, 881, 882, 883, 884, 885, 886, 887, 888, 890, or 891 of the amino acid sequence shown in SEQ ID NO: 18. In some embodiments, the mutation is a mutation to any other natural amino acid residue. In some embodiments, the mutation is a mutation to residue R, H, K, or A. In some embodiments, the mutation is a mutation to residue R. In some embodiments, the mutation is a mutation to residue A.

In some embodiments, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 18, and the positions are selected from N5, D9, E58, S100, N115, K142, C148, S147, K232, S245, I251, Y263, D279, A297, L300, E303, L337, M378, N394, T396, T443, K458, T468, K533, F537, F548, N550, D697, A706, or I788. In some embodiments, the mutation is a mutation to residue R.

In some embodiments, the functional domain has an epigenomic modification activity. In some embodiments, the functional domain has an epigenetic modification activity. In some embodiments, the epigenomic modification or the epigenetic modification comprises, but is not limited to, DNA methylation, RNA methylation, RNA interference, nucleosome positioning, chromatin conformation alteration, chromatin remodeling, histone modification, and modification of long non-coding RNA sequences.

In some embodiments, the functional domain is an epigenomic modification functional domain. In some embodiments, the functional domain is an epigenetic modification functional domain.

In some embodiments, the functional domain is selected from one or more of the following: a nuclease (e.g., FokI), a DNA methyltransferase, a DNA demethylase, a histone methyltransferase, a histone demethylase, a DNA repair enzyme, a DNA damage enzyme, a base deaminase (comprising, but not limited to, an adenine deaminase, a cytosine deaminase), a dismutase, an alkylase, a depurination enzyme, an oxidase, a pyrimidine dimer-forming enzyme, an integrase, a transposase, a recombinase, a polymerase, a ligase, a helicase, a photolyase, a glycosylase, a deglycosylase, an acetyltransferase, a deacetylase, a kinase, a phosphatase, a ubiquitin ligase, a deubiquitinase, an adenylase, a deadenylase, a SUMOylating enzyme, a deSUMOylating enzyme, a myristoylase, and/or a demyristoylase. In some embodiments, the functional domain is an adenine deaminase or a cytosine deaminase.

In some embodiments, the epigenomic modification and the epigenetic modification are used interchangeably.

Table 5 and Table 6 list sites where editing activity is maintained or improved after mutation (e.g., an editing efficiency after mutation is at least 60%, at least 70%, at least 80%, at least 90%, at least 100%, at least 110%, at least 120%, at least 130%, or at least 140% of that of a wild-type protein having the amino acid sequence shown in SEQ ID NO: 18) and sites where editing activity is significantly reduced after mutation (e.g., an editing efficiency after mutation is reduced by at least 40%, at least 50%, at least 60%, at least 70%, at least 80%, at least 90%, at least 95%, at least 96%, at least 97%, at least 98%, or at least 99% compared to that of the wild-type protein having the amino acid sequence shown in SEQ ID NO: 18). The sites may be mutation sites of the Cas12 protein. In some embodiments, the fusion protein is obtained by mutating the sites to other amino acid residues.

In another aspect, one or more embodiments of the present disclosure provide an isolated nucleic acid, the isolated nucleic acid encodes the Cas12 protein, the inactivated Cas12 mutant, or the fusion protein or conjugate as described herein.

In some embodiments, the isolated nucleic acid encodes the Cas12 protein or the fusion protein as described herein.

In some embodiments, the isolated nucleic acid is a DNA or RNA sequence. In some embodiments, the isolated nucleic acid comprises modification. In some embodiments, the isolated nucleic acid comprises a modified nucleotide. In some embodiments, the isolated nucleic acid is a DNA sequence and comprises an RNA base modification. In some embodiments, the isolated nucleic acid is an RNA sequence and comprises a DNA base modification. In some embodiments, the isolated nucleic acid is a messenger RNA (mRNA).

In some embodiments, the isolated nucleic acid comprises a biocompatible natural or non-natural nucleotide modification.

In some embodiments, the isolated nucleic acid comprises any one, two, or more of the following nucleotide modifications: 2'-O-methylation, pseudouridine (Ψ), N⁶-methyladenosine (m⁶A), 5-methylcytidine (m⁵C), 7-methylguanosine (m⁷G), 1-methyladenosine (m¹A), or 5-hydroxymethylcytidine (5hmC).

In some embodiments, at least 30%, at least 40%, at least 50%, at least 60%, at least 70%, at least 80%, at least 90%, at least 95%, at least 96%, at least 97%, at least 98%, at least 99%, or 100% of uracil in the isolated nucleic acid are replaced with pseudouridine. In some embodiments, the isolated nucleic acid is an mRNA, and at least 30%, at least 40%, at least 50%, at least 60%, at least 70%, at least 80%, at least 90%, at least 95%, at least 96%, at least 97%, at least 98%, at least 99%, or 100% of uracil in the mRNA are replaced with pseudouridine.

In some embodiments, the isolated nucleic acid is an mRNA, and the mRNA comprises modification. In some embodiments, the isolated nucleic acid is an mRNA, and the mRNA comprises a modified nucleotide. In some embodiments, the isolated nucleic acid is an mRNA, and the mRNA comprises a cap structure located at the 5' end. In some embodiments, the isolated nucleic acid is an mRNA, and the mRNA comprises a Cap1 structure located at the 5' end.

In some embodiments, the isolated nucleic acid is an mRNA, and the mRNA comprises a modified nucleotide located in a 5'-untranslated region (5'-UTR), an open reading frame (ORF), or a 3'-untranslated region (3'-UTR) of the mRNA. Those skilled in the art will appreciate that a distribution pattern of the modified nucleotides may be optimized according to requirements for expression of a target protein.

In some embodiments, the mRNA comprises a 5'-UTR with a variable length, the 5'-UTR may comprise cis-acting elements that enhance translation initiation efficiency. In some embodiments, the mRNA comprises a 3'-UTR with a variable length, the 3'-UTR may comprise elements that enhance mRNA stability or interact with RNA binding proteins.

In some embodiments, the mRNA comprises a 5'-UTR, and the 5'-UTR comprises untranslated region fragments derived from a human albumin gene, an α-globin gene, a β-globin gene, a γ-globin gene, or a liver highly expressed gene.

In some embodiments, the mRNA comprises one or more 3'-UTR sequences derived from human highly expressed genes to enhance stability and translation efficiency in human cells.

In some embodiments, the mRNA comprises a 3'-UTR, and the 3'-UTR comprises an untranslated region fragment derived from a human albumin gene, an α-globin gene, a β-globin gene, a γ-globin gene, or a liver highly expressed gene.

In some embodiments, the mRNA comprises a 5'-UTR and a 3'-UTR, the 5'-UTR comprises a 5'-UTR fragment derived from a human γ-globin gene, and the 3'-UTR comprises a 3'-UTR fragment derived from a human γ-globin gene.

In some embodiments, the mRNA comprises one or more poly(A) tails, and a length of the poly(A) tail may be about 30 to 300 adenosine residues to improve mRNA stability and binding ability of mRNA to a translation initiation complex.

In some embodiments, the mRNA comprises an optimized codon usage pattern, a codon combination in an open reading frame is determined with reference to high-frequency codons commonly found in a target expression host, e.g., human cells, mammalian cells, or HEK293 cells, to improve translation efficiency.

In some embodiments, the mRNA comprises one, two, or more of the following structural features: an optimized 5'-UTR sequence, a Cap1 structure, a nucleotide containing a 2'-O-methylation modification, pseudouridine substitution, a 3'-UTR enhancer element, a poly(A) tail, and a multiply modified open reading frame.

In some embodiments, a cap structure is introduced into the mRNA after *in vitro* transcription by enzymatic or co-transcriptional capping, and the capping method comprises using 7-methylguanosine (m⁷G) as a cap group and introducing a 2'-O-methyl modification at a first nucleotide to form a Cap1 structure.

In some embodiments, the mRNA further comprises one or more RNA stabilization structural elements, which are located in the 3'-UTR region of the mRNA and may comprise a low GC content region, an AU-rich element, or an exogenous RNA stabilization sequence.

In some embodiments, the cap structure and modification of the mRNA may be used in combination to reduce an activation probability of pattern recognition receptors (e.g., RIG-I, MDA5, TLR7/8) associated with innate immunity, to attenuate an interferon response.

In some embodiments, the mRNA is obtained by cell-free transcription, and dsRNA impurities are removed by high-performance liquid chromatography (HPLC) or other purification methods to reduce non-specific immune stimulation and improve safety in therapeutic applications.

In some embodiments, the isolated nucleic acid is codon optimized for expression in cells.

In some embodiments, the isolated nucleic acid is codon optimized for expression in a eukaryote, a mammal such as a human or a non-human mammal, a plant, an insect, a bird, a reptile, a rodent (e.g., a mouse, a rat), a fish, a worm/nematode, or a yeast.

In another aspect, one or more embodiments of the present disclosure provide a CRISPR-Cas12 system. In some embodiments, the CRISPR-Cas12 system comprises:
(a) the Cas12 protein, the inactivated Cas12 mutant, the fusion protein or conjugate, or the isolated nucleic acid as described herein; and
(b) a guide polynucleotide, or a polynucleotide sequence encoding the guide polynucleotide.

The Cas12 protein, the inactivated Cas12 mutant, or the fusion protein or conjugate forms a complex with the guide polynucleotide; and the guide polynucleotide comprises a guide sequence engineered to guide a sequence-specific binding of the complex to a target nucleic acid.

The isolated nucleic acid encodes the Cas12 protein, the inactivated Cas12 mutant, or the fusion protein or conjugate as described herein.

In some embodiments, the guide polynucleotide comprises a DR sequence linked to the guide sequence.

In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the amino acid sequences shown in SEQ ID NOs: 1-35.

In some embodiments, the Cas12 protein belongs to a Cas12h subtype (subtype V-H), the Cas12 protein forms a complex with a guide polynucleotide, and the complex specifically binds to a target nucleic acid in a eukaryotic cell. In some embodiments, the Cas12 protein forms a complex with a guide polynucleotide, and the complex specifically binds to and cleaves a target nucleic acid in a eukaryotic cell.

In some embodiments, the fusion protein comprises the amino acid sequence of the Cas12 protein.

In some embodiments, the DR sequence has at least 50% sequence identity to any one of the sequences shown in SEQ ID NOs: 36-170 or SEQ ID NOs: 187-195.

In some embodiments, the guide polynucleotide comprises a DR sequence linked to the guide sequence. In some embodiments, the DR sequence has at least 50% sequence identity to any one of the sequences shown in SEQ ID NOs: 36-170 or SEQ ID NOs: 187-195. In some specific embodiments, the DR sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to any one of the sequences shown in SEQ ID NOs: 36-170 or SEQ ID NOs: 187-195. In some embodiments, the DR sequence comprises any one of the sequences shown in SEQ ID NOs: 36-170 or SEQ ID NOs: 187-195.

In some embodiments, the guide sequence comprises 15-60 nucleotides. In some embodiments, the guide sequence comprises 15-50 nucleotides. In some embodiments, the guide sequence comprises 15-40 nucleotides. In some embodiments, the guide sequence comprises 15-35 nucleotides. In some embodiments, the guide sequence comprises 15-30 nucleotides. In some embodiments, the guide sequence comprises 15-25 nucleotides. In some embodiments, the guide sequence comprises 18-25 nucleotides. In some embodiments, the guide sequence comprises 20-25 nucleotides. In some embodiments, the guide sequence comprises 18-22 nucleotides. In some embodiments, the guide sequence comprises 20-22 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, or 40 nucleotides.

In some embodiments, the guide sequence hybridizes to the target nucleic acid, and the guide sequence is 90%-100% complementary to the target nucleic acid.

In some embodiments, the guide sequence hybridizes to the target nucleic acid.

In some embodiments, the guide sequence hybridizes to the target nucleic acid, and the guide sequence is mismatched to the target nucleic acid by no more than one nucleotide.

In some embodiments, the DR sequence comprises 15-100 nucleotides. In some embodiments, the DR sequence comprises 15-90 nucleotides. In some embodiments, the DR sequence comprises 15-80 nucleotides. In some embodiments, the DR sequence comprises 15-70 nucleotides. In some embodiments, the DR sequence comprises 15-60 nucleotides. In some embodiments, the guide sequence comprises 15-50 nucleotides. In some embodiment, the guide sequence comprises 15-40 nucleotides. In some embodiments, the guide sequence comprises 20-40 nucleotides. In some embodiments, the guide sequence comprises 20-30 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides.

In some embodiments, the guide sequence is located at the 3' end of the DR sequence.

In some embodiments, the guide sequence is located at the 5' end of the DR sequence.

In some embodiments, the guide polynucleotide does not comprise a tracrRNA sequence.

In some embodiments, the guide polynucleotide further comprises a tracrRNA sequence.

In some embodiments, the tracrRNA is complementary paired with the DR sequence. In general, the complementary pairing is complementary pairing for partial bases. In some embodiments, the tracrRNA interacts with the DR sequence.

In some embodiments, the tracrRNA sequence is linked to the DR sequence. In some embodiments, the tracrRNA sequence is linked to the DR sequence via a nucleotide sequence. In some embodiments, the tracrRNA sequence is linked to the DR sequence via a nucleotide sequence consisting of 1-10 nucleotides. In some embodiments, the tracrRNA sequence is linked to the DR sequence via a nucleotide sequence consisting of 1, 2, 3, 4, 5, 6, 7, 8, 9, or 10 nucleotides. In some embodiments, the tracrRNA sequence is linked to the DR sequence via a nucleotide sequence consisting of 4 nucleotides. In some embodiments, the tracrRNA sequence is linked to the DR sequence via a 5'-GAAA-3' sequence.

In some embodiments, the tracrRNA sequence is located at the 3' end of the DR sequence.

In some embodiments, the tracrRNA sequence is located at the 5' end of the DR sequence.

In some embodiments, the tracrRNA comprises 10-200 nucleotides. In some embodiments, the tracrRNA comprises 10-190, 10-180, 10-170, 10-160, 10-150, 10-140, 10-130, 10-120, 10-110, 10-100, 10-90, 10-80, 10-70, 10-60, 10-50, 10-40, 10-30, 10-20, 10-100, 10-100, 10-100, 10-100, 10-100, 10-100, 10-100, 20-100, 30-100, 40-100, 20-90, 20-80, 20-70, 20-60, 20-50, or 30-50 nucleotides. In some embodiments, the tracrRNA comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 nucleotides.

In preferred embodiments, the guide polynucleotide is the guide polynucleotide as described herein.

In some embodiments, the target nucleic acid is DNA or RNA. In some embodiments, the target nucleic acid is dsDNA or ssDNA.

In some embodiments, the DNA is eukaryotic DNA. In some embodiments, the eukaryotic DNA is non-human mammalian DNA, non-human primate DNA, human DNA, plant DNA, insect DNA, bird DNA, reptile DNA, rodent DNA, fish DNA, worm/nematode DNA, or yeast DNA.

In some embodiments, the target nucleic acid is a disease or disorder-related gene or a signaling biochemical pathway-related gene, or the target nucleic acid is a reporter gene. For example, the disease or disorder is a hematological disease or disorder, an ophthalmic disease or disorder, a neurological disease or disorder, a respiratory disease or disorder, a hepatic disease or disorder, a metabolic disease or disorder, cancer, or an infectious disease.

In some embodiments, the target nucleic acid is selected from target nucleic acids/target genes described in the patent application with publication No. WO2025061113A1.

In another aspect, one or more embodiments of the present disclosure provide a vector system. The vector system comprises one or more recombinant vectors. The recombinant vector comprises the isolated nucleic acid or the CRISPR-Cas12 system as described herein.

In some embodiments, the recombinant vector further comprises a regulatory sequence.

In some embodiments, the vector system comprises one or more recombinant vectors. The recombinant vector comprises a polynucleotide sequence encoding the Cas12 protein, the inactivated Cas12 mutant, or the fusion protein or the conjugate as described herein, and a polynucleotide sequence encoding the guide polynucleotide.

In some embodiments, the polynucleotide sequence encoding the Cas12 protein, the inactivated Cas12 mutant, or the fusion protein or the conjugate is operably linked to a regulatory sequence 1.

In some embodiments, the polynucleotide sequence encoding the guide polynucleotide is operably linked to a regulatory sequence 2.

In some embodiments, the regulatory sequence 1 and the regulatory sequence 2 are the same or different sequences.

In some embodiments, the regulatory sequence is selected from one or more of: a promoter, an enhancer, an internal ribosome entry site (IRES), or a transcription termination signal. The promoter comprises a constitutive promoter, an inducible promoter, a broad-spectrum promoter, or a tissue-specific promoter, and/or the transcription termination signal comprises a polyadenylation signal or a poly-U sequence.

In some embodiments, a scaffold of the recombinant vector is an adeno-associated virus (AAV) vector, a lentiviral vector, a ribonucleoprotein (RNP) complex, or a virus-like particle (VLP).

In some embodiments, when the scaffold is the AAV vector, the AAV vector is a recombinant AAV vector of serotype AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10, or AAVrh74; when the scaffold is the lentiviral vector, the lentiviral vector is pseudotyped with an envelope protein; in some embodiments, the isolated nucleic acid is linked to an aptamer sequence; and when the scaffold is the VLP, the isolated nucleic acid is linked to a gene encoding a gag protein.

In another aspect, one or more embodiments of the present disclosure provide a delivery system. The delivery system comprises: (a) a delivery tool, and (b) the Cas12 protein, the guide polynucleotide, the inactivated Cas12 mutant, the fusion protein or conjugate, the isolated nucleic acid, the CRISPR-Cas12 system, or the vector system as described herein.

In some embodiments, the delivery tool is a virus, a lipid nanoparticle (LNP), a nanoparticle, a liposome, an exosome, a microbubble, or a gene gun.

In some embodiments, the delivery tool is a LNP comprising the guide polynucleotide and mRNA encoding the Cas12 protein, the inactivated Cas12 mutant, or the fusion protein or conjugate.

In another aspect, one or more embodiments of the present disclosure provide a cell. The cell comprises the Cas12 protein, the guide polynucleotide, the inactivated Cas12 mutant, the fusion protein or conjugate, the isolated nucleic acid, the CRISPR-Cas12 system, or the vector system as described herein.

In some embodiments, the cell is a prokaryotic cell.

In some embodiments, the cell is a eukaryotic cell.

In some embodiments, the eukaryotic cell is a mammalian cell.

In another aspect, one or more embodiments of the present disclosure provide a pharmaceutical composition. The pharmaceutical composition comprises the Cas12 protein, the guide polynucleotide, the inactivated Cas12 mutant, the fusion protein or conjugate, the isolated nucleic acid, the CRISPR-Cas12 system, the vector system, the delivery system, or the cell as described herein.

In some embodiments, the pharmaceutical composition further comprises pharmaceutically acceptable excipients.

In another aspect, one or more embodiments of the present disclosure provide a kit. The kit comprises the Cas12 protein, the guide polynucleotide, the inactivated Cas12 mutant, the fusion protein or conjugate, the isolated nucleic acid, the CRISPR-Cas12 system, the vector system, the delivery system, or the cell as described herein.

In some embodiments, the kit further comprises a cut buffer. The cut buffer may be any buffer known in the art suitable for cleaving the target nucleic acid by the Cas12 protein.

In another aspect, one or more embodiments of the present disclosure provide a use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein in preparing a reagent or medicament for diagnosing, treating, and/or preventing a disease or disorder associated with a target nucleic acid.

In some embodiments, the disease or disorder is a hematological disease or disorder, an ophthalmic disease or disorder, a neurological disease or disorder, a respiratory disease or disorder, a hepatic disease or disorder, a metabolic disease or disorder, a cancer, or an infectious disease. In some embodiments, the reagent or medicament is used to: cleave one or more target nucleic acid molecules or introduce nicks into one or more target nucleic acid molecules, activate or upregulate an expression of the one or more target nucleic acid molecules, activate or inhibit transcription of the one or more target nucleic acid molecules, inactivate the one or more target nucleic acid molecules, visualize, label, or detect the one or more target nucleic acid molecules, bind the one or more target nucleic acid molecules, transport the one or more target nucleic acid molecules, and mask the one or more target nucleic acid molecules.

In some embodiments, the target nucleic acid is selected from target nucleic acids/target genes described in Table 27 of the patent application with publication No. WO2025061113A1. The disease or disorder is the corresponding disease or disorder listed in the Table 27.

In another aspect, one or more embodiments of the present disclosure provide a method for detecting, binding, or cleaving a target nucleic acid, comprising: contacting the target nucleic acid with the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein.

In some embodiments, the method is for non-diagnostic and/or non-therapeutic purposes; and/or the fusion protein or conjugate comprises a detectable marker, e.g., a marker detectable by fluorescence, DNA blotting, or FISH.

In some embodiments, when the method is cleaving the target nucleic acid, the method further comprises performing a cleavage reaction using a cut buffer. The cut buffer may be any buffer known in the art suitable for cleaving the target nucleic acid by the Cas12 protein.

In another aspect, one or more embodiments of the present disclosure provide a method for altering a cell state, comprising contacting the cell with the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein to alter a cell state.

In some embodiments, the method results in one or more of: an increase or decrease in an expression of a specific gene, an induction of cellular senescence *in vitro* or *in vivo,* an induction of cellular cycle arrest *in vitro* or *in vivo,* a cellular growth promotion and/or cellular growth inhibition *in vitro* or *in vivo,* an induction of anergy *in vitro* or *in vivo,* an induction of apoptosis *in vitro* or *in vivo,* and an induction of necrosis *in vitro* or *in vivo.*

In some embodiments, the method is for non-diagnostic and/or non-therapeutic purposes.

In another aspect, one or more embodiments of the present disclosure provide a method for diagnosing, treating, or preventing a disease or disorder associated with a target nucleic acid, comprising administering the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein to a sample from a subject in need or to the subject in need.

In some embodiments e, the target nucleic acid is selected from target nucleic acids/target genes described in Table 27 of the patent application with publication No. WO2025061113A1, and the disease or disorder is the corresponding disease or disorder listed in the Table 27.

In some embodiments, the disease or disorder is a hematological disease or disorder, an ophthalmic disease or disorder, a neurological disease or disorder, a respiratory disease or disorder, a hepatic disease or disorder, a metabolic disease or disorder, a cancer, or an infectious disease.

In another aspect, one or more embodiments of the present disclosure provide a use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein in diagnosing, treating, or preventing a disease or disorder associated with a target nucleic acid.

In some embodiments, the target nucleic acid is selected from target nucleic acids/target genes described in Table 27 of the patent application with publication No. WO2025061113A1, and the disease or disorder is the corresponding disease or disorder listed in the Table 27.

In some embodiments, the disease or disorder is a hematological disease or disorder, an ophthalmic disease or disorder, a neurological disease or disorder, a respiratory disease or disorder, a hepatic disease or disorder, a metabolic disease or disorder, cancer, or an infectious disease.

In some embodiments, the disease or disorder is selected from: hemophilia A, Best yolk-like macular dystrophy, B-cell acute lymphoblastic leukemia, hemophilia B, CDKL5 deficiency, CLN2 disease, Niemann-Pick disease type C, Dravet syndrome, FOXG1syndrome, GM1 ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5deficiency, X-linked myotubular myopathy, X-linked retinoschisis, X-linked retinitis pigmentosa, α1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Sietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1, spinal muscular atrophy, Tay-Sachs disease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase deficiency, Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune deficiency, heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin (ATTR) amyloidosis, alpha-1-antitrypsin deficiency (AATD) liver disease, or AATD lung disease.

In some embodiments, genes associated with the ATTR amyloidosis comprise, but are not limited to, *ATTR.*

Genes associated with the Leber hereditary optic neuropathy comprise, but are not limited to, *MT-ND4.*

Genes associated with the AATD liver disease comprise, but are not limited to, *AATD.*

Genes associated with the AATD lung disease comprise, but are not limited to, *AATD.*

Genes associated with the graft-versus-host disease comprise, but are not limited to, a thymidine kinase gene.

Genes associated with the hereditary retinal dystrophy comprise, but are not limited to, *RPE65.*

Genes associated with the spinal muscular atrophy comprise, but are not limited to, *SMN1.*

Genes associated with the osteoarthritis comprise, but are not limited to, *TGFβ1.*

Genes associated with the hemophilia A comprise, but are not limited to, *factor VIII.*

Genes associated with the hemophilia B comprise, but are not limited to, *factor IX.*

Genes associated with the cystic fibrosis comprise, but are not limited to, *CFTR.*

Genes associated with the Parkinson's disease comprise, but are not limited to, *Gad1, Gad2, PTBP1, KEAP1, RE1, Amigo1, Gprc5c, Let-7a, Pnky, LRRK2, SNCA, GBA, miR-926, miR-9, miR-124, miR-181, HMGB1, TRIM72, GPNMB,* and *REST.*

Genes associated with the Usher's syndrome comprise, but are not limited to, *USH2A.*

Genes associated with the α-thalassemia, β-thalassemia, and sickle cell disease comprise, but are not limited to, *BCL11A, HBG, HBA,* and *HBB.*

Genes related to the pulmonary hypertension comprise, but are not limited to, *eNOS.*

Genes related to the Stargardt's disease comprise, but are not limited to, *ABCA4.*

Genes related to the age-related macular degeneration comprise, but are not limited to, *VEGFA, VEGFR, IL17, Kir7.1, LCN-2, IRAK-M, CD59, LTA4H, GPX4, GLS1, PAPP-A, cGAS, STING, mTOR, GCN2, Nrf2, Ang 2, CTGF,* complement *C3,* complement *C5, CHFR46, DOCK6, CTSS gene, ELN gene,* and *FGF2.*

Genes related to the glaucoma comprise, but are not limited to, *AQP1, ADRB2, NMNTA2, NRP1, Hrh1, Anxa2, OPA1, Cx43, ANGPTL7, MYOC, ROCK1, ROCK2, TIMP1, TIMP2, TIMP3, TIMP4,* carbonic anhydrase *CA2,* carbonic anhydrase *CA4,* and carbonic anhydrase *CA12.*

Genes related to the idiopathic pulmonary fibrosis comprise, but are not limited to, *CTGF.*

Genes related to the hyperlipidemia comprise, but are not limited to, *PCSK9.*

Genes related to the Alzheimer's disease comprise, but are not limited to, *NGF.*

Genes related to the coronary heart disease comprise, but are not limited to, *VEGFA* and *bFGF.*

Genes related to the chronic nephrogenic anemia comprise, but are not limited to, *EPO.*

Genes related to the Leber's congenital amaurosis comprise, but are not limited to, *RPE65.*

Genes related to the retinitis pigmentosa comprise, but are not limited to, *PDE6B.*

Genes related to the phenylketonuria comprise, but are not limited to, *PAH.*

Genes related to the epilepsy comprise, but are not limited to, *GAT1.*

On the basis of conforming to common knowledge in the art, the above conditions may be arbitrarily combined to obtain various embodiments of the present disclosure.

### BRIEF DESCRIPTION OF THE DRAWINGS

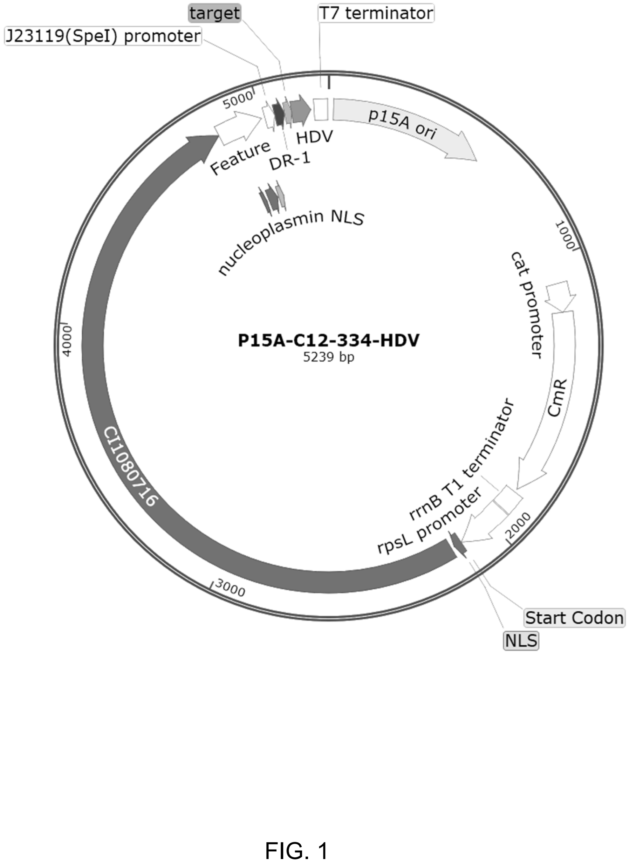

FIG. 1 is a map of a vector P15A-C12-334-HDV according to some embodiments of the present disclosure.

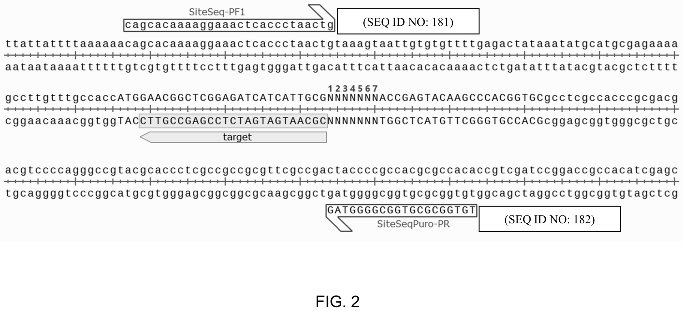

FIG. 2 is a schematic diagram illustrating a targeted cleavage site in a plasmid curing assay according to some embodiments of the present disclosure, where a 5'-end of the target site comprises a 7 nt random sequence, and the sequence and its reverse complementary sequence in the FIG. 2 are shown in SEQ ID NO: 259 and SEQ ID NO: 287, respectively.

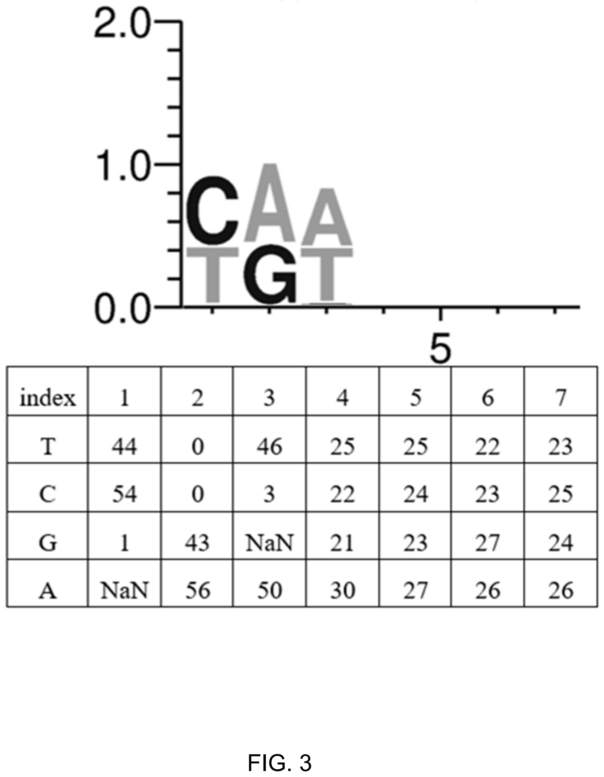

FIG. 3 shows that a PAM sequence captured by C12-334 in a plasmid curing assay is 5'-WYR-3' according to some embodiments of the present disclosure.

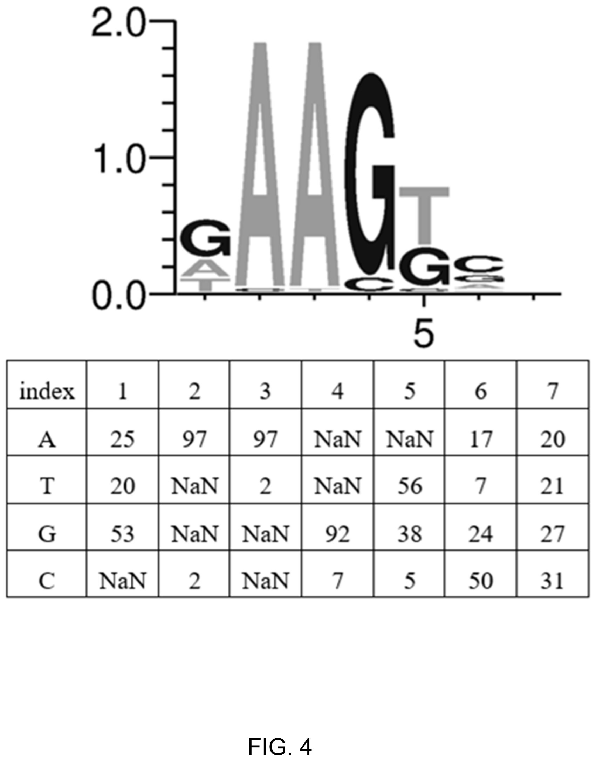

FIG. 4 shows that a PAM sequence captured by C12-335 in a plasmid curing assay is 5'-BMCTTH-3' according to some embodiments of the present disclosure.

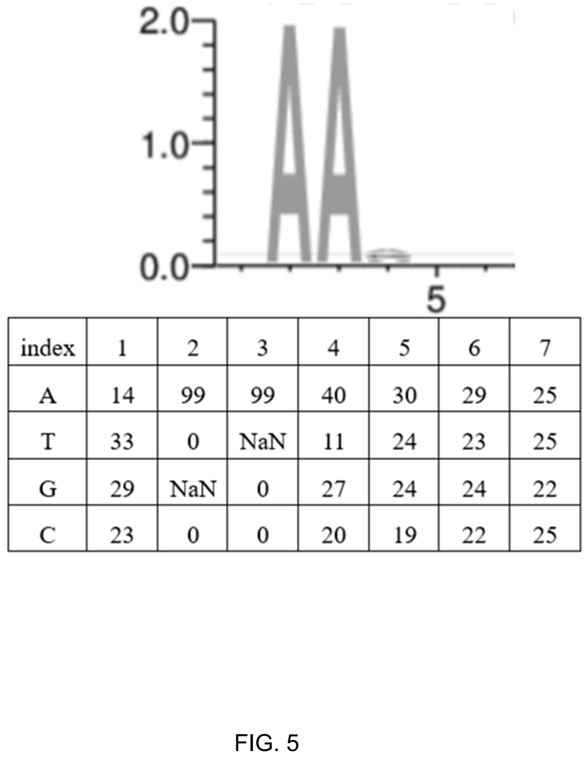

FIG. 5 shows that a PAM sequence captured by C12-336 in a plasmid curing assay is 5'-TTN-3' according to some embodiments of the present disclosure.

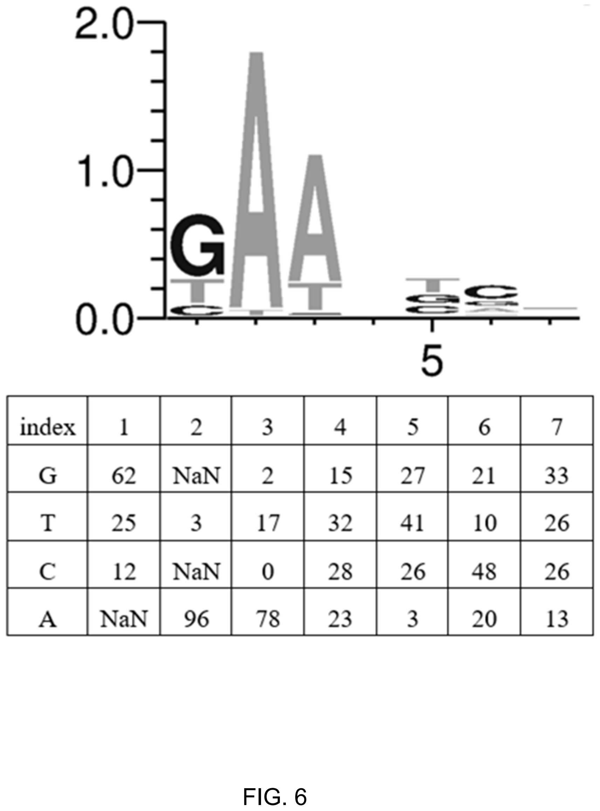

FIG. 6 shows that a PAM sequence captured by C12-340 in a plasmid curing assay is 5'-VNWTV-3', 5'-VNWTC-3', or 5'-VNTTC-3' according to some embodiments of the present disclosure.

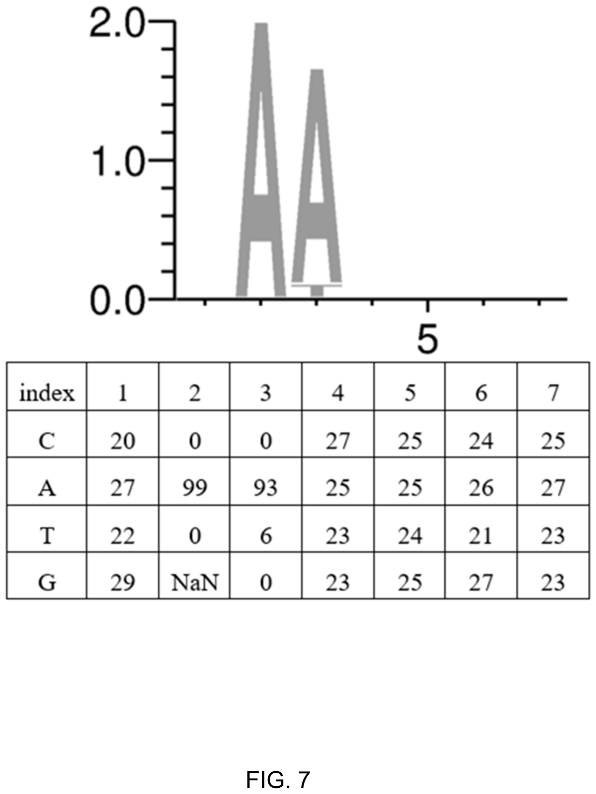

FIG. 7 shows that a PAM sequence captured by C12-341 in a plasmid curing assay is 5'-TTN-3' according to some embodiments of the present disclosure.

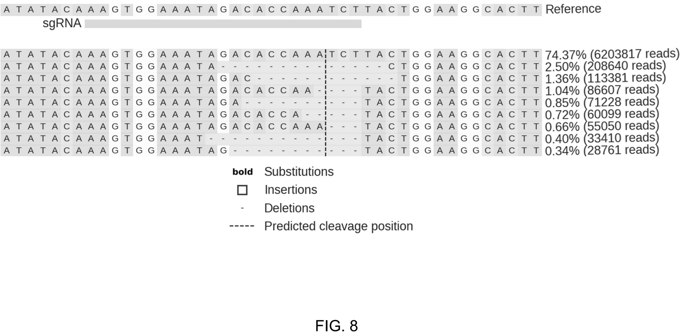

FIG. 8 shows main indel results generated by *TTR* gene editing targeted by C12-334 according to some embodiments of the present disclosure the sequences in the FIG. 8 are shown in SEQ ID NOs: 260-268.

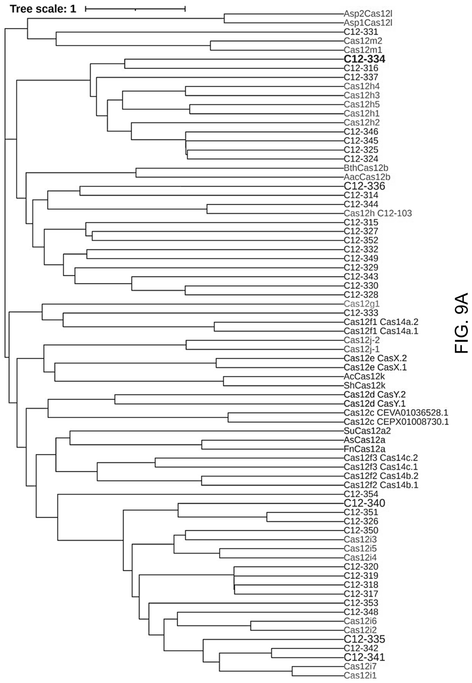

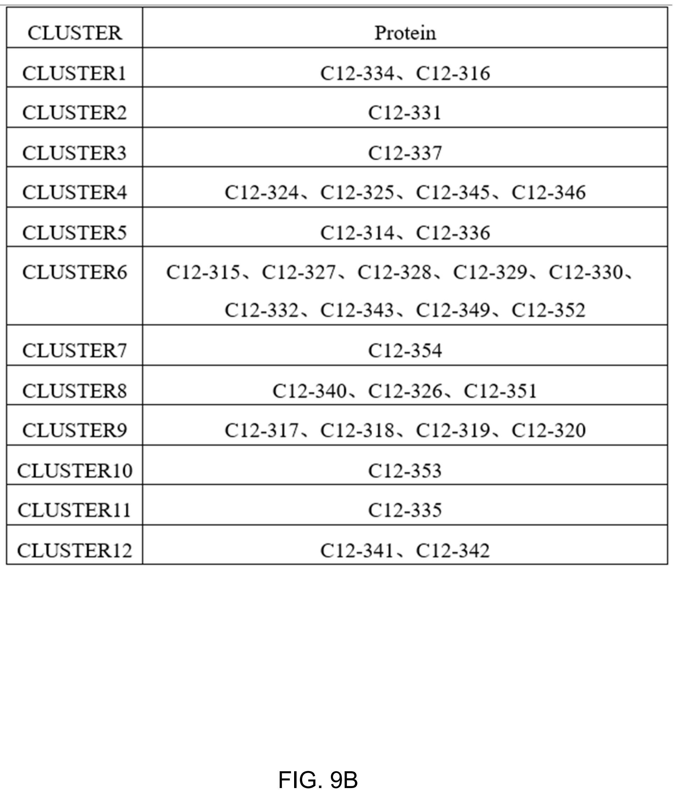

FIG. 9A is a diagram illustrating an evolutionary relationship of Cas proteins and known Cas12 isoform proteins (constructing an evolutionary tree using FastTree after multiple sequence alignment) according to some embodiments of the present disclosure, and FIG. 9B shows the proteins according to some embodiments of the present disclosure. In the evolutionary tree, some proteins of the present disclosure form an independent and distinctly separated branch (a different cluster [CLUSTER]) compared to the known Cas12 proteins, i.e., the proteins of the present disclosure are not mixed with the known Cas12 proteins.

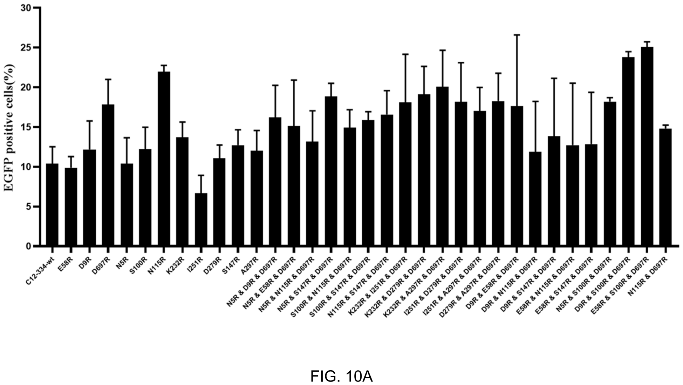

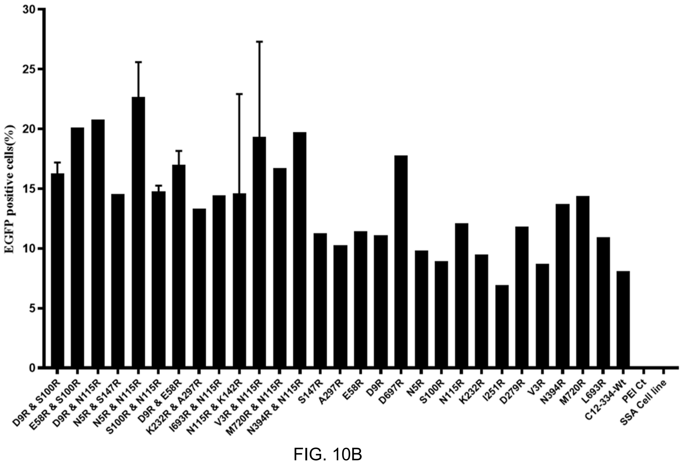

FIG. 10A and FIG. 10B show cleavage activity-based editing efficiencies of various mutants tested in an SSA reporter cell line according to some embodiments of the present disclosure.

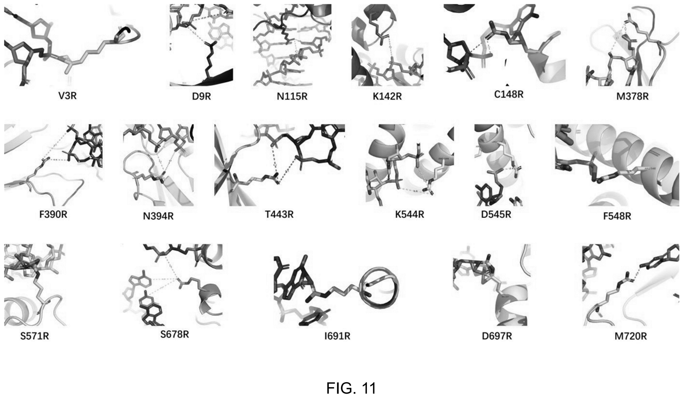

FIG. 11 shows AlphaFold3 structure prediction results based on a C12-334+gRNA+target DNA ternary complex according to some embodiments of the present disclosure, demonstrating that many of advantageous mutants with enhanced gene editing efficiency identified through experimental screening are related to a binding mechanism between the Cas protein and nucleic acids (sgRNA/dsDNA).

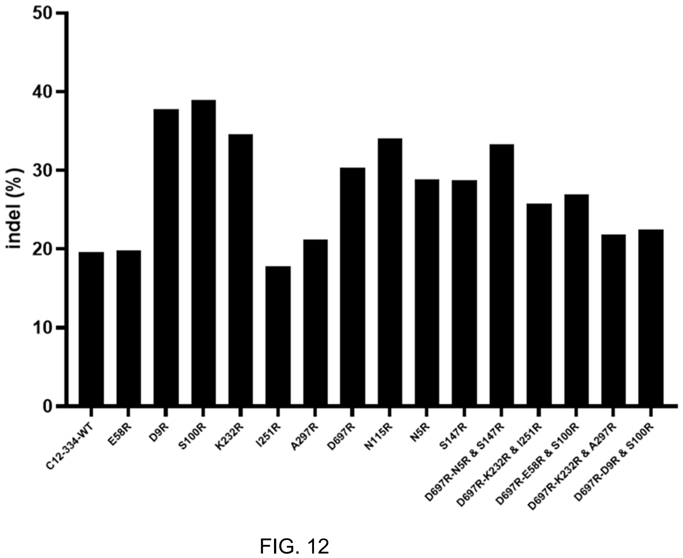

FIG. 12 shows editing efficiencies of different C12-334 mutants targeting an *HPRT1* gene in combination with C12-334-HPRT1-sgRNA05 according to some embodiments of the present disclosure.

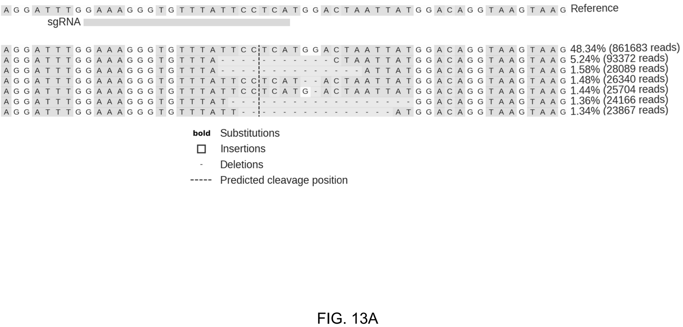

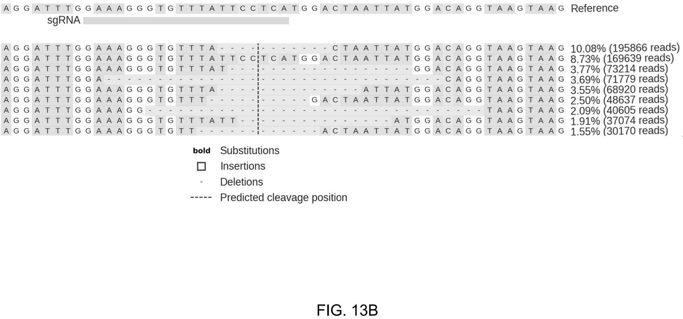

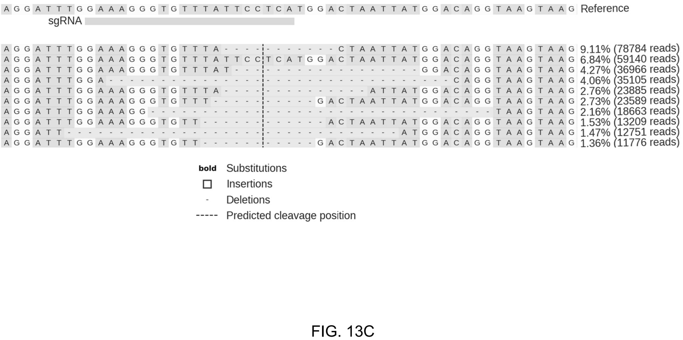

FIG. 13A to FIG. 13C show indel frequencies and distributions detected by NGS after mRNAs of a wild-type C12-334 or C12-334 mutants are respectively combined with a modified gRNA (C12-334-dmHPRT1-sgRNA05-01) to target an *HPRT1* gene according to some embodiments of the present disclosure, where editing efficiencies of the wild-type C12-334 and two mutants reach 48.80%, 90.88%, and 92.77%, respectively, the sequences in FIG. 13A are shown in SEQ ID NOs: 269-275, the sequences in FIG. 13B are shown in SEQ ID NOs: 276-284, and the sequences in FIG. 13C are shown in SEQ ID NOs: 276-286.

### DETAILED DESCRIPTION

In the present disclosure, scientific and technical terms used herein have the meanings commonly understood by those of skill in the art unless otherwise indicated. Additionally, the procedures involving molecular genetics, nucleic acid chemistry, chemistry, molecular biology, biochemistry, cell culture, microbiology, cell biology, genomics, and recombinant DNA, as used herein, are all standard techniques widely employed in their respective fields. At the same time, for better understanding of the present disclosure, definitions and explanations of relevant terms are provided below.

In the present disclosure, the term "multiple" refers to a quantity greater than or equal to 2. In the present disclosure, the term "a plurality of" refers to a quantity greater than or equal to 3.

In the present disclosure, depending on the context, the term "cleavage" refers to cutting a main chain of a polynucleotide chain; and non-limiting examples include complete cleavage of a single-stranded DNA (ssDNA), cleavage of one strand of a double-stranded DNA (dsDNA), or cleavage of both strands of a dsDNA.

In the present disclosure, depending on the context, the term "modification" refers to other forms of chemical reactions of nucleic acid strands other than "cleavage". It includes, but is not limited to, base substitution, insertion, and/or deletion, as well as methylation and demethylation of nucleic acid strands. Non-limiting examples include base substitution on a target nucleic acid strand through single-base editing (e.g., the Cas12 of the present disclosure is fused with a deaminase domain and combined with gRNA), such as nucleotide mutations A→G, C→T, T→C, or G→A, as well as other types of nucleotide mutations (e.g., A→T, C→G, T→A, G→C, etc.). Other examples include base substitution, insertion, or deletion through Prime editing technology (e.g., the Cas12 of the present disclosure is fused with a reverse transcriptase and combined with pegRNA), or base substitution, insertion, or deletion through homology-directed repair (HDR) (e.g., the Cas12 of the present disclosure is combined with gRNA and a donor template). In addition, the Cas12 of the present disclosure is also fused with a DNA methyltransferase or a DNA demethylase and combined with gRNA for targeted modification, to achieve regulation of the methylation level of target nucleic acids.

In the present disclosure, depending on the context, the term "modulating an expression of a target nucleic acid" refers to modulation of the transcription of the target nucleic acid. Non-limiting examples include enhancing or suppressing the transcription of the target nucleic acid using CRISPRa or CRISPRi technologies, by means of a transcriptional activation or repression domain fused to Cas12.

In the present disclosure, letters in amino acid sequences denote single-letter abbreviations for amino acids well known in the art, as described in J. Biol. Chem, 243, p3558 (1968*):* Alanine: Ala-A, Arginine: Arg-R, Aspartic acid: Asp-D, Cysteine: Cys-C, Glutamine: Gln-Q, Glutamic acid: Glu-E, Histidine: His-H, Glycine: Gly-G, Asparagine: Asn-N, Tyrosine: Tyr-Y, Proline: Pro-P, Serine: Ser-S, Methionine: Met-M, Lysine: Lys-K, Valine: Val-V, Isoleucine: Ile-I, Phenylalanine: Phe-F, Leucine: Leu-L, Tryptophan: Trp-W, and Threonine: Thr-T.

In the present disclosure, the term "amino acid difference" refers to the difference of amino acid residues at specific positions in the protein's amino acid sequence, including substitution, insertion, or deletion.

It is well known to those skilled in the art that in proteins or peptides, two adjacent amino acids each lose an OH or H through dehydration condensation to form a peptide bond, and each amino acid exists in the form of an amino acid residue. Thus, in the present disclosure, the terms "amino acid" and "amino acid residue" refer to the same meaning. Further, in the present disclosure, to simplify the expression, the amino acid residue before the substitution is retained before the position of the amino acid residue; the letter before the position indicates the original amino acid residue, and the letter after the position indicates the substituted amino acid residue. For example, "S211" represents that the original amino acid residue at position 211 is S, and when it is substituted with R, it may be expressed as "S211R".

In the present disclosure, the symbol "+" is sometimes used to connect one amino acid mutation on each side, indicating that both point mutations are present simultaneously in a single mutant; if a plurality of point mutations are connected by two or more "+", it represents that these point mutations are present simultaneously.

As used herein, mutation may refer to substitution with any other natural amino acid residue. Alternatively, the mutation is substitution with residue R, H, K or A; alternatively, the mutation is substitution with residue R; alternatively, the mutation is substitution with residue A.

In the present disclosure, if an amino acid is substituted, it refers to that it is substituted with another amino acid residue different from the original amino acid residue. If the original amino acid is a positively charged amino acid and is substituted with a positively charged amino acid, it refers to that it is substituted with another positively charged amino acid residue different from the original one. For example, if an original amino acid residue is R and is substituted with a positively charged amino acid, it refers to that it is substituted with H or K.

In the present disclosure, when referring to an "RNA sequence", "T" in the sequence is used interchangeably with "U". When referring to a "guide sequence", "T" in the sequence is used interchangeably with "U". When referring to a "direct repeat (DR) sequence", "T" in the sequence is used interchangeably with "U". When referring to a "tracrRNA sequence", "T" in the sequence is used interchangeably with "U".

### Sequence identity

As used herein, the term "identity" refers to a sequence matching degree between two polypeptides or between two nucleic acids. The terms "identity", "percent identity", and "sequence identity" are used interchangeably. When a given position in two compared sequences are occupied by the same base or amino acid monomeric subunit (for example, if the same position in each of two DNA molecules is occupied by adenine, or the same position in each of two polypeptides is occupied by lysine), the molecules are considered to be identical at that position. The percent identity between two sequences is calculated by a function: the number of matching positions shared by the two sequences/the total number of compared positions×100%. For example, if there are 6 matching positions in 10 positions of two sequences, then the two sequences have 60% sequence identity. Typically, the alignment is performed when aligning the two sequences to generate the maximum sequence identity. Such alignment may be performed by using published and commercially available alignment algorithms and programs, including but not limited to CLUSTER Ω, MAFFT, Probcons, T-Coffee, Probalign, and BLAST, which may be reasonably selected and used by one of ordinary skill in the art. Those skilled in the art can determine appropriate parameters for sequence alignment, including any algorithm required to achieve an optimal or best alignment for the full length of the compared sequences, as well as any algorithm required to achieve an optimal or best alignment for a local region of the compared sequences.

### CRISPR-Cas12 system

As used herein, the terms "clustered regularly interspaced short palindromic repeats (CRISPR)-CRISPR-Cas system", "CRISPR-Cas12 system", or "CRISPR system" are used interchangeably. The CRISPR-Cas12 system generally comprises amino acid sequences encoding Cas proteins or their encoding nucleic acids, and guide polynucleotides or their encoding nucleic acids.

*Zhang Feng* et al. discovered Cas12a in 2015, categorized as type V in the Class II CRISPR-Cas system. After detailed studies of subtype V-A (Cas12a), *Zhang Feng* et al. reported Cas12b (C2C1) in 2015. In 2017, *Burstein* et al. reported the Cas12e (CasX) nuclease. In 2019, *Winston X. Yan et* al. reported the newly discovered type V Cas effector proteins Cas12c, Cas12h, Cas12i, and Cas12g in detail by bioinformatics analysis.

In some embodiments, the Cas12 protein as described herein refers to a protein having an amino acid sequence, the amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to any one of the sequences shown in SEQ ID NOs: 1-35. When the CRISPR-Cas12 system comprises a fusion protein or conjugate comprising the Cas12 protein and a functional domain, a percent sequence identity between the Cas12 portion of the fusion protein or the conjugate and a reference sequence is calculated.

In the present disclosure, the CRISPR-Cas12 system comprises the Cas12 protein with the amino acid sequence having at least 50% sequence identity to any one of the sequences shown in SEQ ID NOs: 1-35, or a nucleic acid encoding the Cas12 protein; and a guide polynucleotide or a nucleic acid encoding the guide polynucleotide. The guide polynucleotide comprises a DR sequence linked to a guide sequence, the guide sequence is engineered to hybridize to a target nucleic acid, and the guide polynucleotide forms a complex with the Cas12 protein and guides the sequence-specific binding of the complex to the target nucleic acid.

### Guide polynucleotide

As used herein, the term "guide polynucleotide" refers to a molecule in the CRISPR-Cas system that forms a complex with a Cas protein and guides the complex to a target sequence. Typically, the guide polynucleotide comprises a scaffold sequence that is linked to a guide sequence, and the guide sequence may hybridize to a target sequence. Typically, the scaffold sequence comprises a DR sequence, and sometimes, the scaffold sequence also comprises a tracrRNA sequence. In some embodiments, the guide polynucleotide does not comprise a tracrRNA sequence. In some embodiments, the guide polynucleotide comprises a tracrRNA sequence.

In some embodiments, the guide polynucleotide of the CRISPR-Cas12 system is a guide RNA. In some embodiments, the guide polynucleotide is a chemically modified guide polynucleotide. In some embodiments, the guide polynucleotide comprises at least one chemically modified nucleotide.

In some embodiments, the guide polynucleotide comprises at least one guide sequence (or referred to as a spacer sequence) that is linked to at least one DR sequence. In some embodiments, the guide sequence is located at the 3' end of the DR sequence. In some embodiments, the guide sequence is located at the 5' end of the DR sequence.

In some embodiments, the tracrRNA sequence is linked to the DR sequence.

In some embodiments, the tracrRNA sequence is located at the 5' end or 3' end of the DR sequence. In some embodiments, the tracrRNA sequence is located at the 5' end of the DR sequence. In some embodiments, the tracrRNA sequence is located at the 3' end of the DR sequence.

In some embodiments, a nucleotide sequence of the guide polynucleotide comprises the DR sequence and the guide sequence in order from the 5' end to the 3' end.

In some embodiments, the nucleotide sequence of the guide polynucleotide comprises the guide sequence and the DR sequence in order from the 5' end to the 3' end.

In some embodiments, the nucleotide sequence of the guide polynucleotide comprises the tracrRNA, the DR sequence, and the guide sequence in order from the 5' end to the 3' end.

In some embodiments, the nucleotide sequence of the guide polynucleotide comprises the tracrRNA, a linker sequence, the DR sequence, and the guide sequence in order from the 5' end to the 3' end.

In some embodiments, the nucleotide sequence of the guide polynucleotide comprises the tracrRNA, a loop sequence, the DR sequence, and the guide sequence in order from the 5' end to the 3' end.

In some embodiments, a structure of the guide polynucleotide is as follows: 5'-tracrRNA- loop sequence-DR sequence-guide sequence-3'.

In some embodiments, the tracrRNA and the DR sequence of the guide polynucleotide are linked by a nucleotide sequence.

In some embodiments, the tracrRNA sequence is linked to the DR sequence by a nucleotide sequence consisting of 1, 2, 3, 4, 5, 6, 7, 8, 9, or 10 nucleotides. In some embodiments, the tracrRNA sequence is linked to the DR sequence by a nucleotide sequence consisting of 4 nucleotides. In some embodiments, the tracrRNA sequence is linked to the DR sequence by a 5'-GAAA-3' sequence.

In some embodiments, the guide sequence is sufficiently complementary to a target nucleic acid sequence to hybridize to the target nucleic acid and to guide sequence-specific binding of a CRISPR-Cas12 complex to the target nucleic acid. In some embodiments, the guide sequence has 100% complementarity to the target nucleic acid, but the guide sequence may also have less than 100% complementarity to the target nucleic acid, e.g., at least 80%, at least 85%, at least 90%, at least 95%, at least 98%, or at least 99% complementarity.

In some embodiments, the guide sequence is engineered to hybridize to the target nucleic acid and is mismatched to the target nucleic acid by no more than two nucleotides. In some embodiments, the guide sequence is engineered to hybridize to the target nucleic acid and is mismatched to the target nucleic acid by no more than one nucleotide. In some embodiments, the guide sequence is engineered to hybridize to the target nucleic acid and is not mismatched or is mismatched to the target nucleic acid.

In some embodiments, the CRISPR-Cas12 system comprises at least 2, at least 3, at least 4, at least 5, at least 10, or at least 20 different guide polynucleotides. In some embodiments, the guide polynucleotide targets at least 2, at least 3, at least 4, at least 5, at least 10, or at least 20 different target nucleic acid molecules, or targets at least 2, at least 3, at least 4, at least 5, at least 10, or at least 20 different regions of one or more target nucleic acid molecules.

In some embodiments, the guide polynucleotide comprises a constant DR sequence located upstream of a variable guide sequence. In some embodiments, a plurality of guide polynucleotides are portions of an array, which may be portions of a vector, e.g., a viral vector or plasmid. For example, a guide array that comprises a sequence: DR sequence -spacer -DR sequence -spacer-DR sequence-spacer-...-DR sequence -spacer may comprise a plurality of unique unprocessed guide polynucleotides (one for each DR sequence-spacer or spacer-DR sequence). Once introduced into a cell or a cell-free system, the array is processed by the Cas12 protein into several individual mature guide polynucleotides. This allows multiplexing, e.g., delivering a plurality of guide polynucleotides into a cell or system to target a plurality of target nucleic acids or a plurality of regions within a single target nucleic acid.

The ability of the guide polynucleotide to guide the sequence-specific binding of the complex (a CRISPR complex) to the target nucleic acid may be assessed by any suitable assay. For example, components of the CRISPR system sufficient to form the complex (the CRISPR complex), comprising a guide polynucleotide to be tested, may be delivered to a host cell having corresponding target nucleic acid molecules, e.g., by transfection with a vector encoding the components of the CRISPR complex, followed by assessment of preferential cleavage within a target sequence. Similarly, cleavage of the target nucleic acid sequence may be assessed *in vitro* by providing the target nucleic acid and the components of the CRISPR complex comprising the guide polynucleotide to be tested and a control guide polynucleotide different from the guide polynucleotide to be tested, and then comparing the ability of the guide polynucleotide to be tested and the control guide polynucleotide to bind the target nucleic acid or a rate of the guide polynucleotide to be tested and the control guide polynucleotide to cleave the target nucleic acid. The ability of the CRISPR complex to cleave the target nucleic acid or bind the target nucleic acid may also be assessed by the manner described above.

### Cas12 mutants

As used herein, when referring to "a position corresponding to a sequence shown in SEQ ID NO: XX" or a similar textual description, the position may be determined by amino acid sequence alignment, where XX is a positive integer. Typically, the alignment is performed when two sequences are aligned to generate a maximum sequence identity. Such an alignment may be performed by using published and commercially available alignment algorithms and programs such as, but not limited to, Clustal Ω, MAFFT, Probcons, T-Coffee, Probalign, and BLAST, which may be reasonably selected by one of ordinary skill in the art. One skilled in the art may determine appropriate parameters for sequence alignment, including any algorithm needed to achieve an optimal or best alignment for the full length of the compared sequences, as well as any algorithm required to achieve an optimal or best local alignment for the local region of the compared sequences.

In some embodiments, compared to the Cas12 protein having any one of the sequences shown in SEQ ID NOs: 1-35, the Cas12 protein provided herein comprises one or more mutations, e.g., a single amino acid insertion, a single amino acid deletion, a single amino acid substitution, or any combination thereof. In some embodiments, compared to the Cas12 protein having any one of the sequences shown in SEQ ID NOs: 1-35, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions), but retains the ability to bind to a target nucleic acid molecule that is complementary to a guide sequence of a guide polynucleotide, and/or retains the ability to process an RNA transcript containing a guide sequence into a guide polynucleotide molecule. In some embodiments, compared to the Cas12 protein having any one of the sequences shown in SEQ ID NOs: 1-35, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions), but retains the ability to bind to a target nucleic acid molecule that is complementary to a guide sequence of a guide polynucleotide.

One type of modification or mutation comprises substituting an amino acid residue with an amino acid having similar biochemical properties, i.e., a conservative substitution. Typically, the conservative substitution has little or no effect on the activity of the resulting protein or peptide. For example, the conservative substitution refers to an amino acid substitution in the Cas12 protein that does not substantially affect the binding between the Cas12 protein and a target nucleic acid molecule that is complementary to a guide sequence of a gRNA molecule, and/or the process of processing a guide array RNA transcript into gRNA molecules.

More substantial changes may be introduced by using low-conservation substitutions, for example, by selecting residues that differ more significantly in maintaining the following effects: (a) a polypeptide backbone structure in a region where the substitution occurs, e.g., a helical or folded conformation; (b) the charge or hydrophobicity of a region interacted with a target site; or (c) a bulk of an amino acid side chain. The substitutions that are generally expected to produce greatest changes in polypeptide function are (a): a substitution between hydrophilic residues (e.g., serine or threonine) and hydrophobic residues (e.g., leucine, isoleucine, phenylalanine, valine, or alanine); (b) a substitution between cysteine or proline and any other residue; (c) a substitution between residues with a positively charged side chain (e.g., lysine, arginine, or histidine) and residues with a negatively charged side chain (e.g., glutamic acid or aspartic acid); or (d) a substitution between a residue with a bulky side chain (e.g., phenylalanine) and a residue with no side chain (e.g., glycine).

### Cas12 active fragment

In the present disclosure, the Cas12 protein may comprise only a WED-I domain, a Helical-I1 domain, a PI domain, a Helical-I2 domain, a Helical-II domain, a WED-II domain, a RuvC-I domain, a Helical-III domain, a BH domain, a RuvC-II domain, a Nuc domain, and/or a RuvC-III domain.

The Cas12 protein as described herein, in addition to comprising the domains described above, may also comprise domains of Cas12 proteins in the prior art, which together form a complete structure of the Cas12 protein to achieve functions of the Cas12 protein as described herein. The functions comprises, but are not limited to, retaining the ability of the Cas12 protein to form a complex with a gRNA, retaining the ability of the Cas12 protein to form a complex with a gRNA and target a target nucleic acid, retaining the ability of the complex formed by the Cas12 protein with the gRNA to perform targeted modulation of the expression of the target nucleic acid, retaining the ability of the complex formed by the Cas12 protein with the gRNA to perform targeted cleavage of a single strand or double strands of the target nucleic acid, retaining the ability of the Cas12 protein to bind a target nucleic acid molecule that is complementary to a guide sequence of a guide polynucleotide, and/or retaining the ability to process an RNA transcript comprising the guide sequence into guide polynucleotide molecules.

The C12-334 protein comprises the following domain fragments: aa1-24 WED, aa25-109 Helical 1, aa110-182 PI, aa183-340 Helical 1, aa341-447 WED, aa448-522 RuvC, aa523-644 Helical 2, aa645-720 RuvC, aa721-756 Nuc, aa757-769 RuvC, and aa770-891 Nuc.

The "aa" refers to an amino acid. For example, "aa1-24 WED" refers to that amino acids from position 1 to position 24 of the C12-334 protein are the WED domain.

In some embodiments, the Cas12 protein, the inactivated Cas12 mutant, or the fusion protein or the conjugate comprises: (a) any one of the following domain fragments of the C12-334 protein: aa1-24 WED, aa25-109 Helical 1, aa110-182 PI, aa183-340 Helical 1, aa341-447 WED, aa448-522 RuvC, aa523-644 Helical 2, aa645-720 RuvC, aa721-756 Nuc, aa757-769 RuvC, and aa770-891 Nuc; or (b) an amino acid sequence having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to any one of the domain fragments shown in (a). A non-limiting example comprises a new protein obtained by fusing the PI domain or a similar sequence fragment of the C12-334 protein with another protein (e.g., a Cas12 protein, a Cas9 protein, or an IscB protein). The new protein has a function of recognizing a PAM sequence of WYR.

### Inactivated Cas12 mutant

By inactivating the RuvC domain of the Cas12 protein through introducing point mutations, the Cas12 protein loses its endonuclease activity, resulting in dCas12 (dead Cas12) that can only bind a target gene under a mediation of a guide polynucleotide but does not possess a function of cleaving DNA.

Point mutations may also be introduced to partially inactivate the RuvC domain of the Cas12, resulting in a nickase Cas12 (nCas12), which may bind to a target gene and cleave one single strand of a double-stranded nucleic acid without cleaving the other single strand under the mediation of a guide polynucleotide.

Accordingly, the dCas12 or the nCas12 may be fused or conjugated with other domains (including, but not limited to, deaminase domains, transcriptional activation domains, transcriptional repression domains, methylation domains, demethylation domains, histone acetylation domains, and histone deacetylation domains), and the fusion protein or conjugate is guided to a target sequence of a target nucleic acid by a guide polynucleotide to exert corresponding functions through the other domains. For example, a base conversion from cytosine (C) to thymine (T) in a target nucleic acid is achieved by deaminating a cytosine base; a base conversion from adenine (A) to guanine (G) in the target nucleic acid is achieved by deaminating an adenine base; the transcriptional repression of the target nucleic acid is achieved by the transcriptional repression domain KRAB; the transcription of the target nucleic acid is promoted by the transcriptional activation domain VP64; and DNA methylation or expression repression is achieved by a DNMT3A/3B/3L domain.

### Functional domain

In some embodiments, the Cas12 protein or the inactivated Cas12 mutant is covalently linked or fused to a homologous or heterologous functional domain.

In some embodiments, the functional domain has an enzyme activity that modifies a target nucleic acid sequence. The enzyme activity comprises a nuclease activity, a methyltransferase activity, a demethylase activity, a DNA repair activity, a DNA damage activity, a deaminase activity, a dismutase activity, a alkylation activity, a depurination activity, an oxidation activity, a pyrimidine dimer formation activity, an integrase activity, a transposase activity, a recombinase activity, a polymerase activity, a ligase activity, a helicase activity, a photolyase activity, a glycosylase activity, deglycosylation activity, an acetyltransferase activity, a deacetylase activity, a kinase activity, a phosphatase activity, a ubiquitin ligase activity, a deubiquitination activity, an adenylation activity, a deadenylation activity, a SUMOylating activity, a deSUMOylating activity, a myristoylation activity, and/or a demyristoylation activity.

In some embodiments, the functional domain is selected from one or more of the following: a nuclease (e.g., FokI), a methyltransferase, a demethylase, a DNA repair enzyme, a DNA damage enzyme, a deaminase, a dismutase, an alkylase, a depurinase, an oxidase, a pyrimidine dimer-forming enzyme, an integrase, a transposase, a recombinase, a polymerase, a ligase, a helicase, a photolyase, a glycosylase, a deglycosylase, an acetyltransferase, a deacetylase, a kinase, a phosphatase, a ubiquitin ligase, a deubiquitinating enzyme, an adenylylase, a deadenylase, a SUMOylating enzyme, a deSUMOylating enzyme, a myristoylase, and/or a demyristoylase.

In some embodiments, the functional domain is selected from one, two, three, four, or more of the following: a subcellular positioning signal, a DNA binding domain, a protease domain, a transcriptional activation domain, a transcriptional repression domain, a nuclease domain, a deaminase domain, a uracil DNA glycosylase domain (UDG), a uracil DNA glycosylase inhibitory domain (UGI), a methyltransferase, a demethylase, a transcription release factor, a histone acetylase domain, a histone deacetylase domain, a DNA ligase, an affinity tag, a reporter tag, an affinity domain, and/or a reporter domain.

In some embodiments, the deaminase is an adenine deaminase or a cytosine deaminase.

In some embodiments, the deaminase domain is selected from the following: APOBEC1, APOBEC3A, APOBEC3B, APOBEC3C, APOBEC3D, APOBEC3F, an activation-induced cytidine deaminase (AID), cytidine deaminase (CDA) from lamprey, or an engineered mutant of an adenosine deaminase (TadA) that acts on DNA.

In some embodiments, the functional domain has an epigenomic modification activity. In some embodiments, the functional domain has an epigenetic modification activity. In some embodiments, the epigenomic modification or the epigenetic modification comprises, but is not limited to, DNA methylation, RNA methylation, RNA interference, nucleosome positioning, chromatin conformation change, chromatin remodeling, histone modification, and modification of long non-coding RNA sequences.

In some embodiments, the functional domain is an epigenomic modification functional domain. In some embodiments, the functional domain is an epigenetic modification functional domain.

In some embodiments, the functional domain has a single-base editing activity. In some embodiments, the functional domain is a single-base editing functional domain. In some embodiments, the functional domain is a base conversion enzyme. In some embodiments, the functional domain or the base conversion enzyme is a base deaminase. In some embodiments, the functional domain or the base conversion enzyme is an adenine deaminase or a cytosine deaminase. In some embodiments, the functional domain is an adenine deaminase or a cytosine deaminase. In some embodiments, the base conversion enzyme is an adenine deaminase or a cytosine deaminase.

In some embodiments, the transcriptional activation domain is selected from the following: P65, VPR, VP16, VP64, VTR1, VTR2, VTR3, p65, MyoD1, HSF1, RTA, SET7/9, or a histone acetyltransferase. In some embodiments, the transcriptional activation domain is selected from the following: the sequence ETFSDLWKL (SEQ ID NO: 230) from p53 TAD1, the sequence DDIEQWFTE (SEQ ID NO: 231) from p53 TAD2, the sequence SDIMDFVLK (SEQ ID NO: 232) from MLL, the sequence DLLDFSMMF (SEQ ID NO: 233) from E2A, the sequence ETLDFSLVT (SEQ ID NO: 234) from Rtg3, the sequence RKILNDLSS (SEQ ID NO: 235) from CREB, the sequence EAILAELKK (SEQ ID NO: 236) from CREBaB6, the sequence DDWQYLNS (SEQ ID NO: 237) from Gli3, the sequence DDVYNYLFD (SEQ ID NO: 238) from Gal4, the sequence DLFDYDFLV (SEQ ID NO: 239) from Oaf1, the sequence DFFDYDLLF (SEQ ID NO: 240) from Pip2, the sequence EDLYSILWS (SEQ ID NO: 241) from Pdr1, or the sequence TDLYHTLWN (SEQ ID NO: 242) from Pdr3.

In some embodiments, the transcriptional repression domain is selected from the following: Krüppel-associated box protein 1 (KOX1), Krüppel-associated box (KRAB)-associated protein 1 (KAP-1), MAX dimerization protein (MAD), Forkhead box protein O1 (FKHR), Early growth response protein 1 (EGR-1), Estrogen response element-binding domain (ERD), mSin3 interaction domain (SID), a tandem repeat of SID (e.g., SID4X, which is a quadruple repeat of the SID), TGF-β-inducible early growth response protein (TIEG), viral erythroblastosis oncogene A (v-ERB-A), Methyl-CpG-binding domain protein 2 (MBD2), Methyl-CpG-binding domain protein 3 (MBD3), Thyroid hormone receptor alpha (TRa), a histone methyltransferase, a histone deacetylase (HDAC), a nuclear hormone receptor (e.g., an estrogen receptor or a thyroid hormone receptor), DNA methyltransferase (DNMT) family members (e.g., DNMT1, DNMT3A, and DNMT3B), a KRAB domain of Methyl-CpG-binding protein 2 (MeCP2), Ral guanine nucleotide dissociation stimulator-like 2 (ROM2), or Arabidopsis thaliana Histone deacetylase 2A (AtHD2A).

In some embodiments, the transcriptional repression domain is a KRAB domain from a KOX1 protein.

In some embodiments, the nuclease domain is selected from the following: FokI (a restriction nuclease derived from Flavobacterium okeanokoites), a polypeptide with ssDNA cleavage activity, or a polypeptide with dsDNA cleavage activity.

In some embodiments, the methyltransferase domain is selected from a DNA methyltransferase, comprising, but not limited to, DNA methyltransferase 1 (DNMT1), DNA methyltransferase 3 alpha (DNMT3A), and DNA methyltransferase 3 beta (DNMT3B).

In some embodiments, the demethylase is selected from the following: Ten-Eleven Translocation methylcytosine dioxygenase 1 catalytic domain (TET1CD), Ten-Eleven Translocation methylcytosine dioxygenase 1 (TET1), Repressor of Silencing 1 (ROS1), Demeter (DME), Demeter-like protein 2 (DML2), or Demeter-like protein 3 (DML3).

Methylation and demethylation are recognized as important modes of epigenetic gene modulation in the field.

In some embodiments, the homologous or heterologous functional domain refers to a sequence tag that is useful for solubility, purification, or detection of the fusion protein or conjugate. The present disclosure provides suitable protein tag sequences comprising, but not limited to, a biotin carboxyl carrier protein (BCCP) tag, a myc tag (a small epitope tag derived from a c-Myc protein), a calmodulin tag, a FLAG tag (a DYKDDDDK sequence tag), a hemagglutinin (HA) tag, a polyhistidine tag (also known as a His tag), a maltose-binding protein (MBP) tag, a N-utilization substance protein A (nus) tag, a glutathione S-transferase (GST) tag, a green fluorescent protein (GFP) tag, a thioredoxin tag, an S-tag (a short peptide tag derived from RNase A), a Softag (e.g., Softag 1 and Softag 3), a streptavidin-binding peptide tag (strep-tag), a biotin ligase tag, a Fluorescein Arsenical Hairpin binder (FlAsH) tag, a V5 tag (an epitope tag derived from simian virus 5), and an Streptavidin-binding peptide (SBP) tag. Additional suitable sequences are apparent to those skilled in the art.

### Subcellular localization signal

In some embodiments, the Cas12 protein is fused to at least one type of homologous or heterologous subcellular localization signal. In some embodiments, the Cas12 protein is fused to at least one homologous or heterologous subcellular localization signal. Exemplarily, the subcellular localization signal comprises an organelle localization signal, e.g., a nuclear localization signal (NLS), a nuclear export signal (NES), or a mitochondrial localization signal.

Non-limiting examples of NLS include NLS sequences derived from: an NLS of SV40 virus large T antigen, having the amino acid sequence PKKKRKV (SEQ ID NO: 243); an NLS from a nucleoplasmic protein, having the amino acid sequence KRPAATKKAGQAKKKK (SEQ ID NO: 244); a c-myc NLS, having the amino acid sequence PAAKRVKLD (SEQ ID NO: 245) or the amino acid sequence RQRRNELKRSP (SEQ ID NO: 246); a hRNPA1 M9 NLS, having the amino acid sequence NQSSNFGPMKGGNFGGRSSGPYGGGGQYFAKPRNQGGY (SEQ ID NO: 247); a sequence RMRIZFKNKGKDTAELRRRRVEVSVELRKAKKDEQILKRRNV (SEQ ID NO: 248) derived from an IBB domain; a sequence VSRKRPRP (SEQ ID NO: 249) and a sequence PPKKARED (SEQ ID NO: 250) of a myoma T protein; a sequence PQPKKKPL (SEQ ID NO: 251) of human p53; a sequence SALIKKKKKMAP (SEQ ID NO: 252) of mouse c-ablIV; a sequences DRLRR (SEQ ID NO: 253) and a sequence PKQKKRK (SEQ ID NO: 254) of influenza virus NS1; a sequence RKLKKKIKKL (SEQ ID NO: 255) of the hepatitis virus δ antigen; a sequence REKKKFLKRR (SEQ ID NO: 256) of mouse Mx1 protein; a sequence KRKGDEVDGVDEVAKKKSKK (SEQ ID NO: 257) of human poly-ADP-ribose polymerase (PARP) enzyme; and a sequence RKCLQAGMNLEARKTKK (SEQ ID NO: 258) of a steroid hormone receptor. In some embodiments, the nuclear localization sequence has sufficient strength to drive accumulation of the fusion protein or conjugate as described herein within a nucleus of a eukaryotic cell to a detectable amount. In summary, the strength of the nuclear localization activity may be derived from a count of NLS, one or more specific used NLSs, or any combination of these factors. The accumulation within the nucleus may be detected using any suitable technique. For example, a detectable marker may be fused to the Cas protein to allow visualization of its intracellular location, such as in combination with detection manner of a nucleus location (e.g., nucleus-specific dyes such as DAPI). As another example, the cell nucleus may also be isolated from a cell, and its contents are subsequently analyzed using any appropriate manner for detecting protein, such as immunohistochemistry, western blotting, or enzyme activity assays. As another example, the accumulation within the nucleus may also be indirectly determined, such as by assaying an effect of a formation of a nucleic acid-targeting complex (e.g., measuring DNA or RNA cleavage or mutation at a target sequence, or measuring changes in gene expression activity resulting from the formation of a DNA-targeting complex or a RNA-targeting complex and/or an activity of a DNA-targeting Cas protein or a RNA-targeting Cas protein), compared with a control group that is not exposed to a nucleic acid-targeting Cas protein or a nucleic acid-targeting complex, or exposed to a nucleic acid-targeting Cas protein lacking one or more NLSs.

### Nucleic acid/polynucleotide

Nucleic acid or polynucleotide refers to deoxyribonucleic acid (DNA) or ribonucleic acid (RNA) and polymers thereof in a single-stranded or double-stranded form. The term "nucleic acid" comprises, but is not limited to, a gene, cDNA, and mRNA. In one embodiment, the nucleic acid molecule is synthetic (e.g., chemically synthesized) or recombinant. Unless explicitly limited, the term comprises nucleic acids containing analogs or derivatives of natural nucleotides, and the analogs or derivatives have binding properties similar to a reference nucleic acid and are metabolized in a manner similar to naturally occurring nucleotides. Unless otherwise indicated, a particular nucleic acid sequence also implicitly comprises its conservatively modified mutant (e.g., a degenerate codon substitution), an allele, an ortholog, an SNP, a complementary sequence, and an explicitly indicated sequence.

### Vector system

Another aspect of the present disclosure relates to a vector system comprising the CRISPR-Cas12 system described herein. The vector system comprises one or more recombinant vectors, and the recombinant vector comprises a polynucleotide sequence encoding the Cas12 protein and a polynucleotide sequence encoding the guide polynucleotide.

In some embodiments, the vector system comprises at least one plasmid or viral recombinant vector (e.g., a retrovirus, lentivirus, adenovirus, adeno-associated virus, or herpes simplex virus). In some embodiments, the polynucleotide sequence encoding the Cas12 protein and the polynucleotide sequence encoding the guide polynucleotide are located at the same recombinant vector. In some embodiments, the polynucleotide sequence encoding the Cas12 protein and the polynucleotide sequence encoding the guide polynucleotide are located at a plurality of recombinant vectors.

In some embodiments, the polynucleotide sequence encoding the Cas12 protein and/or the polynucleotide sequence encoding the guide polynucleotide is operably linked to a regulatory sequence (also referred to as a regulatory element). The regulatory element comprises a promoter, an enhancer, an internal ribosome entry site (IRES), and other expression control elements (e.g., a transcriptional termination signal such as a polyadenylation signal and a poly-U sequence). The regulatory element comprises an element that enables constitutive expression of a nucleotide sequence in many types of host cells, and an element that enables restrict expression of a nucleotide sequence only in specific host cells (e.g., a tissue-specific regulatory sequence). A tissue-specific promoter may be directly expressed primarily in a desired tissue of interest, e.g., muscle, neuron, bone, skin, blood, a specific organ (e.g., a liver, a pancreas), or a specific cell type (e.g., a lymphocyte). The regulatory element may also guide expression in a time-dependent manner, e.g., in a cell-cycle-dependent or developmental-stage-dependent manner, which may be or may not be tissue-type specific or cell-type specific. In some embodiments, the regulatory element is an enhancer element, e.g., a WPRE, a CMV enhancer, an R-U5 segment in the LTR of HTLV-1, an SV40 enhancer, or an intronic sequence between exons 2 and 3 of rabbit β-globin.

In some embodiments, the recombinant vector comprises a polymerase III (pol III) promoter (e.g., a U6 promoter and an H1 promoter), a polymerase II (pol II) promoter (e.g., a retroviral Rous sarcoma virus (RSV) long terminal repeat (LTR) promoter (optionally with an RSV enhancer), a cytomegalovirus (CMV) promoter (optionally with a CMV enhancer), an SV40 promoter, a dihydrofolate reductase promoter, a β-actin promoter, a phosphoglycerol kinase (PGK) promoter, or an EF1α promoter), or both a pol III promoter and a pol II promoter.

In some embodiments, the promoter is a constitutive promoter, which is continuously active and not modulated by external signals or molecules. Suitable constitutive promoters comprise, but are not limited to, CMV, RSV, SV40, EF1α, CAG, and β-actin promoters. In some embodiments, the promoter is an inducible promoter modulated by an external signal or molecule (e.g., a transcription factor).

In some embodiments, the promoter is a tissue-specific promoter, which may be used to drive tissue-specific expression of the Cas12 protein. Suitable muscle-specific promoters comprise, but are not limited to, CK8, MHCK7, a myoglobin (Mb) promoter, a desmin promoter, a muscle creatine kinase promoter (MCK) and mutants thereof, and an SPc5-12 synthesis promoter. Suitable immune cell-specific promoters comprise, but are not limited to, a B29 promoter (B cells), a CD14 promoter (monocytes), a CD43 promoter (leukocytes and platelets), a CD68 (macrophages) promoter, and an SV40/CD43 promoter (leukocytes and platelets). Suitable blood cell-specific promoters comprise, but are not limited to, a CD43 promoter (leukocytes and platelets), a CD45 promoter (hematopoietic cells), INF-β (hematopoietic cells), a WASP promoter (hematopoietic cells), an SV40/CD43 promoter (leukocytes and platelets), and an SV40/CD45 promoter (hematopoietic cells). Suitable pancreas-specific promoters comprise, but are not limited to, an elastase-1 promoter. Suitable endothelial cell-specific promoters comprise, but are not limited to, a Fit-1 promoter and an ICAM-2 promoter. Suitable neuronal tissue/cell-specific promoters comprise, but are not limited to, a GFAP promoter (astrocytes), an SYN1 promoter (neurons), and NSE/RU5' (mature neurons). Suitable kidney-specific promoters comprise, but are not limited to, a NphsI promoter (podocytes). Suitable bone-specific promoters comprise, but are not limited to, an OG-2 promoter (osteoblasts, dentinogenic cells). Suitable lung-specific promoters comprise, but are not limited to, an SP-B promoter (lung). Suitable liver-specific promoters comprise, but are not limited to, an SV40/Alb promoter. Suitable heart-specific promoters comprise, but are not limited to, α-MHC.

### Adeno-associated virus (AAV) vector

Another aspect of the present disclosure relates to an AAV vector comprising the CRISPR-Cas12 system as described herein, and the AAV vector comprises DNA encoding the Cas12 protein and/or the guide polynucleotide as described herein.

In some embodiments, the AAV vector comprises a DNA sequence encoding the Cas12 protein as described herein. In some embodiments, the AAV vector comprises a DNA sequence encoding the fusion protein as described herein. In some embodiments, the AAV vector comprises a DNA sequence encoding the guide polynucleotide as described herein.

Delivery of the CRISPR-Cas system via the AAV vector was described in Maeder et al., Nature Medicine 25:229-233 (2019*),* the entire contents of which are hereby incorporated by reference. In some embodiments, the AAV vector comprises an ssDNA genome comprising coding sequences for RNA-guided nucleases and the guide RNA, flanked by ITRs.

In some embodiments, the Cas12 protein, the guide polynucleotide, the inactivated Cas12 mutant, the fusion protein or conjugate comprising the Cas12 protein, the isolated nucleic acid, and/or the CRISPR-Cas12 system as described herein are packaged in the AAV vector, e.g., packaged into AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10, or AAVrh74.

In some embodiments, the Cas12 protein, the guide polynucleotide, the inactivated Cas12 mutant, the fusion protein or conjugate comprising the Cas12 protein, the isolated nucleic acid, and/or the CRISPR-Cas12 system as described herein are packaged into AAV2, AAV5, AAV6, AAV8, AAV9, or AAV PHP.eB.

In some embodiments, the AAV vector as described herein is selected from the following: AAV2/2, AAV2/3, AAV2/4, AAV2/5, AAV2/6, AAV2/7, AAV2/8, AAV2/9, AAV2/10, AAV2/11, AAV2/12, AAV2/13, AAV2/PHP.B, AAV2/PHP.B2, AAV2/PHP.B3, AAV2/PHP.A, AAV2/PHP.eB, AAV2/PHP.eS, AAV2/2.7m8, AAV2/8.7m8, AAV2/ShH10, AAV2/rh10, or AAV2/rh74.

In some embodiments, the AAV vector as described herein is selected from the following: AAV2/2, AAV2/5, AAV2/6, AAV2/8, AAV2/9, or AAV2/PHP.eB.

In some embodiments, the CRISPR-Cas12 system as described herein is packaged into the AAV vector, the AAV vector comprising an engineered capsid with tissue tropism, e.g., an engineered eye tissue tropism capsid.

### Lipid nanoparticle

Another aspect of the present disclosure relates to a lipid nanoparticle (LNP) comprising the CRISPR-Cas12 system as described herein, and the LNP comprises the guide polynucleotide and the mRNA encoding the Cas12 protein as described herein.

The LNP delivery of the CRISPR-Cas system was described in Gillmore et al., N. Engl. J. Med. 385:493-502 (2021*).* The LNP is composed of four lipids, comprising a proprietary ionizable lipid LP000001, DSPC, cholesterol, and DMG-PEG2k. An LNP suspension is formulated in an aqueous buffer of Tris, NaCl, and sucrose at pH 7.4. The entire contents of this reference are incorporated herein by reference. In some embodiments, in addition to RNA payloads (Cas12 mRNA and a guide polynucleotide), the LNP further comprises four components: a cationic or ionizable lipid, cholesterol, a helper lipid, and a PEG-lipid. In some embodiments, the cationic or ionizable lipid comprises cKK-E12, C12-200, ALC-0315, DLin-MC3-DMA, DLin-KC2-DMA, FTT5, Moderna SM-102, and Intellia LP01. In some embodiments, the PEG-lipid comprises PEG-2000-C-DMG, PEG-2000-DMG, or ALC-0159. In some embodiments, the helper lipid comprises DSPC. Components of LNP were described in Panuska et al., Nature Reviews Genetics 23:265-280 (2022*).* FDA-approved LNP comprises mutants of four basic components: a cationic or ionizable lipid, cholesterol, a helper lipid, and a polyethylene glycol (PEG) lipid. The entire contents of this reference are incorporated herein by reference.

### Lentiviral vector

Another aspect of the present disclosure relates to a lentiviral vector comprising the CRISPR-Cas12 system as described herein, and the lentiviral vector comprises the guide polynucleotide and the mRNA encoding the Cas12 protein as described herein. In some embodiments, the lentiviral vector is pseudotyped with homologous or heterologous envelope proteins such as VSV-G. In some embodiments, the mRNA encoding the Cas12 protein is linked to an aptamer sequence.

### Ribonucleoprotein (RNP) complex

Another aspect of the present disclosure relates to a RNP complex comprising the CRISPR-Cas12 system as described herein, and the RNP complex is formed by the guide polynucleotide and the Cas12 protein as described herein. In some embodiments, the RNP complex is delivered to eukaryotic cells, mammalian cells, or human cells by microinjection or electroporation. In some embodiments, the RNP complex is packaged into virus-like particles and delivered *in vivo to* mammalian or human subjects.

### Virus-like particle (VLP)

Another aspect of the present disclosure relates to a VLP comprising the CRISPR-Cas12 system as described herein, and the VLP comprises the guide polynucleotide and the Cas12 protein as described herein, or the RNP complex formed by the guide polynucleotide and the Cas12 protein.

The development and application of DNA-free virus-like particles (eVLPs) for efficient packaging and delivery of base editors or Cas9 RNPs was described in Banskota et al., Cell 185(2):250-265 (2022*).* Mangeot et al., Nature Communications 10(1):1-15 (2019*)* revealed engineered murine leukemia virus-like particles (Nanoblades) loaded with Cas9-sgRNA ribonucleoproteins to induce efficient genome editing in cell lines and primary cells (comprising human induced pluripotent stem cells, human hematopoietic stem cells, and mouse bone marrow cells). Campbell et al., Molecular Therapy 27:151-163 (2019*)* revealed specialized extracellular vesicles called "gesicles" to efficiently yet transiently deliver Cas9 ribonucleoproteins targeting the HIV long terminal repeat (LTR) sequence. Gesicles are produced by expressing vesicular stomatitis virus glycoprotein and packaging proteins (as their cargo), thus eliminating the need for transgenic delivery and enabling more precise control over Cas9 expression. Mangeot et al., Molecular Therapy 19(9):1656-1666 (2011*)* revealed that overexpression of the vesicular stomatitis virus glycoprotein (VSV-G) in human cells induces the release of fusogenic vesicles (named gesicles). Biochemical and functional studies showed that glial cells incorporate proteins from producer cells and may deliver them to recipient cells. This protein transduction manner enables the direct transfer of cytoplasmic, nuclear, or surface proteins in target cells. These references all describe engineered VLPs, the entire contents of each of which are incorporated herein by reference.

In some embodiments, the engineered VLP is pseudotyped with homologous or heterologous envelope proteins such as VSV-G. In some embodiments, the Cas12 protein is fused to a gag protein (e.g., MLVgag) via a cleavable linker, and the cleavage of the linker in a target cell exposes an NLS located between the linker and the Cas12 protein. In some embodiments, the fusion protein or conjugate comprises (e.g., from the 5' end to the 3' end) a gag protein (e.g., MLVgag), one or more NESs, a cleavable linker, one or more NLSs, and Cas12, as described in Banskota et al., Cell 185(2):250-265 (2022*).*

In some embodiments, the Cas12 protein is fused to a first dimerization domain that is capable of dimerizing or heterodimerizing with a second dimerization domain fused to a membrane protein, and the presence of a ligand promotes the dimerization and facilitates the enrichment of the Cas12 protein or the fusion protein or conjugate thereof into the VLP, as described in Campbell et al., Molecular Therapy 27:151-163 (2019*).*

### Cell

Another aspect of the present disclosure relates to a cell comprising the CRISPR-Cas12 system as described herein. The cell (e.g., used to generate a cell-free system) may be prokaryotic or eukaryotic. For example, the cell comprises, but is not limited to, bacteria, archaea, plant, fungi, yeast, insect, and mammalian cell, such as *Lactobacillus, Lactococcus, Bacillus* (e.g., *B. subtilis*), *Escherichia* (e.g., *Escherichia coli*), *Clostridium, Saccharomyces* or *Pichia* (e.g., *Saccharomyces cerevisiae* or *Pichia pastoris*), *Kluyveromyces lactis, Salmonella typhimurium,* Drosophila cells, *Caenorhabditis elegans* cell, *Xenopus laevis* cell, SF9 cells, C129 cells, HEK293 cells, *Neurospora,* and immortalized mammalian cell line (e.g., HeLa cell, bone marrow cell line, and lymphoid cell line).

In some embodiments, the cell is a prokaryotic cell such as a bacterial cell (e.g., *Escherichia coli*). In some embodiments, the cell is a eukaryotic cell such as a mammalian cell or a human cell. In some embodiments, the cell is a primary eukaryotic cell, a stem cell, a tumor/cancer cell, a circulating tumor cell (CTC), a blood cell (e.g., a T cell, a B cell, an NK cell, a regulatory T cell (Treg), etc.), a hematopoietic stem cell, a specialized immune cell (e.g., tumor-infiltrating lymphocyte or tumor-suppressive lymphocyte), or a stromal cell in the tumor microenvironment (e.g., a cancer-associated fibroblast, etc.). In some embodiments, the cell is a brain or neuronal cell of the central or peripheral nervous system (e.g., a neuron, an astrocyte, a microglial cell, a retinal ganglion cell, a rod/cone cell, etc.).

### Target nucleic acid or target DNA

In some embodiments, the target nucleic acid is a target DNA.

The CRISPR-Cas12 system as described herein may be used to target one or more target nucleic acid molecules such as target nucleic acid molecules present in biological samples, or environmental samples (e.g., soil, air, or water samples), etc.

In some embodiments, the target nucleic acid is a gene associated with a disease or disorder. In some embodiments, the target nucleic acid is a disease-associated gene. In some embodiments, the disease-associated gene is a pathogenic gene that directly causes the disease. In some embodiments, the disease-associated gene is an abnormal gene that directly causes the disease, or a gene exhibiting abnormal expression. For example, the gene undergoes deleterious mutations, leading to occurrence of disease. As another example, the gene may be overexpressed or underexpressed, resulting in occurrence of disease. In some embodiments, overexpression of the gene leads to disease. In some embodiments, underexpression of the gene leads to disease. In some embodiments, the overexpression of the gene is associated with the occurrence of disease. In some embodiments, the underexpression of the gene is associated with the occurrence of disease.

In some embodiments, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.

The target nucleic acids/target genes and corresponding diseases or disorders were disclosed in Table 27 of the patent application with publication No. WO2025061113A1, which is incorporated herein by reference. These target nucleic acids/target genes may be edited (comprising, but not limited to, introducing an indel, achieving HDR, single-base editing, epigenetic editing, or prime editing, thereby achieving modulation of an expression level of the target nucleic acid or target gene or other related nucleic acids or genes, alteration of the nucleotide sequence, or alteration of the epigenetic modification) by the Cas12 protein, the guide polynucleotide, the inactivated Cas12 mutant, the fusion protein or conjugate comprising the Cas12 protein, the isolated nucleic acid, the CRISPR-Cas12 system, the vector system, the delivery system, the cell, the pharmaceutical composition, and/or the kit as described herein, thereby preventing, diagnosing, or treating the corresponding disease or disorder.

In some embodiments, the target nucleic acid is a reporter gene. Examples of the reporter gene include, but are not limited to, glutathione-S-transferase (GST), horseradish peroxidase (HRP), chloramphenicol acetyltransferase (CAT), β-galactosidase, β-glucuronidase, luciferase, green fluorescent protein (GFP), HcRed, DsRed, cyan fluorescent protein (CFP), yellow fluorescent protein (YFP), and autofluorescent proteins comprising blue fluorescent protein (BFP).

### Use for treatment or prevention of disease

Another aspect of the present disclosure relates to a pharmaceutical composition comprising the Cas12 protein, the guide polynucleotide a, the inactivated Cas12 mutant, the fusion protein or conjugate, the isolated nucleic acid, the CRISPR-Cas12 system, the vector system, the delivery system, or the cell as described herein. For example, the pharmaceutical composition may comprise an AAV vector encoding the Cas12 protein or the inactivated Cas12 mutant and the guide polynucleotide. For example, the pharmaceutical composition may comprise a lipid nanoparticle that comprises the guide polynucleotide and an mRNA encoding the Cas12 protein. For example, the pharmaceutical composition may comprise a lentiviral vector that comprises the guide polynucleotide and the mRNA encoding the Cas12 protein. For example, the pharmaceutical composition may comprise the VLP that comprises the guide polynucleotide and the Cas12 protein, or the RNP complex formed by the guide polynucleotide and the Cas12 protein.

Another aspect of the present disclosure relates to a use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein in cleaving or editing a target nucleic acid in a mammalian cell.

Another aspect of the present disclosure relates to a use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein in any of the following: cleaving one or more target nucleic acid molecules or introducing nicks into the one or more target nucleic acid molecules, activating or upmodulating an expression of the one or more target nucleic acid molecules, activating or inhibiting transcription of the one or more target nucleic acid molecules, inactivating the one or more target nucleic acid molecules, visualizing, labeling, or detecting the one or more target nucleic acid molecules, binding the one or more target nucleic acid molecules, transporting the one or more target nucleic acid molecules, and masking the one or more target nucleic acid molecules.

Another aspect of the present disclosure relates to a use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein in modifying one or more target nucleic acid molecules, and the modifying one or more target nucleic acid molecules comprises one or more of the following: nucleic acid base substitution, nucleic acid base deletion, nucleic acid base insertion, breakage of the target nucleic acid, nucleic acid methylation, and nucleic acid demethylation.

Another aspect of the present disclosure relates to a use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein, in diagnosing, treating, or preventing a disease or disorder associated with the target nucleic acid.

Another aspect of the present disclosure relates to a use of the Cas12 protein, the guide polynucleotide, the inactivated Cas12 mutant, the fusion protein or conjugate, the nucleic acid, the CRISPR-Cas12 system, the vector system, the delivery system, the cell, the pharmaceutical composition, or the kit as described herein in preparing a medicament for diagnosing, treating, or preventing a disease or disorder associated with the target nucleic acid.

In some embodiments, targeted editing is performed on the target nucleic acids/target genes described in table 27 of the patent application with publication No. WO2025061113A1 with the CRISPR-Cas12 system as described herein, thereby preventing, diagnosing, or treating a corresponding disease or disorder.

In some embodiments, the pharmaceutical composition is delivered *in vivo* to a human subject. The pharmaceutical composition may be delivered by any effective route. Exemplary routes of administration include, but are not limited to, intravenous infusion, intravenous injection, intraperitoneal injection, intramuscular injection, intratumoral injection, subcutaneous injection, intradermal injection, intraventricular injection, intravascular injection, intracerebellar injection, intraocular injection, subretinal injection, intravitreal injection, intracameral injection, intratympanic injection, intranasal injection, and inhalation.

### Diagnostic application

Another aspect of the present disclosure relates to an *in* vitro composition that comprises the CRISPR-Cas12 system as described herein and a marked detector DNA that does not hybridize to the guide polynucleotide as described herein.

Another aspect of the present disclosure relates to a use of the CRISPR-Cas12 system as described herein in detecting a target nucleic acid in a nucleic acid sample suspected to contain the target nucleic acid.

Another aspect of the present disclosure relates to a use of the CRISPR-Cas12 system as described herein in detecting a target nucleic acid in a nucleic acid sample that contains the target nucleic acid.

In some embodiments, the detected target nucleic acid is a target RNA.

In some embodiments, the detected target nucleic acid is a target DNA. In some embodiments, a method for detecting the target DNA comprises fusing a Cas12 protein to a fluorescent protein or other detectable marker, and designing a guide polynucleotide containing a guide sequence specific to the target DNA. The binding of Cas12 to the target DNA may be visualized by microscopy or other imaging manners.

In some embodiments, a method for detecting a target nucleic acid in a cell-free system results in production of a detectable marker or enzymatic activity. For example, by using the Cas12 protein, the guide polynucleotide that comprises the guide sequence specific to the target nucleic acid, and the detectable marker, the target nucleic acid may be recognized by Cas12. The binding of Cas12 to the target nucleic acid triggers its DNase activity, which results in cleavage of the target nucleic acid and the detectable marker.

In some embodiments, the detectable marker is DNA linked to a fluorescent probe and a quencher. The complete detectable DNA ligates to the fluorescent probe and quencher, suppressing fluorescence. After the detectable DNA is cleaved by Cas12, the fluorescent probe is released from the quencher and exhibits fluorescent activity. This method may be used to determine whether the target DNA is present in lysed cell samples, lysed tissue samples, blood samples, saliva samples, environmental samples (e.g., water, soil, or air samples), or other lysed cell or cell-free samples. This method may also be used to detect pathogens such as viruses or bacteria, or to diagnose a disease such as cancer.

In some embodiments, the detection of the target nucleic acid is conducive to diagnosing a disease and/or pathological condition, or viral or bacterial infection.

The C12-334, C12-335, C12-336, C12-340, and C12-341 proteins of the present disclosure have DNA cleavage activity. Amino acid sequence lengths of these proteins range from 760aa to 1080aa, which are relatively shorter than amino acid sequence lengths of commonly used SpCas9 protein (1368aa) and AsCpf1 protein (1307aa), and these proteins are more easily packaged by small-capacity gene therapy vectors (e.g., AAV). Moreover, their PAM differs from NGG PAM of SpCas9, thereby expanding the scope of gene editing.

### Examples

The present disclosure is further described below by way of examples, but the present disclosure is not limited to the scope of the examples. The experimental methods without specific conditions in the following examples are performed according to conventional manners and conditions or according to product specifications.

### Example 1. Screening of Cas12 proteins

A plurality of Cas proteins with sequences shown in SEQ ID NOs: 1-35 were obtained through screening using a complex bioinformatics method, as shown in Table 1.

**Table 1. Screened Cas proteins**

| Cas protein | Amino acid sequence (SEQ ID NO) | Cas protein | Amino acid sequence (SEQ ID NO) | Cas protein | Amino acid sequence (SEQ ID NO) |
|---|---|---|---|---|---|
| C12-314 | 1 | C12-329 | 13 | C12-343 | 25 |
| C12-315 | 2 | C12-330 | 14 | C12-344 | 26 |
| C12-316 | 3 | C12-331 | 15 | C12-345 | 27 |
| C12-317 | 4 | C12-332 | 16 | C12-346 | 28 |
| C12-318 | 5 | C12-333 | 17 | C12-348 | 29 |
| C12-319 | 6 | C12-334 | 18 | C12-349 | 30 |
| C12-320 | 7 | C12-335 | 19 | C12-350 | 31 |
| C12-324 | 8 | C12-336 | 20 | C12-351 | 32 |
| C12-325 | 9 | C12-337 | 21 | C12-352 | 33 |
| C12-326 | 10 | C12-340 | 22 | C12-353 | 34 |
| C12-327 | 11 | C12-341 | 23 | C12-354 | 35 |
| C12-328 | 12 | C12-342 | 24 | | |

The DR sequences of the gRNAs corresponding to the Cas proteins are shown in Table 2. When a plurality of DR sequences are present in a single cell of Table 2, the corresponding Cas protein may optionally combine with any one of the plurality of DR sequences for gene editing (i.e., the Cas protein in the table may optionally combine with a guide polynucleotide containing any one corresponding DR sequence for gene editing).

**Table 2. DR sequences corresponding to Cas proteins**

| Cas Protein | Cas_id | DR Sequence (SEQ ID NO) | Cas Protein | Cas_id | DR Sequence |
|---|---|---|---|---|---|
| | | | | | (SEQ ID NO) |
| C12-314 | CI1070721 | 36-37 | C12-335 | CI1080788 | 87-89 |
| C12-315 | CI1058312 | 38-40 | C12-336 | CI1081590 | 90-91 |
| C12-316 | CI1070720 | 41 | C12-337 | CI1081591 | 92-100 |
| C12-317 | CI1076752 | 42-43 | C12-340 | CI1081788 | 101-114 |
| C12-318 | CI1076753 | 44-45 | C12-341 | CI1081790 | 115-116 |
| C12-319 | CI1076756 | 46-47 | C12-342 | CI1081796 | 117-122 |
| C12-320 | CI1076757 | 48-49 | C12-343 | CI1082813 | 123-124 |
| C12-324 | CI1078690 | 50-51 | C12-344 | CI1082815 | 125-133 |
| C12-325 | CI1078840 | 52-58 | C12-345 | CI1082816 | 134-135 |
| C12-326 | CI1080050 | 59-60 | C12-346 | CI1082818 | 136-137 |
| C12-327 | CI1079941 | 61-64 | C12-348 | CI1082995 | 138-141 |
| C12-328 | CI1079971 | 65-69 | C12-349 | CI1083308 | 142-152 |
| C12-329 | CI1079976 | 70-74 | C12-350 | CI1083321 | 153 |
| C12-330 | CI1079843 | 75-76 | C12-351 | CI1083337 | 154 |
| C12-331 | CI1080087 | 77-78 | C12-352 | CI1083596 | 155-160 |
| C12-332 | CI1080100 | 79 | C12-353 | CI1083622 | 161-166 |
| C12-333 | CI1080103 | 80-83 | C12-354 | CI1083653 | 167-170 |
| C12-334 | CI1080716 | 84-86 , 187-195 | | | |

Characteristics of the C12-334, C12-335, C12-336, C12-340, and C12-341 proteins are shown in Table 3.

**Table 3. Characteristics of Cas proteins**

| Cas protein | Length | Predicted subtype based on available data |
|---|---|---|
| C12-334 | 891aa | Cas12h |
| C12-335 | 1068aa | Cas12i |
| C12-336 | 760aa | Cas12h |
| C12-340 | 1008aa | Cas12i |
| C12-341 | 1080aa | Cas12i |

An enzymatic activity center of the C12-334 protein comprises D480, E675, and D757 residues.

An enzymatic activity center of the C12-335 protein comprises D619, E858, and D1035 residues.

An enzymatic activity center of the C12-336 protein comprises D380, E562, and D647 residues.

An enzymatic activity center of the C12-340 protein comprises D609, E827, and D997 residues.

An enzymatic activity center of the C12-341 protein comprises D635, E877, and D1056 residues.

### Example 2. Identification of PAM sequence by in vivo editing assay in bacteria

In the present example, a plasmid library containing a 7nt random sequence was first constructed, then expression plasmids for different Cas proteins were constructed. After the expression plasmids were transformed into bacteria to prepare competent cells, the 7nt random sequence plasmid library was electroporated. If a plasmid in the plasmid library of the 7nt random sequence was recognized and targeted by a Cas protein, the plasmid was cleared from the plasmid library. Bacteria harboring plasmids with corresponding sequences could not grow in a specific antibiotic environment. Specific operations were as follows.

### (1) Construction of the 7nt random sequence plasmid library

A vector plasmid pLVX-EF1a-BSD (SEQ ID NO: 171) was double-digested with EcoRV and XhoI. The linearized vector was recovered by agarose gel electrophoresis and gel extraction. Using the prepared plasmid pCDH-CMV-EGFP-reporter3-EF1-Puro as a template, a primer Puro-PF1 (SEQ ID NO: 172) and a primer Puro-PR1 (SEQ ID NO: 173) were used to obtain a DNA fragment containing a coding sequence of a Puro resistance gene by PCR amplification. The DNA fragment was inserted into the digested vector pLVX-EF1a-BSD by homologous recombination (NEB, Gibson Assembly^{®} Master Mix) to construct a recombinant vector pLVX-7NN-Puro library plasmid (SEQ ID NO: 174) containing the 7nt random sequence. Reaction solution was transformed into Stbl3 competent cells, the cells were plated on an LB plate containing ampicillin and cultured overnight at 37°C, all colonies were scraped, and plasmids were extracted to obtain the library plasmid.

### (2) Synthesis of bacterial expression plasmids for different Cas proteins and preparation of competent cells containing the plasmids

### (a) Synthesis of bacterial expression plasmids for different Cas proteins

Various bacterial expression plasmids were obtained by outsourcing synthesis. Each plasmid expresses a different Cas protein (after codon optimization) and a crRNA (containing a DR sequence corresponding to each Cas protein and a guide sequence targeting the 7nt random sequence plasmid library).

Sequences of the various bacterial expression plasmids are as follows:

>P15A-C12-334-HDV
ATCATAAGATGATCTTCTTGAGATCGTTTTGGTCTGCGCGTAATCTCTTGCTCTGAAAACGAAAAAACC
GCCTTGCAGGGCGGTTTTTCGAAGGTTCTCTGAGCTACCAACTCTTTGAACCGAGGTAACTGGCTTGGA
GGAGCGCAGTCACCAAAACTTGTCCTTTCAGTTTAGCCTTAACCGGCGCATGACTTCAAGACTAACTCC
TCTAAATCAATTACCAGTGGCTGCTGCCAGTGGTGCTTTTGCATGTCTTTCCGGGTTGGACTCAAGACG
ATAGTTACCGGATAAGGCGCAGCGGTCGGACTGAACGGGGGGTTCGTGCATACAGTCCAGCTTGGAGC
GAACTGCCTACCCGGAACTGAGTGTCAGGCGTGGAATGAGACAAACGCGGCCATAACAGCGGAATGAC
ACCGGTAAACCGAAAGGCAGGAACAGGAGAGCGCACGAGGGAGCCGCCAGGGGGAAACGCCTGGTATC
TTTATAGTCCTGTCGGGTTTCGCCACCACTGATTTGAGCGTCAGATTTCGTGATGCTTGTCAGGGGGGC
GGAGCCTATGGAAAAACGGCTTTGCCGCGGCCCTCTCACTTCCCTGTTAAGTATCTTCCTGGCATCTTC

CAGGAAATCTCCGCCCCGTTCGTAAGCCATTTCCGCTCGCCGCAGTCGAACGACCGAGCGTAGCGAGTC
AGTGAGCGAGGAAGCGGAATATATCCTGTATCACATATTCTGCTGACGCACCGGTGCAGCCTTTTTTCT
CCTGCCACATGAAGCACTTCACTGACACCCTCATCAGTGCCAACATAGTAAGCCAGTATACACTCCGCTA
GCGCTGATGTCCGGCGGTGCTTTTGCCGTTACGCACCACCCCGTCAGTAGCTGAACAGGAGGGACAGC
TGATAGAAACAGAAGCCACTGGAGCACCTCAAAAACACCATCATACACTAAATCAGTAAGTTGGCAGCA
TCACCCGACGCACTTTGCGCCGAATAAATACCTGTGACGGAAGATCACTTCGCAGAATAAATAAATCCT
GGTGTCCCTGTTGATACCGGGAAGCCCCGGGCCAACTTTTGGCGAAAATGAGACGTTGATCGGCACGT
AAGAGGTTCCAACTTTCACCATAATGAAATAAGATCACTACCGGGCGTATTTTTTGAGTTATCGAGATTT
TCAGGAGCTAAGGAAGCTAAAATGGAGAAAAAAATCACTGGATATACCACCGTTGATATATCCCAATGG
CATCGTAAAGAACATTTTGAGGCATTTCAGTCAGTTGCTCAATGTACCTATAACCAGACCGTTCAGCTG
GATATTACGGCCTTTTTAAAGACCGTAAAGAAAAATAAGCACAAGTTTTATCCGGCCTTTATTCACATTC
TTGCCCGCCTGATGAATGCTCATCCGGAATTCCGTATGGCAATGAAAGACGGTGAGCTGGTGATATGG
GATAGTGTTCACCCTTGTTACACCGTTTTCCATGAGCAAACTGAAACGTTTTCATCGCTCTGGAGTGAA
TACCACGACGATTTCCGGCAGTTTCTACACATATATTCGCAAGATGTGGCGTGTTACGGTGAAAACCTG
GCCTATTTCCCTAAAGGGTTTATTGAGAATATGTTTTTCGTCTCAGCCAATCCCTGGGTGAGTTTCACC
AGTTTTGATTTAAACGTGGCCAATATGGACAACTTCTTCGCCCCCGTTTTCACCATGGGCAAATATTATA
CGCAAGGCGACAAGGTGCTGATGCCGCTGGCGATTCAGGTTCATCATGCCGTTTGTGATGGCTTCCAT
GTCGGCAGAATGCTTAATGAATTACAACAGTACTGCGATGAGTGGCAGGGCGGGGCGTAATTTGGTAC
ATCTCAAATAAAACGAAAGGCTCAGTCGAAAGACTGGGCCTTTCGTTTTATCTGTTGTTTGTCGGTGAA
CGCTCTCGTTAACTAGCTATACTGATTTCGTCAGACTCACAGTCAAACATGCCGGTCAGTTGGCCTGGT
GATGGCGGGATCGTTGTATATTTCTTGACACCTTTTCGGCACCGCCCTAAAATTCTGCGTCCTCATAAT
ATATGAGGCGATTTATTACGTGTTTACGAAGCAAAAGCTAAAACCAGGAGCTATTTAATGCCGGCAGCT
AAGAAAAAGAAACTGGATGGCAGCGTCGACATGGCCGTGCAGAACGACAGCTGGGACATTCAGAGATG
TCAGAAGCTGAAGCTGGGCAAGAAGGAGCTGAGCCCCATCAACGCCAAGTTCTACGACGACATCCAAGA
GGACTACAGAAAGCTGTTCCCCCTGATCCTGAGCTTCACCCTGACCCCCTACACCTTCGAGGACGAGAA
CGGCGTGGAGCACGTGGTGAGCAGCGAGCAAGTGCTCAAGACCCTGGAGAACAGCGTGGGCAAGAGCC
TGATCGATGATGTGCTGATCATCGGCAGCACCGTGGCCGAGATGCCCCAAGCCTCCGCCTCCTCCTTCT
ACGGCCTCTTCTACAACAACTACAGCTGCAACGACAAGGCCAAGTGGACCCAAGCCAAGAGCGACTTCC
TGGACAAGCTGCTGACCTACACCGACGAGCAGCTGGAGGCCAAGCTGGAGGGCGACAGCTGCCTGAGA
CAGATGCCCCTGGTGGAGTGGAAGAAGGTGAAGGAGAAGCTGCTGGAGGGCAACGACAAGAAAGAGG
TGTGGGAGAGCGTGAGCGGCAAGCTGGCCAACAAGGTGAACAGCAGCTACAGCAGAGTGAGAAAGGAG
CTGGAGATCCAAGTGAGACAGCCCGACAACAGAGAGTACTGCAGCACCCTGAGCGAGATGCTGAGACT
GCAAGTGAAGAGCTGCTATCAGAAGAACCTGGACCATCAGAAGGTGACCCAAGAGCTGCTGACCAAGG
TGAACAACTGCAGCCAAACCAACCCCAAGATCTTCGACCTGATCGCCAACTTCAGCGACAGACTGTACA
GCATCGGCACCGGCCTGAGCAAGAACGTGCTGCTGAGAAGCATCGACTGCGTGAACAAGGGCACCCTG
GCCTCCAACCCCACCTACAAGATCGCCATCGCCGAGCTGCTGAAGCCCGAGTTCAGCGAGATCCTGACC

CTGAAGAAGGAGGAGCTGATCAGAGCCTACAACGGCGTGAAGATCAGAGATCAGCTGAAGAGAAGAAA
GGTGTACCCTAGACTGCCTAGCTTCAAGAATGACTACAAGGTGATGTTCGGCCTGAGCAGCCTGGCCAA
GTTCAAAATCAGAGTGGAGGACAAGAAGATCAAGATCGCCTTCAGCAACGGCGAGGAAGAGCTGTTCA
TGAACAGCCACTACTTCCACGACCTGGAGGTGGTGTTCGACGAGAGCAACAAGACCGCCAAGCAGTTCA
TCATCAAGTTCAGACACAAGCTGAAGTCCAACAAGAAGTTCGCCGTGAGCGACCTGATCACCGGCTATG
TGAAACAAATCGGCCTGCAGAAGAAAAACGGCTCCTTCTATGTGACCCTGATGTTCACCATGAAGCACG
ACGAGAAGATCCTGAAGCTGGAGAGATTCTTCAAGACCGCCTCCCCCGACATGAGCAAGTACACCGACC
TGCCCGACAAGATCAGAGTGGCCGGCTTCGACCTGAACATCAGCAACCCCGTGGTGGGCTGCATCGCC
GAGATCGACAAGAACGGCAAGGGCCCCCTGAACAGCATCGACTTCGGCAAGGGCAACCTGGTGGCCGG
CCCCGACATCGTGTGCCAAGACACCCTGATGAGCAACAGAGTGAAGAGATGCAAGCAGCTGATCTTCAA
GGTGAAGGACGCCATCAAGGACTGCAAGTTCAGCAACAGCAACAACACCAAGATGAACGACGCCACCAT
CAGCTTCCTGAAGAGACTGGCCTCCCCCTCCCAAAGCCCTAGATGCATGATTCAGACCTGGATCAAGAA
CCTGAAGAAGAGACTGAAGAAGCTGCACAGCATCATCAGAGCCTCCGGCTATGTGAACATTAGCGAGG
GCCTGAGAATGCTGGAGGCCCAAGACGCCATGAAAAGCCTGATCAGCAGCTACGAGAGATTCCACCTG
AAGAGCGGCGAGATGCTGGCCGCCAAGAAGAACATCACCGCCAACAACCGGAGACAGAACTTCAGACA
GTTCATCAGCAGAAAGATCGCCTCCAAGATCGTGCAGTACAGCAAGGGCTGCGACGTGATCTTCATCGA
GGACCTGAGCCTGGACTTCGACAGCGACAACAAGAACAACAGCCTGATCAGACTGTTCAGCGCCGACG
GCCTGATCAAGTGCATCACCGACGCCGCCTACAAGGCCGGCATCGGCGTGGTGCTGGTGGACCCCATG
GGCACAAGCAAGACCGACCCCGTGACCGCCAAGGTGGGCTACAGAAACCTGAAGAACAAGAACTACCT
GTACGTGGAGCGGGACGGCGTGCTGGGCTGGGTGGACGCCGACAAGATCGCCTCCCTGAACGTGCTG
ATCAGAGGCCTGGGCCACAGCATCGTGCCCTACAAGTTCTACGTGAAGGGCAAGAAAAAGGACGTGAT
CGGCGTGGACCTGGTGGAGAAAGAGGTGGGCAAGAGACTGCAGAGATACTTCACCATGCAGCACGGCA
GCATCAAGCAGCCCATCTTCAAGATCGACAACGACAAGGTGACCCTGCTGAAGAAGGCCAACAAGGGCG
ACAACCTGATCGAGAACGCCTTCCTGTACGCCCACGGCGACGACTTCTGCACCGCCGACAACCACAGAA
ACCAAGGCAAGGAGATCATGCACAGAGTGGACAGCGGCGAGCCCGTGGTGGAGTTCGACCTGACCCCC
TGCAGCGAGAGCGGCTACAAGAGCTTCCAAGCCAAGACAGGCGGCGGCCCCGGCGGCGGCGCCGCCGC
CGGCAGCGGCAGCCCTAAGAAAAAACGAAAAGTTGGCAGCGGAAGCAAAAGGCCGGCGGCCACGAAAA
AGGCCGGCCAGGCAAAAAAGAAAAAGCTCGAGTACCCATACGATGTTCCAGATTACGCTTGAGAATTCG
GTACCTTGACAGCTAGCTCAGTCCTAGGTATAATACTAGTGTGCGAAACGGTCTCGTTAGAGGCTG
GTTCAAGCACCGCAATGATGATCTCCGAGCCGTTCGGCCGGCATGGTCCCAGCCTCCTCGCTGGC
GCCGGCTGGGCAACATGCTTCGGCATGGCGAATGGGACCGTACGTCGACGCTAGCATAACCCCTTGGG
GCCTCTAAACGGGTCTTGAGGGGTTTTTTGGAT (SEQ ID NO: 176).

There are a plasmid P15A-C12-335-HDV (SEQ ID NO: 177), a plasmid P15A-C12-336-HDV (SEQ ID NO: 178), a plasmid P15A-C12-340-HDV (SEQ ID NO: 179), a plasmid P15A-C12-341-HDV (SEQ ID NO: 180).

The underlined and non-bold sequence is a coding sequence of the Cas protein. The bold and italicized sequence is a coding sequence of the crRNA (gRNA). The bold, italicized, and underlined sequence is a coding sequence of the guide sequence.

A map of the vector P15A-C12-334-HDV is exemplarily shown in FIG. 1.

### (b) Preparation of competent cells containing different bacterial expression plasmids

Each bacterial expression plasmid for the Cas protein was transformed into DH5a competent cells. A single colony was picked and inoculated into an LB medium containing chloramphenicol and cultured overnight at 37°C.

Electrocompetent cells were prepared according to the following operations.

The culture bacterial solution was inoculated into 100 mL of fresh LB medium containing chloramphenicol at a ratio of 1:100 for scale-up cultivation at 37°C and 220 rpm.

When OD600 reached 0.5, the culture bacterial solution was transferred to a 50 mL centrifuge tube, and pre-cooled on ice for 30 min.

The culture bacterial solution was centrifuged at 4000 rpm for 10 min at 4°C to collect cells, and the cells were resuspended in an equal volume of pre-cooled sterile water.

The above operations were repeated.

The cells were resuspended in 1/10 volume of pre-cooled sterile water containing 10% glycerol and aliquoted at 50 µL per tube, and competent cells containing bacterial expression plasmids of Cas proteins were obtained and stored at -80°C.

### (c) Performing plasmid curing to identify a PAM sequence of the Cas protein

100 ng pLVX-7NN-Puro library plasmid was electroporated into competent cells containing bacterial expression plasmids of the Cas proteins and into DH5a competent cells, respectively and marked as Lib1 (electroporated into competent cells containing the bacterial expression plasmids of the Cas proteins) and Lib2 (electroporated into DH5a competent cells), respectively.

After electroporation, 10 mL of LB medium was added, and the cells were resuscitated by culturing at 37°C and 220 rpm for 2 h.

The resuscitated bacterial solution was centrifuged at 4000 rpm for 2 min to collect the cells. The cells were resuspended in 400 µL of LB medium and plated on an LB plate. The bacterial solution electroporated into DH5a competent cells was plated on an LB plate containing ampicillin, the bacterial solution electroporated into the competent cells containing bacterial expression plasmids of the Cas proteins was plated on an LB plate containing chloramphenicol and ampicillin, and the bacterial solutions were cultured overnight at 37°C.

Bacterial cells were scraped from each culture plate, and plasmid DNA was extracted by alkaline lysis.

100 ng of each of the two extracted plasmid DNAs was used as a PCR template. A primer SiteSeq-PF1 (SEQ ID NO: 181) and a primer SiteSeqPuro-PR (SEQ ID NO: 182) were used for PCR amplification (as shown in FIG. 2) to obtain fragments. The obtained fragments were used for amplicon library construction using an NGS library construction kit (Xunshi Biotechnology, SynplSeq DNA Library Prep Kit for Illumina), followed by NGS sequencing. A specific library construction process is detailed in a kit instruction.

NGS sequencing differences between the Lib1 cells and the Lib2 cells were compared and analyzed, and PAM sequences are identified based on captured sequences, as shown in FIGs. 3-7.

Results show that a PAM sequence recognized by C12-334 is 5'-WYR-3'; a PAM sequence recognized by C12-335 is 5'-BMCTTH-3'; a PAM sequence recognized by C12-336 is 5'-TTN-3'; a PAM sequence recognized by C12-340 is 5'-VNWTV-3', 5'-VNWTC-3', or 5'-VNTTC-3'; and a PAM sequence recognized by C12-341 is 5'-TTN-3'. W is A or T, Y is C or T, R is A or G, B is C, G, or T, M is A or C, H is A, T, or C, N is A, T, C, or G, and V is A, C, or G.

### Example 3. Cleavage activity of C12-334 protein on target nucleic acid in 293T cells

In the present example, an sgRNA targeting *TTR* genes in HEK293T cells was first designed, and a guide sequence is a sequence shown in SEQ ID NO: 183. An expression cassette for the C12-334 protein and an expression cassette of the sgRNA were cloned into a commonly used mammalian expression vector pCDNA3.1(+) to obtain an expression vector plasmid C12-334-TTR-sgRNA05 (SEQ ID NO: 184). After the expression vector plasmid was transfected into HEK293T cells, the cleavage activity of the C12-334 protein in 293T cells was verified by NGS sequencing.

### Detection of TTR gene editing efficiency

Plating: 293T cells were plated when a cell confluence reached 70-80%, and a count of cells seeded in a 24-well plate was 5*10^5 cells/well.

Transfection: Transfection was performed 12-14 h after plating. 100 µL Opti-MEM, 1.5 µL Polyethylenimine (PEI) (Yeasen Biotechnology, MW25000), and 500 ng plasmid C12-334-TTR-sgRNA05 were added to each well of a 24-well plate, mixed, and added to 293T cells for cell transfection after placing at room temperature for 20 min. After overnight transfection, a fresh culture medium was replaced, and culture was continued.

DNA extraction, PCR amplification, and NGS library construction: After 72 h of culture, the cells were washed with PBS, and then 100 µL of cell lysis solution (Viagen, DirectPCR^{®} Lysis Reagent (Cell)) was added for lysis to obtain lysate containing genomic DNA. The region near the target sequence was amplified for the genomic DNA using the primers TTR-NGS-PF1 (SEQ ID NO: 185) and TTR-NGS-PR1 (SEQ ID NO: 186). The PCR product was subjected to the NGS library construction and sequencing, and the sequencing result was analyzed. The indel efficiency is higher than 23.58%, as shown in FIG. 8.

### Example 4. Construction of different reporter system cell lines

A fluorescent reporter system is an important tool for evaluating gene editing efficiency, which primarily reflect the occurrence of editing events by monitoring changes in fluorescent signals. Common fluorescent reporter systems comprise a reporter system that restores GFP coding to produce a fluorescent signal by introducing an Indel (insertion/deletion), and a fluorescent system that restores GFP expression through single-strand annealing (SSA). An SSA fluorescent reporter system was designed based on "single-strand annealing repair pathway" in a DNA double-strand break repair mechanism.

For the above two scenarios, a pCDH-CMV-GFP-Reporter3-EF1a-Puro cell line (abbreviated as Reporter3 cell line) that restores GFP coding to produce a fluorescent signal based on indel, and a pCDH-CMV-SSA-GXXFP3 cell line (abbreviated as SSA cell line) that restores GFP coding to produce the fluorescent signal based on SSA were constructed.

Construction strategy for the two different cell lines: different GFP expression modules were inserted into a lentiviral plasmid backbone, followed by lentiviral packaging and infection to integrate the different modules into the chromosomes of HEK293 cells. Different cell lines with stably inherited fluorescent systems were obtained through drug selection.

Core sequence of the GFP expression module for constructing the Reporter3 cell line is as follows:

tctagagcgagaaaagccttgtttgccaccATGGAACGGCTCGGAGATCATCATTGCGTCGCGAGGTGAGCA
AGGGCGAGGAGCTGTTCACCGGGGTGGTGCCCATCCTGGTCGAGCTGGACGGCGACGTAAACGGCCAC
AAGTTCAGCGTGTCCGGCGAGGGCGAGGGCGATGCCACCTACGGCAAGCTGACCCTGAAGTTCATCTG
CACCACCGGCAAGCTGCCCGTGCCCTGGCCCACCCTCGTGACCACCCTGACCTACGGCGTGCAGTGCTT
CAGCCGCTACCCCGACCACATGAAGCAGCACGACTTCTTCAAGTCCGCCATGCCCGAAGGCTACGTCCA
GGAGCGCACCATCTTCTTCAAGGACGACGGCAACTACAAGACCCGCGCCGAGGTGAAGTTCGAGGGCG
ACACCCTGGTGAACCGCATCGAGCTGAAGGGCATCGACTTCAAGGAGGACGGCAACATCCTGGGGCAC
AAGCTGGAGTACAACTACAACAGCCACAACGTCTATATCATGGCCGACAAGCAGAAGAACGGCATCAAG
GTGAACTTCAAGATCCGCCACAACATCGAGGACGGCAGCGTGCAGCTCGCCGACCACTACCAGCAGAAC
ACCCCCATCGGCGACGGCCCCGTGCTGCTGCCCGACAACCACTACCTGAGCACCCAGTCCAAGCTGAGC
AAAGACCCCAACGAGAAGCGCGATCACATGGTCCTGCTGGAGTTCGTGACCGCCGCCGGGATCACTCTC
GGCATGGACGAGCTGTACAAGTAAgcggccgc (SEQ ID NO: 175).

The uppercase base sequence is a GFP expression cassette, which is not an integer multiple of 3 (causing frameshift). The bold uppercase sequence is the target region. After editing, indels are introduced in the region, which may restore the normal reading frame of GFP, enabling normal GFP expression and fluorescence production. The designed spacer sequence is the bold and underlined uppercase sequence, i.e., GAACGGCTCGGAGATCATCATTG (SEQ ID NO: 196), and a corresponding PAM is ATG.

Core sequence of the GFP expression module for constructing the SSA cell line is as follows:

tctaggccaccATGGTGAGCAAGGGCGAGGAGCTGTTCACCGGGGTGGTGCCCATCCTGGTCGAGCTGGA
CGGCGACGTAAACGGCCACAAGTTCAGCGTGTCCGGCGAGGGCGAGGGCGATGCCACCTACGGCAAGC
TGACCCTGAAGTTCATCTGCACCACCGGCAAGCTGCCCGTGCCCTGGCCCACCCTCGTGACCACCCTGA
CCTACGGCGTGCAGTGCTTCAGCCGCTACCCCGACCACATGAAGCAGCACGACTTCTTCAAGTCCGCCA
TGCCCGAAGGCTACGTCCAGGAGCGCACCATCTTCTTCAAGGACGACGGCAACTACAAGACCCGCGCCG
AGGTGAAGTTCGAGGGCGACACCCTGGTGAACCGCATCGAGCTGAAGGGCATCGACTTCAAGGAGGAC
GGCAACATCCTGGGGCACAAGCTGGAGTACAACTACAACAGCCACAACGTCTATATCATGGCCTTCCCT
TTATCTCTTAGGGATAACAGGGTAATAGAGATAAAGTAGGATGGAACGGCTCGGAGATCATC
ATTGCGTAAGGCCTAAGATAGTAATATAGCCATGCCCGAAGGCTACGTCCAGGAGCGCACCATCTT
CTTCAAGGACGACGGCAACTACAAGACCCGCGCCGAGGTGAAGTTCGAGGGCGACACCCTGGTGAACC
GCATCGAGCTGAAGGGCATCGACTTCAAGGAGGACGGCAACATCCTGGGGCACAAGCTGGAGTACAAC
TACAACAGCCACAACGTCTATATCATGGCCGACAAGCAGAAGAACGGCATCAAGGTGAACTTCAAGATC
CGCCACAACATCGAGGACGGCAGCGTGCAGCTCGCCGACCACTACCAGCAGAACACCCCCATCGGCGAC
GGCCCCGTGCTGCTGCCCGACAACCACTACCTGAGCACCCAGTCCGCCCTGAGCAAAGACCCCAACGAG
AAGCGCGATCACATGGTCCTGCTGGAGTTCGTGACCGCCGCCGGGATCACTCTCGGCATGGACGAGCT
GTACAAGTAAAGCGGCCGCGACTCTAGtctaggcggccgc (SEQ ID NO: 197).

The uppercase base sequence is the GFP expression cassette, which is not an integer multiple of 3 (causing frameshift) and contains an SSA homologous fragment (as shown in the uppercase, non-bold, and underlined sequence). The bold uppercase portion is the target region, and editing of the region leads to homologous fragment recombination, thereby restoring the normal reading frame of GFP, enabling normal GFP expression and fluorescence production. The bold and underlined uppercase sequence is the designed spacer sequence, i.e., GAACGGCTCGGAGATCATCATTG (SEQ ID NO: 198), a corresponding PAM is ATG.

The lentiviral backbone plasmids for constructing the two different cell lines are as follows: pCDH-CMV-GFP-Reporter3-EF1a-Puro (SEQ ID NO: 199) and pCDH-CMV-SSA-GXXFP3 (SEQ ID NO: 200).

### Example 5. Construction of different mutants by site-directed mutagenesis of C12-334 protein

The C12-334 protein has an amino acid sequence as follows:

MAVQNDSWDIQRCQKLKLGKKELSPINAKFYDDIQEDYRKLFPLILSFTLTPYTFEDENGVEHVVSSEQVLK
TLENSVGKSLIDDVLIIGSTVAEMPQASASSFYGLFYNNYSCNDKAKWTQAKSDFLDKLLTYTDEQLEAKLE
GDSCLRQMPLVEWKKVKEKLLEGNDKKEVWESVSGKLANKVNSSYSRVRKELEIQVRQPDNREYCSTLSE
MLRLQVKSCYQKNLDHQKVTQELLTKVNNCSQTNPKIFDLIANFSDRLYSIGTGLSKNVLLRSIDCVNKGTL
ASNPTYKIAIAELLKPEFSEILTLKKEELIRAYNGVKIRDQLKRRKVYPRLPSFKNDYKVMFGLSSLAKFKIRVE

DKKIKIAFSNGEEELFMNSHYFHDLEVVFDESNKTAKQFIIKFRHKLKSNKKFAVSDLITGYVKQIGLQKKNG
SFYVTLMFTMKHDEKILKLERFFKTASPDMSKYTDLPDKIRVAGFDLNISNPVVGCIAEIDKNGKGPLNSIDF
GKGNLVAGPDIVCQDTLMSNRVKRCKQLIFKVKDAIKDCKFSNSNNTKMNDATISFLKRLASPSQSPRCMI
QTWIKNLKKRLKKLHSIIRASGYVNISEGLRMLEAQDAMKSLISSYERFHLKSGEMLAAKKNITANNRRQNF
RQFISRKIASKIVQYSKGCDVIFIEDLSLDFDSDNKNNSLIRLFSADGLIKCITDAAYKAGIGVVLVDPMGTSK
TDPVTAKVGYRNLKNKNYLYVERDGVLGWVDADKIASLNVLIRGLGHSIVPYKFYVKGKKKDVIGVDLVEK
EVGKRLQRYFTMQHGSIKQPIFKIDNDKVTLLKKANKGDNLIENAFLYAHGDDFCTADNHRNQGKEIMHR
VDSGEPVVEFDLTPCSESGYKSFQAK (SEQ ID NO: 18).

### 1) Mutation template plasmid information

The sequence of C12-334 and the target sequence of the corresponding reporter system were first constructed into a plasmid pCDNA3.1(+), and a resistance gene was changed from Amp to Kan, and a template plasmid C12-334-pCDHPAM02 (SEQ ID NO: 201) was obtained. The template plasmid encodes the C12-334 protein and a gRNA, where the gRNA guide sequence is CAATGATGATCTCCGAGCCGTTC (SEQ ID NO: 202).

Different mutation clones were obtained by designing two mutation primers F/R at mutation sites to introduce a required mutation sequence. Combined with universal primers at both ends of the vector, PCR amplification was performed to obtain two mutation fragments F1 and F2. The mutation fragments F1 and F2 are then combined with the enzymatically linearized vector to obtain the mutant plasmid through homologous recombination. Taking the two amino acid positions 115 and 697 as examples, the following describes how to construct single-point mutant (N115R, D697R) and multi-point mutant (N115R&D697R) plasmids. Specific operations were as follows.

Primers for constructing site-directed mutagenesis at positions 115 and 697 are shown in Table 4.

**Table 4. Primers**

| Primer Name | Primer Sequence |
|---|---|
| ChkCas12-PF1 | SEQ ID NO: 203 |
| ChkCas12-PR4 | SEQ ID NO: 204 |
| 334_115_F | gctgcAGAgacaaggccaagtggac (SEQ ID NO: 205) |
| 334_115_R | cttggccttgtcTCTgcagctgtagttgttgtag (SEQ ID NO: 206) |
| 334_697_F | gcgccAGAggcctgatcaagtgcatc (SEQ ID NO: 207) |
| 334_697_R | cttgatcaggccTCTggcgctgaacagtctgatc (SEQ ID NO: 208) |

Using plasmid C12-334-pCDHPAM02 as template, a mutation fragment N115R-F1 was obtained by PCR amplification (Ultrahipf^{™} DNA Polymerase Kit) with primers ChkCas12-PF1+334_115_R, and a mutation fragment N115R-F2 was obtained by PCR amplification with primers ChkCas12-PR4+334_115_F. The plasmid C12-334-pCDHPAM02 was digested with HindIII+KpnI, and 5665 bp vector fragment was gel-purified. *In vitro recombination* (NEB, Gibson Assembly^{®} Master Mix) was performed on vector fragment and fragments N115R-F1 and N115R-F2, followed by heat-shock transformation into *Escherichia coli* to obtain a mutant plasmid C12-334-pCDHPAM02-115 with site-directed mutagenesis at position 115.

Using the same method, the primer 334_115_F was replaced with the primer 334_697_F, the primer 334_115_R was replaced with the primer 334_697_R, and a mutant plasmid C12-334-pCDHPAM02-697 with site-directed mutagenesis at position 697 was obtained. Except for the need to design mutation primers targeting each different site, specific construction method for Cas protein mutant plasmids at other different positions was consistent with the method described above for constructing the mutant plasmids at positions 115 and 697.

### 2) Verification of editing activity of Cas protein mutants

Reporter3 or SSA cell culture and plating: cells were plated when the cell lines reached a confluence of 70-80%, and a count of cells seeded in a 24-well plate was 5×10^5 cells/well.

Transfection: transfection was performed 12-14 h after plating. 1.5 µL of PEI (Yeasen Biotechnology) and 500 ng mutant plasmid were added to100 µL of Opti-MEM of each well of a 24-well plate, mixed and placed at room temperature for 20 min, then added to a corresponding cell line for cell transfection. After overnight transfection, a fresh medium was replaced, and culture was continued for an additional 72 h. Flow cytometry was used for detection, and the editing efficiency of different mutation clones was characterized based on a percentage of GFP-positive cells. The fold change in the editing efficiency of each mutant was calculated relative to the wild-type protein. The test was repeated, and results were averaged.

### 3) Construction of combination mutation clones and validation of editing activity

Based on the editing efficiency results of the different mutation sites described above, different mutation sites were selected for multiple mutation combinations. The construction of the double-site mutant plasmid at positions 115 and 697 was used as an example.

Using the plasmid C12-334-pCDHPAM02 as a template, a mutation fragment N115R-F1 was obtained by PCR amplification with primers ChkCas12-PF1+334_115_R, a mutation fragment N115R-D697R-F1 was obtained by the PCR amplification with primers 334_115_F+334_697_R, and a mutation fragment D697R-F2 was obtained by the PCR amplification with primers ChkCas12-PR4 +334_697_F. The plasmid C12-334-pCDHPAM02 was digested with HindIII and KpnI, and a 5665bp vector fragment was gel-purified. The vector fragment was subjected to *in vitro* recombination with fragments N115R-F1, N115R-D697R-F1, and D697R-F2, followed by heat-shock transformation into *Escherichia coli* to obtain a mutant plasmid C12-334-pCDH-115-697. The construction method for plasmids with other multi-site mutations were consistent with the construction method for C12-334-pCDH-115-697.

Using the same method as described previously in the example, the editing activity of the multi-site mutants was validated using the SSA cell line. The editing efficiency of different mutation clones was characterized based on the percentage of GFP-positive cells. The fold change in the editing efficiency of each mutant was calculated relative to the wild-type protein. The test was repeated, and results were averaged.

The editing efficiency results for various mutants (single-site mutants or multi-site mutants) are shown in Table 5 and Table 6. The data in the tables are the average values of the repeated test results.

The editing efficiency of multi-site mutants in the SSA cell line is shown in FIGs. 10A-10B.

**Table 5. Editing efficiency of various mutants in the reporter3 cell line**

| Group (wild-type/ mutant/ control) | Editing efficiency (%) | Group (wild-type/ mutant/ control) | Editing efficiency (%) | Group (wild-type/ mutant/ control) | Editing efficiency (%) |
|---|---|---|---|---|---|
| Wild-Type C12-334 | 28.45 | I294R | 31.64 | L589R | 10.80 |
| PEI Control | 0.45 | A295R | 24.94 | K590R | 3.26 |
| Cell Line Control | 0.39 | I296R | 31.71 | K591R | 32.32 |
| S91R & V3G | 31.49 | A297R | 30.15 | L592R | 18.93 |
| N110R & S113C | 28.80 | E298R | 21.76 | H593R | 20.42 |
| M1R | 33.63 | L299R | 5.01 | S594R | 30.05 |
| A2R | 29.69 | L300R | 29.98 | I595R | 31.36 |
| V3R | 33.28 | K301R | 30.34 | I596R | 11.21 |
| Q4R | 33.85 | P302R | 29.73 | A598R | 30.80 |
| N5R | 33.09 | E303R | 23.95 | S599R | 26.98 |
| D6R | 30.25 | F304R | 32.14 | G600R | 25.11 |
| S7R | 30.69 | S305R | 22.46 | Y601R | 0.54 |
| W8R | 1.70 | E306R | 16.52 | V602R | 33.78 |
| D9R | 33.44 | I307R | 5.29 | N603R | 31.78 |
| I10R | 23.55 | L308R | 26.46 | I604R | 20.06 |
| Q11R | 35.70 | T309R | 28.35 | S605R | 0.92 |
| C13R | 2.89 | L310R | 29.56 | E606R | 4.62 |
| Q14R | 6.33 | K311R | 29.49 | G607R | 7.03 |
| K15R | 29.27 | K312R | 33.83 | L608E | 5.69 |
| L16R | 14.20 | E313R | 32.18 | M610R | 11.44 |
| K17R | 22.52 | E314R | 29.43 | L611R | 7.30 |
| L18R | 16.94 | L315R | 24.14 | E612R | 11.77 |
| G19R | 23.44 | I316R | 28.01 | A613R | 1.53 |
| K20R | 21.84 | A318R | 12.39 | Q614R | 30.29 |
| K21R | 27.34 | Y319R | 21.38 | D615R | 1.12 |
| E22R | 32.21 | N320R | 28.58 | A616R | 26.07 |
| L23R | 14.54 | G321R | 20.65 | M617R | 13.59 |
| S24R | 26.90 | V322R | 16.12 | K618R | 34.03 |
| P25R | 22.59 | K323R | 25.93 | S619R | 31.55 |
| I26R | 29.12 | I324R | 31.25 | L620R | 3.41 |
| N27R | 12.85 | D326R | 25.00 | I621R | 29.06 |
| A28R | 34.67 | Q327R | 35.22 | S622R | 0.90 |
| K29R | 27.47 | L328R | 22.84 | S623R | 31.20 |
| F30R | 7.42 | K329R | 32.42 | Y624R | 1.51 |
| Y31R | 5.76 | K332R | 18.14 | E625R | 14.13 |
| D32R | 30.51 | K332R | 27.54 | F627R | 13.25 |
| D33R | 6.81 | V333R | 28.40 | H628R | 24.23 |
| I34R | 0.50 | Y334R | 8.51 | L629R | 21.14 |
| Q35R | 20.41 | P335R | 11.51 | K630R | 31.54 |
| E36R | 32.22 | L337R | 29.95 | S631R | 28.35 |
| D37R | 0.86 | P338R | 26.01 | G632R | 29.80 |
| Y38R | 27.61 | S339R | 29.65 | E633R | 29.09 |
| K40R | 31.71 | F340R | 22.92 | M634R | 35.51 |
| L41R | 12.98 | K341R | 32.33 | L635R | 33.74 |
| F42R | 19.83 | N342R | 28.86 | A636R | 30.76 |
| P43R | 27.92 | D343R | 7.74 | A637R | 28.51 |
| L44R | 28.36 | Y344R | 27.22 | K638R | 33.41 |
| I45R | 0.58 | K345R | 21.99 | K639R | 22.99 |
| L46R | 25.09 | V346R | 17.49 | N640R | 33.61 |
| S47R | 28.92 | M347R | 1.37 | I641R | 33.84 |
| F48R | 0.38 | F348R | 18.45 | T642R | 36.42 |
| T49R | 2.34 | G349R | 4.86 | A643R | 32.78 |
| L50R | 30.23 | L350R | 18.64 | N644R | 30.13 |
| T51R | 11.38 | S351R | 4.48 | N645R | 35.42 |
| P52R | 7.28 | S352R | 6.12 | Q648R | 35.52 |
| Y53R | 19.62 | L353R | 12.30 | N649R | 35.10 |
| T54R | 29.49 | A354R | 23.86 | F650R | 12.42 |
| F55R | 14.92 | K355R | 27.00 | Q652R | 34.93 |
| E56R | 29.08 | F356R | 1.05 | F653R | 28.41 |
| D57R | 25.43 | K357R | 24.30 | I654R | 3.95 |
| E58R | 32.32 | I358R | 0.86 | S655R | 23.01 |
| N59R | 26.35 | V360R | 20.11 | K657R | 33.55 |
| G60R | 29.22 | E361R | 24.17 | I658R | 3.59 |
| V61R | 25.27 | D362R | 25.56 | S660R | 31.94 |
| E62R | 22.52 | K363R | 28.13 | K661R | 33.97 |
| H63R | 25.31 | K364R | 31.01 | I662R | 0.88 |
| V64R | 28.35 | I365R | 18.38 | V663R | 26.23 |
| V65R | 28.69 | K366R | 26.16 | Q664R | 29.50 |
| S66R | 28.58 | I367R | 1.76 | Y665R | 24.08 |
| S67R | 27.18 | A368R | 24.91 | S666R | 11.95 |
| E68R | 29.65 | F369R | 14.39 | K667R | 32.92 |
| Q69R | 30.56 | S370R | 21.53 | G668R | 30.75 |
| V70R | 29.73 | N371R | 28.95 | C669R | 12.00 |
| L71R | 29.84 | G372R | 25.65 | D670R | 27.67 |
| K72R | 31.32 | E373R | 20.00 | V671R | 0.66 |
| T73R | 31.79 | E374R | 15.37 | I672R | 9.38 |
| L74R | 6.96 | E375R | 5.69 | F673R | 0.50 |
| E75R | 28.28 | L376R | 21.89 | I674R | 6.95 |
| N76R | 29.55 | F377R | 16.55 | E675R | 0.77 |
| S77R | 27.49 | M378R | 29.16 | D676R | 23.17 |
| V78R | 22.30 | M378R | 25.06 | L677R | 0.90 |
| G79R | 29.22 | N379R | 33.98 | S678R | 32.37 |
| K80R | 15.72 | S380R | 23.41 | L679R | 0.28 |
| S81R | 32.14 | H381R | 26.19 | D680R | 29.63 |
| L82R | 26.64 | Y382R | 23.06 | F681R | 10.65 |
| I83R | 27.59 | F383R | 0.84 | D682R | 31.50 |
| D84R | 36.23 | H384R | 31.31 | S683R | 34.36 |
| D85R | 19.26 | D385R | 29.89 | D684R | 23.16 |
| V86R | 8.51 | L386R | 22.14 | N685R | 32.36 |
| L87R | 27.97 | E387R | 31.77 | K686R | 33.49 |
| I88R | 37.88 | E387R | 28.47 | N687R | 23.24 |
| I89R | 0.51 | V388R | 16.01 | N688R | 22.01 |
| G90R | 1.94 | V389R | 27.88 | S689R | 32.39 |
| S91R | 37.76 | F390R | 35.76 | L690R | 25.46 |
| T92R | 25.68 | D391R | 23.31 | I691R | 33.01 |
| V93R | 0.72 | E392R | 20.14 | L693R | 24.37 |
| A94R | 31.22 | S393R | 31.13 | F694R | 0.73 |
| E95R | 32.75 | N394R | 29.92 | S695R | 7.76 |
| M96R | 31.06 | K395R | 31.12 | A696R | 1.97 |
| P97R | 27.51 | T396R | 29.98 | D697R | 35.72 |
| Q98R | 1.42 | A397R | 27.08 | G698R | 37.24 |
| A99R | 32.96 | K398R | 25.25 | L699R | 17.08 |
| S100R | 33.87 | Q399R | 27.51 | I700R | 30.97 |
| A101R | 5.46 | F400R | 13.72 | K701R | 15.08 |
| S102R | 1.12 | I401R | 26.85 | C702R | 32.18 |
| S103R | 0.49 | I402R | 0.85 | I703R | 0.92 |
| F104R | 2.86 | K403R | 26.98 | T704R | 36.59 |
| Y105R | 0.56 | F404R | 10.71 | D705R | 32.79 |
| G106R | 0.48 | H406R | 32.24 | A706R | 30.12 |
| L107R | 0.63 | K407R | 33.69 | A707R | 8.24 |
| F108R | 0.62 | L408R | 31.82 | Y708R | 27.96 |
| Y109R | 6.59 | K409R | 33.37 | K709R | 33.15 |
| N110R | 27.92 | S410R | 30.82 | A710R | 34.38 |
| N111R | 8.19 | N411R | 28.47 | G711R | 24.04 |
| Y112R | 0.43 | K412R | 33.23 | I712R | 33.42 |
| S113R | 29.45 | K413R | 33.71 | G713R | 20.03 |
| C114R | 25.50 | F414R | 25.41 | V714R | 29.53 |
| N115R | 34.66 | A415R | 32.39 | V715R | 1.01 |
| D116R | 22.03 | V416R | 11.44 | L716R | 27.07 |
| K117R | 30.51 | S417R | 29.44 | V717R | 0.61 |
| A118R | 20.59 | D418R | 0.69 | D718R | 1.59 |
| K119R | 20.39 | L419R | 37.08 | P719R | 5.98 |
| W120R | 14.99 | I420R | 24.74 | M720R | 34.14 |
| T121R | 1.54 | T421R | 21.61 | G721R | 0.90 |
| Q122R | 26.84 | G422R | 21.08 | T722R | 0.61 |
| A123R | 33.11 | Y423R | 26.33 | S723R | 0.57 |
| K124R | 29.46 | V424R | 0.72 | K724R | 22.99 |
| S125R | 29.20 | K425R | 27.83 | T725R | 0.89 |
| D126R | 31.11 | Q426R | 23.80 | D726R | 0.96 |
| F127R | 9.86 | I427R | 2.13 | P727R | 1.23 |
| L128R | 0.32 | G428R | 7.87 | V728R | 5.17 |
| D129R | 26.23 | L429R | 3.71 | T729R | 1.36 |
| K130R | 24.46 | Q430R | 11.45 | A730R | 0.61 |
| L131R | 0.56 | K431R | 37.26 | K731R | 35.27 |
| L132R | 29.02 | K432R | 35.47 | V732R | 28.03 |
| T133R | 17.21 | N433R | 30.87 | G733R | 0.82 |
| Y134R | 25.23 | G434R | 29.56 | Y734R | 10.17 |
| T135R | 24.60 | S435R | 17.80 | N736R | 0.78 |
| D136R | 22.39 | F436R | 16.59 | L737R | 34.13 |
| E137R | 23.41 | Y437R | 24.36 | K738R | 25.18 |
| Q138R | 22.73 | V438R | 0.85 | N739R | 26.78 |
| L139R | 6.70 | T439R | 3.51 | K740R | 5.58 |
| E140R | 29.42 | L440R | 0.74 | N741R | 31.89 |
| A141R | 30.65 | M441R | 13.62 | Y742R | 35.96 |
| K142R | 29.86 | F442R | 16.37 | L743R | 0.53 |
| L143R | 11.20 | T443R | 32.66 | Y744R | 2.89 |
| E144R | 28.01 | M444R | 30.43 | V745R | 0.33 |
| G145R | 26.54 | K445R | 31.89 | E746R | 28.87 |
| D146R | 19.87 | H446R | 26.24 | D748R | 14.80 |
| S147R | 34.22 | D447R | 30.63 | G749R | 22.55 |
| C148R | 30.43 | E448R | 32.83 | V750R | 18.04 |
| L149R | 18.20 | K449R | 32.61 | L751R | 2.38 |
| Q151R | 32.40 | I450R | 30.25 | G752R | 0.82 |
| M152R | 8.96 | L451R | 34.45 | W753R | 34.80 |
| P153R | 26.61 | K452R | 32.52 | V754R | 1.08 |
| L154R | 25.23 | L453R | 15.84 | D755R | 0.89 |
| V155R | 28.41 | E454R | 27.31 | A756R | 0.96 |
| E156R | 26.28 | F456R | 0.80 | D757R | 0.69 |
| W157R | 2.15 | F457R | 0.71 | K758R | 29.99 |
| K158R | 26.70 | K458R | 32.47 | I759R | 0.50 |
| K159R | 28.37 | T459R | 22.92 | A760R | 0.70 |
| V160R | 11.98 | A460R | 0.54 | S761R | 0.93 |
| K161R | 27.24 | S461R | 29.31 | L762R | 0.82 |
| E162R | 27.54 | P462R | 3.77 | N763R | 0.38 |
| K163R | 19.64 | D463R | 29.13 | V764R | 0.55 |
| L164R | 13.77 | M464R | 22.43 | L765R | 0.32 |
| L165R | 29.05 | S465R | 33.97 | I766R | 1.07 |
| E166R | 32.92 | K466R | 31.90 | G768R | 0.68 |
| G167R | 21.73 | Y467R | 14.03 | L769R | 1.07 |
| N168R | 28.04 | T468R | 32.57 | G770R | 0.41 |
| D169R | 25.89 | D469R | 23.84 | H771R | 17.96 |
| K170R | 25.16 | L470R | 12.79 | S772R | 0.54 |
| K171R | 29.21 | P471R | 26.57 | I773R | 1.40 |
| E172R | 24.81 | D472R | 29.91 | V774R | 1.00 |
| V173R | 6.60 | K473R | 34.19 | P775R | 0.86 |
| W174R | 2.20 | I474R | 4.47 | Y776R | 0.46 |
| E175R | 27.60 | V476R | 0.75 | K777R | 31.85 |
| S176R | 19.48 | A477R | 4.22 | F778R | 0.85 |
| V177R | 10.64 | G478R | 0.72 | Y779R | 17.40 |
| S178R | 27.98 | F479R | 0.58 | V780R | 0.47 |
| G179R | 17.63 | D480R | 0.65 | K781R | 30.21 |
| K180R | 32.34 | L481R | 0.60 | G782R | 26.00 |
| L181R | 0.69 | N482R | 0.62 | K783R | 30.69 |
| A182R | 27.05 | I483R | 0.61 | K784R | 32.09 |
| N183R | 23.92 | S484R | 14.88 | K785R | 28.76 |
| K184R | 20.44 | N485R | 24.55 | D786R | 24.91 |
| V185R | 18.49 | P486R | 0.63 | V787R | 26.96 |
| N186R | 17.88 | V487R | 0.79 | I788R | 28.49 |
| S187R | 30.18 | V488R | 0.65 | G789R | 16.87 |
| S188R | 29.42 | G489R | 1.24 | V790R | 33.06 |
| Y189R | 1.11 | C490R | 0.72 | D791R | 31.43 |
| S190R | 26.63 | I491R | 26.90 | L792R | 24.75 |
| V192R | 0.54 | A492R | 0.23 | V793R | 33.10 |
| K194R | 25.98 | E493R | 30.97 | E794R | 25.42 |
| E195R | 19.84 | I494R | 0.65 | K795R | 32.04 |
| L196R | 15.87 | D495R | 30.56 | E796R | 32.13 |
| E197R | 29.11 | K496R | 17.10 | V797R | 24.12 |
| I198R | 10.55 | N497R | 19.30 | G798R | 25.11 |
| Q199R | 36.01 | G498R | 8.61 | K799R | 20.69 |
| V200R | 2.49 | K499R | 22.07 | L801R | 8.61 |
| Q202R | 20.88 | G500R | 0.67 | Q802R | 19.00 |
| P203R | 27.77 | P501R | 14.81 | Y804R | 0.35 |
| D204R | 27.37 | L502R | 0.59 | F805R | 2.75 |
| N205R | 23.27 | N503R | 29.70 | T806R | 30.30 |
| E207R | 10.02 | S504R | 0.51 | M807R | 0.68 |
| Y208R | 0.74 | I505R | 1.32 | Q808R | 15.30 |
| L209R | 0.57 | D506R | 0.82 | H809R | 4.03 |
| S210R | 18.48 | F507R | 2.40 | G810R | 1.95 |
| T211R | 1.42 | G508R | 0.61 | S811R | 19.85 |
| S213R | 1.50 | K509R | 34.03 | I812R | 31.11 |
| S213R | 1.39 | G510R | 0.55 | K813R | 33.38 |
| E214R | 28.64 | N511R | 31.47 | Q814R | 23.54 |
| M215R | 5.04 | L512R | 15.74 | P815R | 0.99 |
| M215R | 8.20 | V513R | 27.49 | I816R | 22.83 |
| L216R | 0.69 | A514R | 32.61 | F817R | 0.66 |
| L218R | 30.10 | G515R | 9.47 | K818R | 31.96 |
| Q219R | 24.09 | P516R | 0.60 | I819R | 25.76 |
| V220R | 0.89 | D517R | 26.15 | D820R | 27.58 |
| K221R | 25.91 | I518R | 26.18 | N821R | 30.85 |
| S222R | 0.47 | V519R | 13.99 | D822R | 24.26 |
| C223R | 21.67 | C520R | 3.28 | K823R | 30.81 |
| Y224R | 26.09 | Q521R | 1.57 | V824R | 0.65 |
| Q225R | 27.99 | D522R | 2.05 | T825R | 32.46 |
| K226R | 32.41 | T523R | 15.36 | L826R | 7.12 |
| N227R | 33.06 | L524R | 32.99 | L827R | 22.77 |
| L228R | 30.12 | M525R | 31.24 | K828R | 33.03 |
| D229R | 34.72 | S526R | 16.58 | K829R | 28.86 |
| H230R | 14.28 | N527R | 30.09 | A830R | 6.95 |
| H230R | 13.53 | V529R | 0.90 | N831R | 27.82 |
| Q231R | 27.40 | K530R | 2.96 | K832R | 32.65 |
| K232R | 32.17 | C532R | 12.22 | G833R | 28.19 |
| V233R | 25.63 | K533R | 32.22 | D834R | 11.65 |
| T234R | 19.94 | N533R | 30.82 | N835R | 34.84 |
| Q235R | 29.86 | Q534R | 28.44 | L836R | 19.55 |
| E236R | 30.37 | L535R | 29.05 | I837R | 0.52 |
| L237R | 27.71 | I536R | 1.86 | E838R | 22.14 |
| L238R | 34.03 | F537R | 24.68 | N839R | 23.18 |
| T239R | 8.24 | K538R | 29.95 | A840R | 28.67 |
| K240R | 32.14 | V539R | 2.30 | F841R | 5.64 |
| V241R | 28.22 | K540R | 28.14 | L842R | 0.83 |
| N242R | 30.03 | D541R | 29.47 | Y843R | 0.42 |
| N243R | 30.29 | A542R | 21.66 | A844R | 0.80 |
| C244R | 27.01 | I543R | 7.28 | H845R | 2.39 |
| S245R | 31.10 | K544R | 30.49 | G846R | 0.41 |
| Q246R | 25.53 | D545R | 29.24 | D847R | 25.80 |
| T247R | 33.33 | C546R | 23.37 | D848R | 30.64 |
| N248R | 25.98 | K547R | 28.35 | F849R | 0.37 |
| P249R | 28.91 | F548R | 35.54 | C850R | 33.81 |
| K250R | 12.47 | S549R | 30.38 | T851R | 16.68 |
| I251R | 31.11 | N550R | 23.58 | A852R | 29.95 |
| F252R | 24.17 | S551R | 29.23 | D853R | 26.45 |
| D253R | 28.09 | N552R | 30.70 | N854R | 32.40 |
| L254R | 22.12 | N553R | 1.18 | H855R | 10.63 |
| I255R | 0.48 | T554R | 29.10 | N857R | 27.37 |
| A256R | 25.45 | K555R | 29.73 | Q858R | 28.00 |
| N257R | 16.67 | M556R | 27.68 | G859R | 36.28 |
| F258R | 0.85 | N557R | 30.31 | K860R | 33.98 |
| S259R | 28.70 | D558R | 27.01 | E861R | 28.42 |
| D260R | 26.35 | A559R | 29.96 | I862R | 3.15 |
| L262R | 1.89 | T560R | 26.82 | M863R | 28.90 |
| Y263R | 33.47 | I561R | 29.81 | H864R | 33.69 |
| S264R | 24.02 | S562R | 31.94 | V866R | 15.97 |
| I265R | 28.31 | F563R | 0.71 | D867R | 34.88 |
| G266R | 23.18 | L564R | 20.47 | S868R | 30.39 |
| G266R | 27.89 | L564R | 17.89 | G869R | 20.02 |
| T267R | 33.58 | K565R | 28.90 | E870R | 14.04 |
| G268R | 30.41 | L567R | 28.44 | P871R | 22.50 |
| L269R | 26.39 | A568R | 23.55 | V872R | 25.39 |
| S270R | 33.73 | S569R | 26.39 | V873R | 32.77 |
| K271R | 29.71 | S569R | 19.35 | E874R | 0.79 |
| N272R | 33.34 | P570R | 25.92 | F875R | 1.67 |
| K273R | 28.88 | P570R | 4.32 | D876R | 21.29 |
| V273R | 25.78 | S571R | 3.36 | L877R | 1.89 |
| L274R | 0.46 | Q572R | 19.35 | T878R | 24.05 |
| L275R | 33.04 | S573R | 25.17 | P879R | 19.17 |
| S277R | 8.06 | S573R | 1.67 | C880R | 24.96 |
| I278R | 25.62 | P574R | 17.81 | S881R | 0.63 |
| D279R | 32.05 | C576R | 20.57 | E882R | 9.18 |
| C280R | 26.16 | M577R | 14.41 | S883R | 29.08 |
| V281R | 29.64 | M577R | 27.78 | G884R | 9.67 |
| N282R | 15.44 | I578R | 1.91 | Y885R | 0.75 |
| K283R | 27.47 | I578R | 2.56 | K886R | 30.93 |
| G284R | 31.32 | Q579R | 12.90 | S887R | 0.41 |
| T285R | 28.48 | T580R | 22.44 | F888R | 0.39 |
| L286R | 26.72 | T580R | 27.95 | Q889R | 23.94 |
| A287R | 28.86 | W581R | 20.78 | A890R | 11.47 |
| S288R | 27.18 | I582R | 1.38 | K891R | 31.09 |
| N289R | 35.75 | K583R | 24.17 | A659R | 2.76 |
| P290R | 26.27 | N584R | 29.48 | I662R | 0.98 |
| T291R | 26.61 | L585R | 20.32 | D670R | 0.83 |
| Y292R | 19.69 | K586R | 28.45 | K117R & G106S | 5.47 |
| K293R | 33.99 | K587R | 23.50 | S126R & Q138H | 14.405 |
| K117R & G106S | 5.47 | K432R & E314K | 2.085 | M807R & L801M | 0.73 |
| S126R & Q138H | 14.405 | I494M & G498R | 5.63 | M634R & G782S | 31.53 |
| D136Y & C209R | 0.54 | M215I & P501R | 16.805 | D684R & S683C | 22.59 |
| C223R & N342S | 15.7 | A460T & S504R | 0.5 | M378R & G349D | 2.6 |
| Q521R & V487M | 14.045 | N511R & P516H | 25.845 | V750R & V671M | 22.3 |
| V388R & D418E | 19.825 | K565R & P570S | 25.325 | F55R & N59T | 20.55 |
| K312R & I316N & R317S & A318G & Y319F & N320S & G321S | 13.865 | S605R & L502Q | 0.79 | | |
| I427R & Y423S | 1.595 | K724R & D726A | 0.88 | | |

**Table 6. Editing efficiency of various mutants in the SSA cell line**

| Group (wild-type/ mutant/ control) | Editing efficiency (%) | Group (wild-type/ mutant/ control) | Editing efficiency (%) | Group (wild-type/ mutant/ control) | Editing efficiency (%) | Group (wild-type/ mutant/ control) | Editing efficiency (%) |
|---|---|---|---|---|---|---|---|
| Wild-Type C12-334 | 12.72 | Y224R | 4.13 | T443R | 17.70 | C669R | 1.19 |
| Cell Line Control | 0.01 | Q225R | 14.95 | M444R | 7.05 | D670R | 6.24 |
| PEI Control | 0.03 | K226R | 10.38 | K445R | 13.26 | D670R | 0.01 |
| M1R | 10.05 | N227R | 9.36 | H446R | 7.90 | V671R | 0.01 |
| A2R | 10.44 | L228R | 13.19 | D447R | 5.82 | I672R | 0.30 |
| V3R | 16.54 | D229R | 12.96 | E448R | 8.29 | F673R | 0.00 |
| Q4R | 9.56 | H230R | 0.94 | K449R | 6.34 | I674R | 0.33 |
| N5R | 13.68 | H230R | 0.92 | I450R | 9.59 | E675R | 0.01 |
| D6R | 13.62 | Q231R | 6.69 | L451R | 12.54 | D676R | 6.70 |
| S7R | 7.72 | K232R | 10.86 | K452R | 6.43 | L677R | 0.00 |
| W8R | 0.02 | V233R | 15.53 | L453R | 4.64 | S678R | 16.13 |
| D9R | 16.11 | T234R | 11.39 | E454R | 4.36 | L679R | 0.04 |
| I10R | 6.39 | Q235R | 10.43 | F456R | 0.00 | D680R | 9.79 |
| C13R | 0.02 | E236R | 12.37 | F457R | 0.01 | D682R | 13.98 |
| Q14R | 0.39 | L237R | 6.88 | K458R | 15.95 | S683R | 11.98 |
| K15R | 11.41 | L238R | 13.14 | T459R | 4.43 | D684R | 4.61 |
| L16R | 2.11 | T239R | 0.29 | A460R | 0.01 | N685R | 15.16 |
| K17R | 6.56 | K240R | 7.48 | S461R | 9.60 | K686R | 16.07 |
| L18R | 3.97 | V241R | 9.25 | P462R | 0.06 | N687R | 6.38 |
| G19R | 9.24 | N242R | 8.45 | D463R | 8.98 | N688R | 2.67 |
| K20R | 14.79 | N243R | 8.17 | M464R | 4.20 | S689R | 14.47 |
| K21R | 13.80 | C244R | 10.05 | S465R | 10.39 | L690R | 5.33 |
| E22R | 8.56 | S245R | 12.88 | K466R | 13.92 | I691R | 18.90 |
| L23R | 1.69 | Q246R | 9.89 | Y467R | 1.49 | L693R | 6.50 |
| S24R | 5.95 | T247R | 11.80 | T468R | 14.43 | F694R | 0.01 |
| P25R | 6.03 | N248R | 9.40 | D469R | 7.28 | S695R | 1.07 |
| I26R | 9.73 | P249R | 9.63 | L470R | 0.77 | A696R | 0.15 |
| N27R | 2.18 | K250R | 11.02 | P471R | 6.52 | D697R | 16.77 |
| A28R | 14.26 | I251R | 6.32 | D472R | 8.76 | G698R | 13.78 |
| K29R | 13.72 | F252R | 7.61 | K473R | 10.22 | L699R | 2.97 |
| F30R | 0.50 | D253R | 8.42 | I474R | 0.10 | I700R | 13.01 |
| Y31R | 0.18 | L254R | 11.84 | V476R | 0.00 | K701R | 6.32 |
| D32R | 11.74 | I255R | 0.02 | A477R | 0.16 | C702R | 10.10 |
| D33R | 0.55 | A256R | 11.87 | G478R | 0.01 | I703R | 0.01 |
| I34R | 0.02 | N257R | 6.77 | F479R | 0.01 | T704R | 16.63 |
| Q35R | 3.31 | F258R | 0.03 | D480R | 0.01 | D705R | 14.96 |
| E36R | 10.73 | S259R | 14.42 | L481R | 0.01 | A706R | 14.33 |
| D37R | 0.02 | D260R | 14.70 | N482R | 0.00 | A707R | 0.45 |
| Y38R | 9.04 | L262R | 0.12 | I483R | 0.00 | Y708R | 9.39 |
| K40R | 11.39 | Y263R | 14.04 | S484R | 8.22 | K709R | 9.81 |
| L41R | 1.08 | S264R | 11.28 | N485R | 12.81 | A710R | 12.60 |
| F42R | 2.33 | I265R | 13.78 | P486R | 0.01 | G711R | 10.97 |
| P43R | 5.96 | G266R | 13.97 | V487R | 0.00 | I712R | 9.98 |
| L44R | 9.73 | G266R | 13.72 | V488R | 0.00 | G713R | 2.27 |
| I45R | 0.02 | T267R | 15.36 | G489R | 0.05 | V714R | 10.56 |
| L46R | 9.34 | G268R | 6.90 | C490R | 0.01 | V715R | 0.02 |
| S47R | 8.10 | L269R | 7.02 | I491R | 11.04 | L716R | 9.26 |
| F48R | 0.01 | S270R | 9.69 | A492R | 0.01 | V717R | 0.03 |
| T49R | 0.06 | K271R | 10.29 | E493R | 6.82 | D718R | 0.14 |
| L50R | 7.20 | N272R | 9.99 | I494R | 0.00 | P719R | 0.88 |
| T51R | 6.04 | K273R | 9.29 | D495R | 11.28 | M720R | 16.79 |
| P52R | 0.31 | V273R | 6.41 | K496R | 10.65 | G721R | 0.03 |
| Y53R | 3.47 | L274R | 0.06 | G500R | 0.00 | T722R | 0.01 |
| T54R | 5.17 | L275R | 12.07 | P501R | 2.42 | S723R | 0.01 |
| F55R | 14.60 | S277R | 1.23 | L502R | 0.00 | K724R | 8.86 |
| E56R | 12.62 | I278R | 13.61 | N503R | 10.65 | T725R | 0.01 |
| D57R | 5.41 | D279R | 11.40 | S504R | 0.01 | D726R | 0.00 |
| E58R | 8.79 | C280R | 11.01 | I505R | 0.05 | P727R | 0.01 |
| N59R | 5.18 | V281R | 8.60 | D506R | 0.01 | V728R | 0.41 |
| G60R | 7.55 | N282R | 12.36 | G508R | 0.00 | T729R | 0.03 |
| V61R | 7.48 | K283R | 14.94 | K509R | 9.29 | A730R | 0.01 |
| E62R | 11.15 | G284R | 9.04 | G510R | 0.00 | K731R | 12.98 |
| H63R | 13.45 | T285R | 11.47 | N511R | 13.99 | V732R | 7.27 |
| V64R | 12.05 | L286R | 14.12 | L512R | 2.25 | G733R | 0.01 |
| V65R | 7.14 | A287R | 9.76 | V513R | 8.45 | Y734R | 0.48 |
| S66R | 5.10 | S288R | 13.58 | A514R | 9.29 | N736R | 0.00 |
| S67R | 13.17 | N289R | 12.15 | G515R | 6.03 | L737R | 9.91 |
| E68R | 13.26 | P290R | 9.39 | P516R | 0.00 | K738R | 7.09 |
| Q69R | 9.89 | T291R | 4.74 | D517R | 6.57 | N739R | 12.24 |
| V70R | 11.69 | Y292R | 10.37 | I518R | 8.60 | K740R | 2.00 |
| L71R | 12.97 | K293R | 13.71 | V519R | 2.78 | N741R | 13.81 |
| K72R | 9.49 | I294R | 12.45 | C520R | 0.11 | Y742R | 18.11 |
| T73R | 11.55 | A295R | 7.78 | Q521R | 0.02 | L743R | 0.02 |
| L74R | 0.39 | I296R | 8.33 | D522R | 0.66 | Y744R | 0.04 |
| E75R | 7.27 | A297R | 7.76 | T523R | 4.44 | V745R | 0.01 |
| N76R | 9.81 | E298R | 6.82 | L524R | 11.62 | E746R | 8.35 |
| S77R | 10.52 | L299R | 0.27 | M525R | 11.83 | D748R | 5.46 |
| V78R | 4.63 | L300R | 14.86 | S526R | 2.56 | G749R | 4.33 |
| G79R | 9.60 | K301R | 13.31 | N527R | 10.96 | V750R | 13.01 |
| K80R | 13.85 | P302R | 7.26 | V529R | 0.00 | L751R | 0.08 |
| S81R | 13.17 | E303R | 13.61 | K530R | 0.01 | G752R | 0.02 |
| L82R | 6.69 | F304R | 7.90 | C532R | 1.22 | W753R | 13.76 |
| I83R | 7.30 | S305R | 13.57 | K533R | 14.83 | V754R | 0.02 |
| D84R | 13.00 | E306R | 7.77 | N533R | 12.19 | D755R | 0.01 |
| D85R | 8.18 | I307R | 0.69 | Q534R | 9.74 | A756R | 0.01 |
| V86R | 0.31 | L308R | 11.27 | L535R | 8.11 | D757R | 0.00 |
| L87R | 4.82 | T309R | 10.59 | I536R | 0.20 | K758R | 7.70 |
| I89R | 0.00 | L310R | 7.62 | F537R | 16.80 | I759R | 0.00 |
| G90R | 0.02 | K311R | 7.37 | K538R | 11.61 | A760R | 0.00 |
| T92R | 5.42 | K312R | 10.56 | V539R | 0.07 | S761R | 0.00 |
| V93R | 0.01 | E313R | 10.31 | K540R | 6.35 | L762R | 0.00 |
| A94R | 10.16 | E314R | 11.30 | D541R | 6.25 | N763R | 0.02 |
| E95R | 16.32 | L315R | 9.47 | A542R | 11.86 | V764R | 0.00 |
| M96R | 12.31 | I316R | 10.42 | I543R | 0.35 | L765R | 0.01 |
| P97R | 10.98 | A318R | 8.30 | K544R | 11.23 | I766R | 0.00 |
| Q98R | 2.39 | Y319R | 5.50 | D545R | 11.83 | G768R | 0.01 |
| A99R | 11.64 | N320R | 13.86 | C546R | 6.95 | L769R | 0.00 |
| S100R | 12.17 | G321R | 4.66 | K547R | 8.11 | G770R | 0.03 |
| A101R | 0.12 | V322R | 6.83 | F548R | 16.09 | H771R | 6.88 |
| S102R | 0.02 | K323R | 9.27 | S549R | 9.61 | S772R | 0.01 |
| S103R | 0.00 | I324R | 7.71 | N550R | 14.53 | I773R | 0.01 |
| F104R | 0.38 | D326R | 0.70 | S551R | 10.92 | V774R | 0.00 |
| Y105R | 0.00 | Q327R | 12.00 | N552R | 11.66 | P775R | 0.00 |
| G106R | 0.02 | L328R | 5.91 | N553R | 13.75 | Y776R | 0.01 |
| L107R | 0.01 | K329R | 10.92 | T554R | 12.51 | K777R | 8.28 |
| F108R | 0.01 | K332R | 7.60 | K555R | 12.28 | F778R | 0.01 |
| Y109R | 0.60 | K332R | 12.23 | M556R | 10.56 | Y779R | 2.76 |
| N110R | 10.45 | V333R | 9.24 | N557R | 14.84 | V780R | 0.00 |
| N111R | 15.89 | Y334R | 1.73 | D558R | 10.99 | K781R | 12.43 |
| Y112R | 0.00 | P335R | 0.53 | A559R | 9.12 | G782R | 6.68 |
| S113R | 13.47 | L337R | 11.78 | T560R | 9.96 | K783R | 12.60 |
| C114R | 6.42 | P338R | 8.50 | I561R | 10.72 | K784R | 12.18 |
| N115R | 10.76 | S339R | 13.80 | S562R | 11.12 | K785R | 13.90 |
| D116R | 14.32 | F340R | 6.16 | F563R | 0.03 | D786R | 12.92 |
| K117R | 9.40 | K341R | 10.22 | L564R | 3.68 | V787R | 14.37 |
| A118R | 3.56 | N342R | 10.30 | L564R | 5.16 | I788R | 15.62 |
| K119R | 2.67 | D343R | 1.55 | K565R | 12.12 | G789R | 4.10 |
| W120R | 0.91 | Y344R | 4.35 | L567R | 9.32 | V790R | 14.87 |
| T121R | 0.02 | K345R | 2.68 | A568R | 7.26 | D791R | 11.23 |
| Q122R | 9.10 | V346R | 1.68 | S569R | 7.15 | L792R | 15.17 |
| A123R | 5.71 | M347R | 0.05 | P570R | 9.13 | V793R | 13.42 |
| K124R | 10.41 | F348R | 2.40 | P570R | 6.44 | E794R | 8.03 |
| S125R | 11.62 | G349R | 0.14 | S571R | 11.45 | K795R | 11.69 |
| D126R | 11.47 | L350R | 5.63 | Q572R | 12.56 | E796R | 7.92 |
| F127R | 0.15 | S351R | 0.88 | S573R | 6.68 | V797R | 12.82 |
| L128R | 0.00 | S352R | 0.16 | S573R | 4.72 | G798R | 5.97 |
| D129R | 5.88 | L353R | 1.24 | P574R | 7.66 | L801R | 1.69 |
| K130R | 12.33 | A354R | 5.80 | C576R | 10.43 | Q802R | 11.00 |
| L131R | 0.01 | K355R | 7.53 | M577R | 7.07 | Y804R | 0.01 |
| L132R | 5.27 | F356R | 0.04 | M577R | 10.20 | F805R | 0.11 |
| T133R | 6.94 | K357R | 13.48 | I578R | 0.02 | T806R | 15.78 |
| Y134R | 2.10 | I358R | 0.02 | I578R | 0.39 | M807R | 0.01 |
| T135R | 7.14 | V360R | 10.17 | Q579R | 1.15 | Q808R | 1.61 |
| D136R | 4.66 | E361R | 11.12 | T580R | 8.58 | H809R | 0.24 |
| E137R | 10.97 | D362R | 14.16 | T580R | 12.05 | G810R | 0.31 |
| Q138R | 10.05 | K363R | 10.84 | W581R | 4.36 | S811R | 5.44 |
| L139R | 0.06 | K364R | 8.83 | I582R | 0.05 | I812R | 5.96 |
| E140R | 11.82 | I365R | 5.81 | K583R | 10.29 | K813R | 11.09 |
| A141R | 11.67 | K366R | 13.80 | N584R | 11.77 | Q814R | 6.76 |
| K142R | 18.31 | I367R | 0.02 | L585R | 5.04 | P815R | 0.01 |
| L143R | 8.52 | A368R | 13.85 | K586R | 9.58 | I816R | 7.21 |
| E144R | 11.53 | F369R | 2.29 | K587R | 11.01 | F817R | 0.05 |
| G145R | 14.18 | S370R | 4.52 | L589R | 1.07 | K818R | 10.52 |
| D146R | 10.92 | N371R | 14.44 | K590R | 0.15 | I819R | 6.70 |
| S147R | 9.92 | G372R | 2.20 | K591R | 11.78 | D820R | 8.29 |
| C148R | 13.66 | E373R | 8.82 | L592R | 3.89 | N821R | 13.69 |
| L149R | 1.24 | E374R | 5.93 | H593R | 6.60 | D822R | 9.47 |
| Q151R | 12.30 | E375R | 0.95 | S594R | 12.03 | K823R | 13.93 |
| M152R | 0.87 | L376R | 10.26 | I595R | 12.37 | V824R | 0.02 |
| P153R | 9.81 | F377R | 3.54 | I596R | 1.77 | T825R | 9.42 |
| L154R | 7.32 | M378R | 16.55 | A598R | 13.36 | L826R | 0.69 |
| V155R | 9.78 | M378R | 15.86 | S599R | 10.25 | L827R | 3.58 |
| E156R | 3.38 | N379R | 11.75 | G600R | 1.28 | K828R | 15.33 |
| W157R | 0.03 | S380R | 11.93 | Y601R | 0.01 | K829R | 8.74 |
| K158R | 12.24 | H381R | 9.68 | V602R | 8.75 | A830R | 0.53 |
| K159R | 7.28 | Y382R | 9.00 | N603R | 8.29 | N831R | 8.82 |
| V160R | 0.54 | F383R | 0.02 | I604R | 2.38 | K832R | 10.57 |
| K161R | 13.60 | H384R | 6.82 | S605R | 0.03 | G833R | 9.88 |
| E162R | 10.13 | D385R | 6.65 | E606R | 0.13 | D834R | 0.76 |
| K163R | 12.38 | L386R | 7.13 | G607R | 0.48 | N835R | 11.33 |
| L164R | 0.42 | E387R | 8.76 | L608E | 0.41 | L836R | 2.79 |
| L165R | 6.73 | E387R | 12.93 | M610R | 0.70 | I837R | 0.02 |
| E166R | 9.49 | V388R | 5.03 | L611R | 0.43 | E838R | 4.68 |
| G167R | 10.98 | V389R | 8.80 | E612R | 1.37 | N839R | 3.79 |
| N168R | 9.02 | F390R | 11.55 | A613R | 0.04 | A840R | 6.45 |
| D169R | 9.86 | D391R | 3.90 | Q614R | 6.01 | F841R | 0.33 |
| K170R | 7.17 | E392R | 12.93 | D615R | 0.01 | L842R | 0.04 |
| K171R | 13.45 | S393R | 12.91 | A616R | 7.06 | Y843R | 0.01 |
| E172R | 8.69 | N394R | 18.18 | M617R | 1.43 | A844R | 0.02 |
| V173R | 0.19 | K395R | 6.75 | K618R | 11.70 | H845R | 0.07 |
| W174R | 0.20 | T396R | 8.66 | S619R | 10.96 | G846R | 0.01 |
| E175R | 14.72 | A397R | 10.69 | L620R | 0.16 | D847R | 4.59 |
| S176R | 12.06 | K398R | 13.13 | I621R | 8.89 | D848R | 8.72 |
| V177R | 0.87 | Q399R | 12.53 | S622R | 0.01 | F849R | 0.00 |
| S178R | 10.25 | F400R | 2.41 | S623R | 12.05 | C850R | 9.18 |
| G179R | 9.44 | I401R | 5.43 | Y624R | 0.06 | T851R | 1.23 |
| K180R | 7.54 | I402R | 0.02 | E625R | 2.47 | A852R | 7.64 |
| L181R | 0.01 | K403R | 13.05 | F627R | 5.26 | D853R | 11.28 |
| A182R | 11.18 | F404R | 1.06 | H628R | 7.04 | N854R | 13.21 |
| N183R | 7.30 | H406R | 6.27 | L629R | 3.36 | H855R | 0.68 |
| K184R | 11.56 | K407R | 9.72 | K630R | 7.67 | N857R | 12.90 |
| V185R | 4.33 | L408R | 6.87 | S631R | 13.74 | Q858R | 9.36 |
| N186R | 3.46 | K409R | 10.45 | G632R | 8.47 | G859R | 15.08 |
| S187R | 10.86 | S410R | 12.56 | E633R | 5.59 | K860R | 9.57 |
| S188R | 10.09 | N411R | 10.50 | M634R | 12.41 | E861R | 8.81 |
| Y189R | 0.01 | K412R | 12.99 | L635R | 10.62 | I862R | 0.08 |
| S190R | 9.69 | K413R | 9.74 | A636R | 11.56 | M863R | 11.58 |
| V192R | 0.07 | F414R | 9.45 | A637R | 6.96 | H864R | 12.82 |
| K194R | 10.33 | A415R | 5.73 | K638R | 9.51 | V866R | 1.74 |
| E195R | 14.00 | V416R | 12.15 | K639R | 9.79 | D867R | 14.39 |
| L196R | 7.04 | S417R | 5.44 | N640R | 7.05 | S868R | 10.66 |
| E197R | 10.82 | D418R | 0.00 | I641R | 12.08 | G869R | 4.12 |
| I198R | 1.18 | L419R | 10.22 | T642R | 16.06 | E870R | 2.28 |
| Q199R | 14.70 | I420R | 12.51 | A643R | 12.94 | P871R | 4.50 |
| V200R | 0.08 | T421R | 7.03 | N644R | 13.68 | V872R | 2.74 |
| Q202R | 4.23 | G422R | 2.19 | N645R | 10.50 | V873R | 10.81 |
| P203R | 7.46 | Y423R | 4.72 | Q648R | 9.49 | E874R | 0.03 |
| D204R | 10.45 | V424R | 0.01 | N649R | 11.36 | F875R | 0.02 |
| N205R | 6.62 | K425R | 2.87 | F650R | 2.72 | D876R | 4.31 |
| E207R | 3.33 | Q426R | 2.17 | Q652R | 12.49 | L877R | 0.02 |
| Y208R | 0.01 | I427R | 0.02 | F653R | 7.69 | T878R | 6.53 |
| L209R | 0.01 | G428R | 0.20 | I654R | 0.79 | P879R | 0.86 |
| S210R | 5.57 | L429R | 0.15 | S655R | 7.91 | C880R | 6.48 |
| T211R | 0.04 | Q430R | 0.49 | K657R | 12.28 | S881R | 0.02 |
| S213R | 0.00 | K431R | 13.83 | I658R | 0.16 | E882R | 0.12 |
| S213R | 0.02 | K432R | 8.31 | A659R | 0.07 | S883R | 9.66 |
| E214R | 6.43 | N433R | 9.26 | S660R | 9.93 | G884R | 0.83 |
| M215R | 0.95 | G434R | 8.66 | K661R | 7.51 | Y885R | 0.00 |
| M215R | 1.65 | S435R | 8.61 | I662R | 0.02 | K886R | 13.62 |
| L216R | 0.01 | F436R | 3.47 | I662R | 0.00 | S887R | 0.02 |
| L218R | 12.59 | Y437R | 4.29 | V663R | 8.17 | F888R | 0.01 |
| Q219R | 2.40 | V438R | 0.01 | Q664R | 10.50 | Q889R | 6.72 |
| V220R | 0.01 | T439R | 0.12 | Y665R | 3.72 | A890R | 1.35 |
| K221R | 5.89 | L440R | 0.03 | S666R | 0.63 | K891R | 9.43 |
| S222R | 0.01 | M441R | 0.34 | K667R | 15.87 | S126R & Q138H | 5.53 |
| C223R | 3.47 | F442R | 1.82 | G668R | 12.07 | S605R & L502Q | 0.00 |
| C223R & N342S | 1.61 | K117R & G106S | 0.39 | M378R & G349D | 0.00 | S91R & V3G | 10.95 |
| D136Y & C209R | 0.01 | K312R & I316N & R317S & A318G & Y319F & N320S & G321S | 0.65 | M634R & G782S | 7.96 | V388R & D418E | 3.29 |
| D684R & S683C | 6.49 | K432R & E314K | 0.12 | M807R & L801M | 0.01 | V750R & V671M | 3.84 |
| F55R & N59T | 11.97 | K565R & P570S | 9.27 | N110R & S113C | 15.01 | | |
| I427R & Y423S | 0.01 | K724R & D726A | 0.02 | N511R & P516H | 7.32 | | |
| I494M & G498R | 0.13 | M215I & P501R | 2.37 | Q521R & V487M | 0.90 | | |

Analysis of the AlphaFold3 structure prediction results based on the ternary complex of C12-334+gRNA+target DNA shows that many of the advantageous mutants with enhanced gene editing efficiency identified in the experimental screening are associated to the binding mechanisms between the Cas proteins and nucleic acids (sgRNA/dsDNA). After mutating to Arg, the corresponding amino acids enhance the binding ability of the Cas proteins to sgRNA/dsDNA by forming salt bridges with the backbone phosphate groups of sgRNA or dsDNA and forming cation-n interactions or hydrogen bonds with the bases, thereby improving the editing efficiency, as shown in Table 7 and FIG. 11.

In addition, D480, E675, and D757 belong to an enzyme active center. After mutation of these residues or nearby residues, the cleavage activity is completely or partially lost.

The C12-334 protein comprises domains (represented by amino acid residue position ranges): aa1-24 WED, aa25-109 Helical 1, aa110-182 PI, aa183-340 Helical 1, aa341-447 WED, aa448-522 RuvC, aa523-644 Helical 2, aa645-720 RuvC, aa721-756 Nuc, aa757-769 RuvC, and aa770-891 Nuc.

**Table 7. Structural analysis of mutants with enhanced cleavage activity**

| C12-334 mutation | Structural analysis (based on predicted structure after the corresponding site is mutated to R) | Domain |
|---|---|---|
| V3R | potential formation of salt bridge with phosphate groups of DNA target strand (TS) near PAM | WED |
| D9R | potential formation of salt bridge with phosphate groups of DNA TS near PAM | WED |
| S100R | potential formation of salt bridge with phosphate groups of DNA non-target strand (NTS) near PAM | Helical 1 |
| N115R | potential formation of salt bridge with phosphate groups of DNA TS near PAM | PI |
| K142R | potential formation of salt bridge with phosphate group of DNA NTS strand near PAM | PI |
| K232R | interaction with phosphate groups of DNA TS near PAM | Helical 1 |
| D279R | interaction with phosphate groups of DNA NTS | Helical 1 |
| M378R | potential formation of salt bridge with backbone phosphate groups of nucleotide 1 of gRNA | WED |
| N394R | potential formation of salt bridge with phosphate groups of DNA TS near PAM | WED |
| F537R | interaction with phosphate groups of DNA NTS | Helical 2 |
| F548R | potential formation of salt bridge with backbone phosphate groups of DNA TS | Helical 2 |
| I691R | potential interaction with side chain bases of DNA NTS | RuvC |
| L693R | potential formation of salt bridge and hydrogen bonds with backbone phosphate groups of sqRNA | RuvC |
| D697R | potential formation of salt bridge with backbone phosphate groups of DNA TS | RuvC |
| T704R | on the same helix as D697, potentially similar mechanism, and potential combined mutagenesis with 697 | RuvC |
| M720R | potential interaction with side chain bases of DNA NTS | RuvC |
| I788R | unclear mechanism, distant from surrounding chains | Nuc |
| C148R | potential formation of salt bridge with phosphate groups of DNA NTS near PAM | PI |
| N371R | potential formation of salt bridge with phosphate groups of DNA NTS near PAM | WED |
| F390R | potential formation of salt bridge with phosphate groups of DNA NTS near PAM | WED |
| T443R | potential formation of salt bridge with phosphate groups of DNA TS near PAM | RuvC |
| K533R | interaction with phosphate groups of DNA NTS | Helical 2 |
| K544R | potential formation of salt bridge with backbone phosphate groups of sqRNA | Helical 2 |
| D545R | potential formation of salt bridge with backbone phosphate groups of DNA TS | Helical 2 |
| S571R | potential formation of salt bridge with backbone phosphate groups of qRNA | Helical 2 |
| K591R | unclear reason, and no interaction with nucleic acids | Helical 2 |
| S678R | potential formation of salt bridge with backbone phosphate groups of DNA TS, spatial position relatively close to 697, potentially similar mechanism and potential combination | RuvC |

### Example 6. Detection of editing efficiency of different mutants targeting HPRT1

In this example, a target site with WYR as a PAM sequence was first selected for *HPRT1* gene (GeneBank:NG_012329.2). sgRNAs targeting different positions were designed and constructed (as shown in Table 8). The sgRNAs were combined with different mutants to detect the editing efficiency.

**Table 8. sgRNA targeting HPRT1**

| sgRNA name | PAM | Spacer sequence | sgRNA sequence |
|---|---|---|---|
| C12-334-HPRT1-sgRNA01 | TCA | CCACGACGCCAGGGCTG CGGGTCG (SEQ ID NO: 209) | gtgcgaaacggtctcgttagaggctggttcaagcacCCAC GACGCCAGGGCTGCGGGTCG (SEQ ID NO: 214) |
| C12-334-HPRT1-sgRNA02 | ACG | ACGCCAGGGCTGCGGG TCGCCA (SEQ ID NO: 210) | gtgcgaaacggtctcgttagaggctggttcaagcacACG CCAGGGCTGCGGGTCGCCA (SEQ ID NO: 215) |
| C12-334-HPRT1-sgRNA03 | ATG | AACCAGGTTATGACCTT GATTT (SEQ ID NO: 211) | gtgcgaaacggtctcgttagaggctggttcaagcacAACC AGGTTATGACCTTGATTT (SEQ ID NO: 216) |
| C12-334-HPRT1-sgRNA04 | TTA | TGCTGAGGATTTGGAAA GGGTG (SEQ ID NO: 212) | gtgcgaaacggtctcgttagaggctggttcaagcacTGCT GAGGATTTGGAAAGGGTG (SEQ ID NO: 217) |
| C12-334-HPRT1-sgRNA05 | TTG | GAAAGGGTGTTTATTCC TCATGG (SEQ ID NO: 213) | gtgcgaaacggtctcgttagaggctggttcaagcacGAA AGGGTGTTTATTCCTCATGG (SEQ ID NO: 218) |

sgRNA plasmids were constructed according to the sgRNA sequences in the above table. A vector plasmid SpCas9-gRNA-pUC57Kan (SEQ ID NO: 219) was linearized by digestion with BbsI (Thermofisher) and XhoI (Thermofisher). Primers were synthesized for different sgRNA sequences. The primers were annealed and ligated into the linearized vector. The ligation product was transformed into *Escherichia coli* to obtain final sgRNA expression vector plasmids.

### 1) Editing efficiency of wild-type C12-334 in combination with different sgRNAs targeting HPRT1

A plasmid of wild-type C12-334, C12-334-pCDHPAM02, was first combined with five different sgRNA plasmids. The combinations were transfected into HEK293T cells using PEI. Cells were collected 48 h after transfection and the cells were lysed using DirectPCR^{®} Lysis Reagent (Cell) (VIAGEN). Different primers were selected for PCR amplification according to different target sites. Sanger sequencing was performed, and the editing efficiency was determined using TIDE analysis.

Specific amplification and sequencing primers are shown in Table 9. Editing efficiency results are shown in Table 10. It indicates that C12-334-HPRT1-sgRNA05 has the highest editing efficiency.

**Table 9. Amplification and sequencing primers for different target sites**

| Amplification primer name | Primer sequence | Corresponding sgRNA | Sequenci ng primer |
|---|---|---|---|
| HPRT1-PF1 | TGCGACGAGCCCTCAGGCGA (SEQ ID NO: 220) | C12-334-HPRT1-sgRNA01 and C12-334-HPRT1-sgRNA02 | HPRT1-PR1 |
| HPRT1-PR1 | CTTCCAGGGAAGGGCCTCTCCC (SEQ ID NO: 221) | | |
| HPRT1-PF2 | GCCCGGCCTGTTGTTTTCTTACAT (SEQ ID NO: 222) | C12-334-HPRT1-sgRNA03, C12-334-HPRT1-sgRNA04, and C12-334-HPRT1-sgRNA05 | HPRT1-PR2 |
| HPRT1-PR2 | AGACTCTGGCTAGAGTTCCTTCTTC CAT (SEQ ID NO: 223) | | |

**Table 10. Editing efficiency of wild-type C12-334 targeting HPRT1 target site**

| Target sgRNA | PAM | Editing efficiency |
|---|---|---|
| C12-334-HPRT1-sgRNA01 | TCA | 1.20% |
| C12-334-HPRT1-sgRNA02 | ACG | 3.60% |
| C12-334-HPRT1-sgRNA03 | ATG | 2.90% |
| C12-334-HPRT1-sgRNA04 | TTA | 3.7% |
| C12-334-HPRT1-sgRNA05 | TTG | 9.10% |

### 2) Editing efficiency of different C12-334 mutants in combination with C12-334-HPRT1-sgRNA05

The editing efficiency testing method was the same as that described in "1)". Different mutants were combined with C12-334-HPRT1-sgRNA05, and the combinations were transfected into HEK293T cells using PEI. Cells were collected 48 h after transfection and lysed using DirectPCR^{®} Lysis Reagent (Cell) (VIAGEN). PCR amplification was performed and Sanger sequencing was conducted. The editing efficiency was determined using TIDE analysis. Editing results of different mutants are shown in FIG. 12, which shows editing efficiencies of single-point mutants and triple-point mutants.

### Example 7

In this example, the editing activity testing was performed by delivering modified gRNA (C12-334-dmHPRT1-sgRNA05-01) and mRNA.

A sequence of C12-334-dmHPRT1-sgRNA05-01 gRNA targeting *HPRT1* gene is dG*dT*dT*dGdCdAdAdTdCdCdCdAdAdGrGrUrGrCrGrArArArCrGrGrUrCrUrCrGrUrUrArGrArGrGrCr UrGrGrUrUrCrArArGrCrArCrGrArArArGrGrGrUrGrUrUrUrArUrUrCrCrUrCrA*mU*mG*mG (SEQ ID NO: 224). First 14 nucleotides at the 5' end are DNA base-modified sequences, first 3 bases at the 5' end of the gRNA are phosphorothioate-modified, and the last 3 bases are phosphorothioate-modified and 2'-methoxy-modified.

Coding mRNAs for wild-type C12-334, and N115R and D697R mutants were obtained by *in vitro* transcription, which were respectively combined with chemically synthesized C12-334-dmHPRT1-sgRNA05-01. The combinations were transformed into HEK293 cells by electroporation, and cells were collected 72 h after transformation and lysed using DirectPCR^{®} Lysis Reagent (Cell) (VIAGEN). PCR amplification was performed using primers ChkHPRT1-NGS-PF1 (SEQ ID NO: 225) and ChkHPRT1-NGS-PR1 (SEQ ID NO: 226), a library was constructed after PCR amplification, NGS sequencing was performed, and the editing efficiency was detected. The editing efficiencies of wild-type C12-334, N115R mutant, and D697R mutant in combination with C12-334-dmHPRT1-sgRNA05-01 reach 48.80%, 90.88%, and 92.77%, respectively, as shown in FIGs. 13A-13C.

### Example 8. Activity testing of C12-314 to C12-333, C12-337, C12-342 to C12-354 in Table 1

### 1. Vector construction

A vector plasmid pET28a was double-digested with BamHI and XhoI. The linearized vector was recovered by agarose gel electrophoresis and gel extraction. A DNA fragment encoding the Cas protein of the present disclosure was obtained. The DNA fragment was inserted into a cloning region of a vector pET28a by homologous recombination (NEB, Gibson Assembly^{®} Master Mix) to construct a recombinant vector. The reaction solution was transformed into Stbl3 competent cells, and the competent cells were plated on LB plates with kanamycin sulfate resistance. After overnight culture at 37°C, clones were picked for sequencing identification.

Positive clones with correct sequences were picked for overnight culture. After plasmid extraction, the plasmids were transformed into expression strain Rosetta (DE3). Cells were plated on the LB plates containing kanamycin sulfate, and cultured overnight at 37°C.

### 2. Recombinant protein expression

A single clone was inoculated into 5 mL of LB culture medium containing kanamycin sulfate, and cultured overnight at 37°C.

The single clone was reinoculated into 500 mL of LB culture medium containing kanamycin sulfate at a ratio of 1:100 and cultured at 37°C and 220 rpm until OD reached 0.6. IPTG was added to a final concentration of 0.2 mM and induction was performed at 16°C for 24 h.

Cells were washed with 15 mL of PBS, cell pellets were collected by centrifugation, and lysis buffer was added for sonication disruption. The supernatant containing the recombinant protein was obtained by centrifugation at 10,000 g for 30 min. The supernatant was filtered through a 0.45 µm filter and then loaded onto a column for purification.

### 3. Recombinant protein purification

A Cas recombinant protein was obtained by purification using 6×His tags at the N-terminus as purification tags. The purification was performed through Immobilized Metal Ion Affinity Chromatography (IMAC) and chromatographic purification. The recombinant protein has a structure of His tag-NLS-Cas-NLS-NLS. The purified recombinant protein was detected by SDS-PAGE electrophoresis.

### 4. Determination of PAM sequence recognized by the Cas protein

A single guide RNA (sgRNA) containing a specific guide sequence was mixed with the purified recombinant protein. The mixture was used to cleave an *in vitro* cleavage substrate containing the spacer sequence and the 7nt random sequence. After incubation at 37°C, the product was purified, a library was constructed, NGS sequencing and analysis were performed to determine the PAM sequence recognized by the Cas protein.

The sequence of the designed *in vitro* cleavage substrate is as follows:

ggagttcagacgtgtgctcttccgatctcagcacaaaaggaaactcaccctaactgtaaagtaattgtgtgttttgagactataaatatgc
atgcgagaaaagccttgtttgccaccatGGAACGGCTCGGAGATCATCATTGCGNNNNNNNgtgagcaagggcgagg
agctgttcaccggggtggtgcccatcctggtcgagctggacggcgacgtaaacggccacaagttcagcgtgtccggcagatcggaag
agcacacgtctgaactcc (SEQ ID NO: 227).

In the sequence, N represents any one of A, T, C, or G.

The cleavage substrate was sent to a sequencing company for PCR-free library construction and NGS sequencing.

### 5. Preparation of sgRNA

The sgRNA (5'-DR-guide sequence-3') containing any corresponding DR sequence of the Cas12 protein in Table 2 was transcribed *in vitro.* The transcription product was precipitated and purified using LiCl. The guide sequence is GUGAGCAAGGGCGAGGAGCUGUUC (SEQ ID NO: 228) or CGCAAUGAUGAUCUCCGAGCCGUUCC (SEQ ID NO: 229).

PAM library cleavage, NGS sequencing, and analysis of NGS results: the captured 7nt random sequences were obtained using WebLogo software for data analysis according to the method described in the reference (A compact Cas9 ortholog from Staphylococcus Auricularis (SauriCas9) expands the DNA targeting scope. PLoS biology, 2020,18(3), e3000686.). The PAM sequence was identified accordingly.

### 6. Testing of in vitro cleavage activity of Cas protein

The aforementioned sgRNA and the recombinant protein were mixed, the mixture was used to cleave a target DNA (dsDNA or ssDNA) *in vitro,* and the cleavage product was visualized by gel electrophoresis to demonstrate the cleavage effect of the Cas protein.

### 7. Testing of editing activity in eukaryotic cells

The aforementioned sgRNA, with the guide sequence replaced by a guide sequence targeting human cells (i.e., any one of the sequences shown in SEQ ID NOs: 209-213), was mixed with the recombinant protein and incubated to form an RNP. The RNP was transfected into 293T cells. After overnight transfection, a fresh medium was replaced, and the culture was continued.

DNA extraction, PCR amplification, and Sanger sequencing: after 72 h of culture, the cells were washed with PBS. Then, 100 µL of cell lysis buffer was added for lysis to obtain a lysate containing genomic DNA. A region near the target sequence in the genomic DNA was amplified by PCR. The PCR product was sent to a sequencing company for Sanger sequencing. Sequencing data analysis: the sequencing chromatogram and information related to the gRNA guide sequence were analyzed by TIDE to obtain the editing efficiency for the target nucleic acid.

## Claims

1. A Cas12 protein, wherein the Cas12 protein conforms to any one of the following (a)-(c):
(a) the Cas12 protein is selected from the group consisting of a CLUSTER1 protein, a CLUSTER2 protein, a CLUSTER3 protein, a CLUSTER4 protein, a CLUSTERS protein, a CLUSTER6 protein, a CLUSTER7 protein, a CLUSTER8 protein, a CLUSTER9 protein, a CLUSTER10 protein, a CLUSTER11 protein, and a CLUSTER12 protein;
(b) the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of amino acid sequences shown in SEQ ID NOs: 1-35; and
(c) the Cas12 protein belongs to a Cas12h subtype, wherein the Cas12 protein forms a complex with a guide polynucleotide, and the complex specifically binds to a target nucleic acid in a eukaryotic cell;
preferably, the Cas12 protein retains a function of a protein having an amino acid sequence as shown in any one of SEQ ID NOs: 1-35;
preferably, a protospacer adjacent motif (PAM) sequence (5'→3') recognized by the Cas12 protein is selected from any one or more of the following:
A, C, T, G,
TA, TC, GN, AA, AG, TG, AN, GG, CG, TN, NT, NG, GT, NA, CC, AC, GC, AT, CT, GA, TT, CN, NC, CA,
NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG,
NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, and NNGC;
preferably, the PAM sequence (5'→3') recognized by the Cas12 protein is selected from any one or more of the following: WYR, BMCTTH, TTN, VNWTV, VNWTC, and VNTTC, wherein W is A or T, Y is C or T, R is A or G, B is C, G, or T, M is A or C, H is A, T, or C, N is A, T, C, or G, and V is A, C, or G;
preferably, the Cas12 protein forms a complex with the guide polynucleotide; further, the complex specifically binds to the target nucleic acid; further, the complex is capable of cleaving the target nucleic acid, modifying the target nucleic acid, and/or regulating an expression of the target nucleic acid;
preferably, the Cas12 protein forms a complex with the guide polynucleotide, wherein the guide polynucleotide comprises a guide sequence that is reverse complementary to the target nucleic acid; further, the guide polynucleotide comprises a scaffold sequence that interacts with the Cas12 protein; further, the scaffold sequence comprises a direct repeat (DR) sequence;
preferably, the scaffold sequence does not comprise a tracrRNA sequence;
preferably, the Cas12 protein is a nuclease-inactivated mutant; preferably, the Cas12 protein is a dead Cas12 mutant or a nickase Cas12 mutant; preferably, the Cas12 protein has an inactivated RuvC domain;
preferably, the Cas12 protein forms a complex with a guide polynucleotide, and the complex specifically binds to a target nucleic acid in a eukaryotic cell; further preferably, the Cas12 protein forms a complex with a guide polynucleotide, and the complex specifically binds to a target nucleic acid and cleaves the target nucleic acid in a eukaryotic cell;
preferably, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to an amino acid sequence shown in SEQ ID NO: 18; preferably, the Cas12 protein is non-natural or engineered; preferably, the Cas12 protein forms a complex with a guide polynucleotide, and the complex specifically binds to a target nucleic acid; preferably, the complex specifically binds to and cleaves the target nucleic acid, or the complex specifically binds to the target nucleic acid but does not cleave the target nucleic acid; preferably, the complex is non-natural or engineered; preferably, the guide polynucleotide comprises a guide sequence and a scaffold sequence; preferably, the guide sequence is reverse complementary to the target nucleic acid, and the scaffold sequence interacts with the Cas12 protein; preferably, the scaffold sequence is a direct repeat sequence; preferably, the guide sequence is located at a 5' end or a 3' end of the scaffold sequence; preferably, the guide polynucleotide is non-natural or engineered; preferably, the PAM sequence recognized by the Cas12 protein is 5'-WYR-3', wherein W is A or T, Y is C or T, and R is A or G; preferably, the PAM sequence recognized by the Cas12 protein is 5'-ACA-3', 5'-TCA-3', 5'-ATA-3', 5'-TTA-3', 5'-ACG-3', 5'-TCG-3', 5'-ATG-3', 5'-TTG-3', and/or 5'-TTN-3'; preferably, the scaffold sequence comprises a nucleotide sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of sequences shown in SEQ ID NO: 84-86 or SEQ ID NO: 187-195; preferably, the scaffold sequence comprises a nucleotide sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to a sequence shown in SEQ ID NO: 84; preferably, the Cas12 protein has at least one mutation in at least 1, at least 2, at least 3, at least 4, at least 5, at least 6, at least 7, at least 8, at least 9, at least 10, at least 11, or at least 12 of amino acid residues corresponding to positions 1-891 of the amino acid sequence shown in SEQ ID NO: 18; preferably, the mutation is a mutation to any other natural amino acid residue; preferably, the mutation is a mutation to residue R, H, K, or A; preferably, the mutation is a mutation to residue R; preferably, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 18, and the positions are selected from N5, D9, E58, S100, N115, K142, C148, S147, K232, S245, I251, Y263, D279, A297, L300, E303, L337, M378, N394, T396, T443, K458, T468, K533, F537, F548, N550, D697, A706, and I788; preferably, the mutation is a mutation to residue R; preferably, the Cas12 protein has at least one mutation at amino acid residues corresponding to any 1, any 2, or any 3 positions in the amino acid sequence shown in SEQ ID NO: 18, and the positions are selected from D480, E675, and D757; preferably, the mutation is a mutation to any other natural amino acid residue; preferably, the mutation is a mutation to residue A.

2. A guide polynucleotide, comprising:
(i) a direct repeat sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to a sequence shown in any one of SEQ ID NOs: 36-170 and 187-195, and
(ii) a guide sequence engineered to hybridize with a target nucleic acid; wherein the direct repeat sequence is linked to the guide sequence, and the guide polynucleotide forms a complex with a Cas12 protein and guides sequence-specific binding of the complex to the target nucleic acid;
preferably, the Cas12 protein is the Cas12 protein of claim 1;
preferably, the guide sequence comprises 15-60 nucleotides;
preferably, the guide sequence hybridizes with the target nucleic acid, and the guide sequence is mismatched to the target nucleic acid by no more than one nucleotide;
preferably, the guide polynucleotide does not comprise or comprises a tracrRNA;
preferably, the tracrRNA sequence is linked to the direct repeat sequence;
preferably, the tracrRNA comprises 10-200 nucleotides;
preferably, the guide sequence is located at a 3' end of the direct repeat sequence;
preferably, the guide sequence is located at a 5' end of the direct repeat sequence.

3. An inactivated Cas12 mutant, wherein the inactivated Cas12 mutant is a nuclease-inactivated mutant of the Cas12 protein of claim 1;
preferably, the inactivated Cas12 mutant is a dead Cas12 mutant or a nickase Cas12 mutant;
preferably, the inactivated Cas12 mutant has an inactivated RuvC domain.

4. A fusion protein or conjugate, comprising:
(1) the Cas12 protein of claim 1, or the inactivated Cas12 mutant of claim 3; and
(2) a homologous or heterologous functional domain;
preferably, the functional domain has an enzymatic activity for modifying the target nucleic acid sequence; the enzymatic activity comprising a nuclease activity, a methyltransferase activity, a demethylase activity, a DNA repair activity, a DNA damage activity, a deaminase activity, a dismutase activity, an alkylation activity, a depurination activity, an oxidation activity, a pyrimidine dimer formation activity, an integrase activity, a transposase activity, a recombinase activity, a polymerase activity, a ligase activity, a helicase activity, a photolyase activity, a glycosylase activity, a deglycosylation activity, an acetyltransferase activity, a deacetylase activity, a kinase activity, a phosphatase activity, a ubiquitin ligase activity, a deubiquitination activity, an adenylation activity, a deadenylation activity, a SUMOylating activity, a deSUMOylating activity, a myristoylation activity, and/or a demyristoylation activity;
preferably, the homologous or heterologous functional domain is selected from one or more of the following: a subcellular localization signal, a DNA binding domain, a protease domain, a transcriptional activation domain, a transcriptional repression domain, a nuclease domain, a deaminase domain, a uracil DNA glycosylase domain (UDG), a uracil DNA glycosylase inhibitor domain (UGI), a methylase, a demethylase, a transcription release factor, a histone acetyltransferase domain, a histone deacetylase domain, a DNA ligase, an affinity tag, a reporter tag, an affinity domain, and a reporter domain;
preferably, the nuclease domain comprises a polypeptide with an ssDNA cleavage activity and/or a polypeptide with a dsDNA cleavage activity;
preferably, the Cas12 protein or the inactivated Cas12 mutant is directly or indirectly linked to the homologous or heterologous functional domain; preferably, the direct linkage is a covalent linkage, and the indirect linkage is a linkage through an amino acid linker or a non-amino acid linker;
preferably, the homologous or heterologous functional domain is fused or conjugated at N-terminal, C-terminal, or internally with respect to the Cas12 protein or the inactivated Cas12 mutant;
preferably, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence shown in SEQ ID NO: 18; preferably, the Cas12 protein is non-natural or engineered; preferably, the Cas12 protein forms a complex with a guide polynucleotide, and the complex specifically binds to a target nucleic acid; preferably, the complex specifically binds to and cleaves the target nucleic acid, or the complex specifically binds to the target nucleic acid but does not cleave the target nucleic acid; preferably, the complex is non-natural or engineered; preferably, the guide polynucleotide comprises a guide sequence and a scaffold sequence; preferably, the guide sequence is reverse complementary to the target nucleic acid, and the scaffold sequence interacts with the Cas12 protein; preferably, the scaffold sequence is a direct repeat sequence; preferably, the guide sequence is located at the 5' end or the 3' end of the scaffold sequence; preferably, the guide polynucleotide is non-natural or engineered; preferably, a PAM sequence recognized by the Cas12 protein is 5'-WYR-3', wherein W is A or T, Y is C or T, and R is A or G; preferably, the PAM sequence recognized by the Cas12 protein is 5'-ACA-3', 5'-TCA-3', 5'-ATA-3', 5'-TTA-3', 5'-ACG-3', 5'-TCG-3', 5'-ATG-3', 5'-TTG-3', and/or 5'-TTN-3'; preferably, the scaffold sequence comprises a nucleotide sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of sequences shown in SEQ ID NO: 84-86 and 187-195; preferably, the scaffold sequence comprises a nucleotide sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to a sequence shown in SEQ ID NO: 84; preferably, the Cas12 protein has at least one mutation in at least 1, at least 2, at least 3, at least 4, at least 5, at least 6, at least 7, at least 8, at least 9, at least 10, at least 11, or at least 12 of amino acid residues corresponding to positions 1-891 of the sequence shown in SEQ ID NO: 18; preferably, the mutation is a mutation to any other natural amino acid residue; preferably, the mutation is a mutation to residue R, H, K, or A; preferably, the mutation is a mutation to residue R; preferably, the Cas12 protein has at least one mutation at amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 18, and the positions are selected from N5, D9, E58, S100, N115, K142, C148, S147, K232, S245, I251, Y263, D279, A297, L300, E303, L337, M378, N394, T396, T443, K458, T468, K533, F537, F548, N550, D697, A706, and I788; preferably, the mutation is a mutation to residue R; preferably, the Cas12 protein has at least one mutation at amino acid residues corresponding to any 1, any 2, or any 3 positions of the amino acid sequence shown in SEQ ID NO: 18, and the positions are selected from D480, E675, and D757; preferably, the mutation is a mutation to any other natural amino acid residue; preferably, the mutation is a mutation to residue A; preferably, the functional domain has an epigenomic modification activity or an epigenetic modification activity; preferably, the epigenomic modification or the epigenetic modification comprises, but is not limited to, DNA methylation, RNA methylation, RNA interference, nucleosome positioning, chromatin conformation change, chromatin remodeling, histone modification, and modification of long non-coding RNA sequences; preferably, the functional domain is an epigenomic modification functional domain or an epigenetic modification functional domain; preferably, the functional domain has a single-base editing activity; preferably, the functional domain is selected from one or more of the following: a nuclease (e.g., FokI), a DNA methyltransferase, a DNA demethylase, a histone methyltransferase, a histone demethylase, a DNA repair enzyme, a DNA damage enzyme, a base deaminase (including, but not limited to, an adenine deaminase, a cytosine deaminase), a dismutase, an alkylase, a depurinase, an oxidase, a pyrimidine dimer-forming enzyme, an integrase, a transposase, a recombinase, a polymerase, a ligase, a helicase, a photolyase, a glycosylase, a deglycosylase, an acetyltransferase, a deacetylase, a kinase, a phosphatase, a ubiquitin ligase, a deubiquitinating enzyme, an adenylylase, a deadenylase, a SUMOylating enzyme, a deSUMOylating enzyme, a myristoylase, and/or a demyristoylase; preferably, the functional domain is an adenine deaminase or a cytosine deaminase.

5. An isolated nucleic acid, wherein the isolated nucleic acid encodes the Cas12 protein of claim 1, the inactivated Cas12 mutant of claim 3, or the fusion protein or conjugate of claim 4;
preferably, the nucleic acid is codon optimized for expression in cells;
preferably, the nucleic acid is codon optimized for expression in a prokaryotic cell;
preferably, the nucleic acid is codon optimized for expression in a eukaryotic cell;
preferably, the nucleic acid is codon optimized for expression in an eukaryote, a mammal such as a human or a non-human mammal, a plant, an insect, a bird, a reptile, a rodent (e.g., a mouse, a rat), a fish, a worm/nematode, or a yeast.

6. A CRISPR-Cas12 system, comprising:
a) the Cas12 protein of claim 1, the inactivated Cas12 mutant of claim 3, the fusion protein or conjugate of claim 4, or the isolated nucleic acid according to claim 5; and
b) a guide polynucleotide, or a polynucleotide sequence encoding the guide polynucleotide;
preferably, the Cas12 protein, the inactivated Cas12 mutant, the fusion protein or the conjugate forms a complex with the guide polynucleotide; and the guide polynucleotide comprises a guide sequence engineered to guide a sequence-specific binding of the complex to a target nucleic acid;
preferably, the guide polynucleotide comprises a direct repeat sequence linked to the guide sequence, preferably, the direct repeat sequence has at least 50% sequence identity to any one of sequences shown in SEQ ID NOs: 36-170 and 187-195;
preferably, the guide sequence comprises 15-35 nucleotides, and/or the guide sequence hybridizes to the target nucleic acid, the guide sequence is 90%-100% complementary to the target nucleic acid, preferably, the guide sequence is mismatched to the target nucleic acid by no more than one nucleotide.
preferably, the guide sequence comprises 15-60 nucleotides;
preferably, the guide sequence hybridizes with the target nucleic acid;
preferably, the guide sequence is mismatched to the target nucleic acid by no more than one nucleotide;
preferably, the guide sequence is located at the 3' end of the direct repeat sequence;
preferably, the guide sequence is located at the 5' end of the direct repeat sequence;
preferably, the target nucleic acid is DNA or RNA, preferably, dsDNA or ssDNA;
preferably, the DNA is eukaryotic DNA; preferably, the eukaryotic DNA is non-human mammalian DNA, non-human primate DNA, human DNA, plant DNA, insect DNA, bird DNA, reptile DNA, rodent DNA, fish DNA, worm/nematode DNA, or yeast DNA;
preferably, the target nucleic acid is a disease or disorder-related gene or a signaling biochemical pathway-related gene, or the target nucleic acid is a reporter gene.

7. A vector system, comprising one or more recombinant vectors, wherein the one of the recombinant vectors comprise the isolated nucleic acid of claim 5, or the CRISPR-Cas12 system of claim 6;
preferably, the recombinant vector further comprises a regulatory sequence;
preferably, a polynucleotide sequence encoding the Cas12 protein, the inactivated Cas12 mutant, or the fusion protein or the conjugate is operably linked to a regulatory sequence, and/or a polynucleotide sequence encoding the guide polynucleotide is operably linked to the regulatory sequence; more preferably, the regulatory sequence is preferably selected from at least one of a promoter, an enhancer, an internal ribosome entry site (IRES), and a transcription termination signal, wherein the promoter comprises a constitutive promoter, an inducible promoter, a broad-spectrum promoter, or a tissue-specific promoter, and/or the transcription termination signal comprises a polyadenylation signal or a poly-U sequence;
preferably, a scaffold of the recombinant vector is an adeno-associated virus (AAV) vector, a lentiviral vector, a ribonucleoprotein (RNP) complex, or a virus-like particle; preferably, when the scaffold is the AAV vector, the AAV vector is a recombinant AAV vector of serotype AAV1, AAV2, AAV3, AAV4, AAV5, AAV6, AAV7, AAV8, AAV9, AAV10, AAV11, AAV12, AAV13, AAV PHP.B, AAV PHP.B2, AAV PHP.B3, AAV PHP.A, AAV PHP.eB, AAV PHP.eS, AAV2.7m8, AAV8.7m8, AAV ShH10, AAVrh10, or AAVrh74; when the scaffold is the lentiviral vector, the lentiviral vector is pseudotyped with an envelope protein;
preferably, the isolated nucleic acid is linked to an aptamer sequence;
when the scaffold of the recombinant vector is the virus-like particle, the isolated nucleic acid is linked to a gene encoding a gag protein.

8. A delivery system, comprising:
(1) a delivery tool; and
(2) the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, or the vector system of claim 7;
preferably, the delivery tool is a virus, a lipid nanoparticle, a nanoparticle, a liposome, an exosome, a microbubble, or a gene gun;
preferably, the delivery tool is the lipid nanoparticle, and the lipid nanoparticle comprises the guide polynucleotide and an mRNA encoding the Cas12 protein, the inactivated Cas12 mutant, or the fusion protein or conjugate.

9. A cell, comprising the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, or the delivery system of claim 8;
preferably, the cell is a prokaryotic cell;
preferably, the cell is a eukaryotic cell;
preferably, the eukaryotic cell is a mammalian cell.

10. A pharmaceutical composition, comprising the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, or the cell of claim 9;
preferably, the pharmaceutical composition comprises pharmaceutically acceptable excipients.

11. A kit, comprising the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, the cell of claim 9, or the pharmaceutical composition of claim 10.

12. A use of the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, the cell of claim 9, the pharmaceutical composition of claim 10, or the kit of claim 11 in preparing a reagent or medicament for diagnosing, treating, and/or preventing a disease or disorder associated with a target nucleic acid;
preferably, the disease or disorder is a hematological disease or disorder, an ophthalmic disease or disorder, a neurological disease or disorder, a respiratory disease or disorder, a hepatic disease or disorder, a metabolic disease or disorder, cancer, or an infectious disease; and/or the reagent or medicament is used to: cleave one or more target nucleic acid molecules or introduce nicks into one or more target nucleic acid molecules, activate or upregulate an expression of the one or more target nucleic acid molecules, activate or inhibit transcription of the one or more target nucleic acid molecules, inactivate the one or more target nucleic acid molecules, visualize, label, or detect the one or more target nucleic acid molecules, bind the one or more target nucleic acid molecules, transport the one or more target nucleic acid molecules, and mask the one or more target nucleic acid molecules.

13. A method for detecting, binding, or cleaving a target nucleic acid, comprising using the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, the cell of claim 9, the pharmaceutical composition of claim 10, or the kit of claim 11 to contact the target nucleic acid;
preferably, the method is for non-diagnostic and/or non-therapeutic purposes; and/or the fusion protein or conjugate comprises a detectable marker, such as, a marker detectable by fluorescence, DNA blotting, or FISH.

14. A method for altering a cell state, comprising using the Cas12 protein of to claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, the cell of claim 9, the pharmaceutical composition of claim 10, or the kit of claim 11 to contact a cell to alter the cell state;
preferably, the method results in one or more of: an increase or decrease in an expression of a specific gene, an induction of cellular senescence in vitro or in vivo, an induction of cellular cycle arrest in vitro or in vivo, a cellular growth promotion and/or cellular growth inhibition in vitro or in vivo, an induction of anergy in vitro or in vivo, an induction of apoptosis in vitro or in vivo, and an induction of necrosis in vitro or in vivo;
preferably, the method is for non-diagnostic and/or non-therapeutic purposes.

15. A method for diagnosing, treating, or preventing a disease or disorder associated with a target nucleic acid, comprising administering the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, the cell of claim 9, the pharmaceutical composition of claim 10, or the kit of claim 11 to a sample from a subject in need or a subject in need;
preferably, the disease or disorder is a hematological disease or disorder, an ophthalmic disease or disorder, a neurological disease or disorder, a respiratory disease or disorder, a hepatic disease or disorder, a metabolic disease or disorder, a cancer, or an infectious disease.

16. A use of the Cas12 protein of claim 1, the guide polynucleotide of claim 2, the inactivated Cas12 mutant of claim 3, the fusion protein or conjugate of claim 4, the nucleic acid of claim 5, the CRISPR-Cas12 system of claim 6, the vector system of claim 7, the delivery system of claim 8, the cell of claim 9, the pharmaceutical composition of claim 10, or the kit of claim 11 in diagnosing, treating, or preventing a disease or disorder associated with a target nucleic acid;
preferably, the disease or disorder is a hematological disease or disorder, an ophthalmic disease or disorder, a neurological disease or disorder, a respiratory disease or disorder, a hepatic disease or disorder, a metabolic disease or disorder, a cancer, or an infectious disease.
